# EUROPEAN PATENT SPECIFICATION

(11) **EP 2 041 145 B1**
(45) Date of publication and mention of the grant of the patent: **27.10.2010**
(21) Application number: 07786995.6
(22) Date of filing: 03.07.2007
(51) Int. Cl.: C07D 519/00, A61K 31/4375, A61K 31/5025, A61K 31/4985, A61P 31/04

(54) **AZATRICYCLIC COMPOUNDS AND THEIR USE**
AZATRICYCLISCHE VERBINDUNGEN UND DEREN VERWENDUNG
COMPOSÉS AZATRICYCLIQUES ET LEUR UTILISATION

(30) Priority: 03.07.2006 GB 0613208
(43) Date of publication of application: 01.04.2009
(73) Proprietor: Glaxo Group Limited, Greenford Middlesex UB6 0NN (GB)
(72) Inventor: BROOKS, Gerald, Harlow, Essex CM19 5AW (GB); MILES, Timothy James, Stevenage, Hertfordshire SG1 2NY (GB)
(74) Representative: Valentine, Jill Barbara
(86) International application number: PCT/EP2007/056664
(87) International publication number: WO 2008/003690

(56) References cited:
- WO-A-03/064431
- GB-A- 1 394 373

## Description

This invention relates to novel compounds, compositions containing them and their use as antibacterials.

WO02/08224, WO02/50061, WO02/56882, WO02/96907, WO2003087098, WO2003010138, WO2003064421, WO2003064431, WO2004002992, WO2004002490, WO2004014361, WO2004041210,WO2004096982, WO2002050036, WO2004058144, WO2004087145, WO06002047, WO06014580, WO06010040, WO06017326, WO06012396, WO06017468, WO06020561, WO01/25227, WO02/40474, WO02/07572, WO2004035569, WO2004089947, WO04024712, WO04024713, W004087647, WO2005016916, WO2005097781, WO06010831, WO04035569, WO04089947, WO06021448, WO06032466, WO06038172, WO06046552, WO06134378 and WO06137485 disclose quinoline, naphthyridine, morpholine, cyclohexane, piperidine and piperazine derivatives having antibacterial activity.

This invention provides a compound of formula (I) or a pharmaceutically acceptable salt, solvate and/or N-oxide thereof: 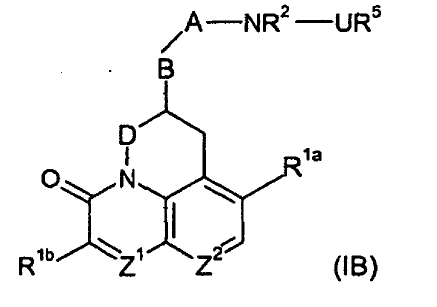 wherein:
one of B and D is CH₂ and the other is a bond;
one of Z¹ and Z² is CH or N and the other is CH;
R^{1a} and R^{1b} are independently selected from hydrogen; halogen; cyano; (C₁₋₆)alkyl; (C₁₋₆)alkylthio; trifluoromethyl; trifluoromethoxy; carboxy; hydroxy optionally substituted with (C₁₋₆)alkyl or (C₁₋₆)alkoxy-substituted (C₁₋₆)alkyl; (C₁₋₆)alkoxy-substituted (C₁₋₆)alkyl; hydroxy (C₁₋₆)alkyl; an amino group optionally N-substituted by one or two (C₁₋₆)alkyl, formyl, (C₁₋₆)alkylcarbonyl or (C₁₋₆)alkylsulphonyl groups; or aminocarbonyl wherein the amino group is optionally substituted by (C₁₋₄)alkyl;
provided that R^{1b} is H when Z¹ is N;
R² is hydrogen, or (C₁₋₄)alkyl, or together with R⁶ forms Y as defined below; A is a group (i): 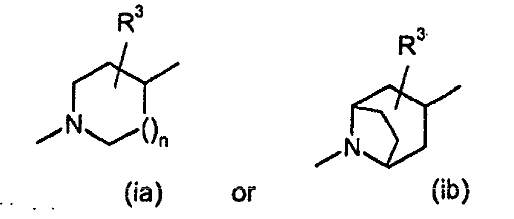
in which: R³ is as defined for R^{1a} or R^{1b} or is oxo and n is 1 or 2:
or A is a group (ii) 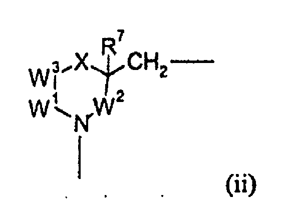
wherein:
W¹, W² and W³ are CR⁴R⁸
or W² and W³ are CR⁴R⁸ and W¹ represents a bond between W³ and N;
X is O, CR⁴R⁸, or NR⁶;
one R⁴ is as defined for R^{1a} and R^{1b} and the remainder and R⁸ are hydrogen or one R⁴ and R⁸ are together oxo and the remainder are hydrogen;
R⁶ is hydrogen or (C₁₋₆)alkyl; or together with R² forms Y;
R⁷ is hydrogen; halogen; hydroxy optionally substituted with (C₁₋₆)alkyl; or (C₁₋₆ )alkyl;
Y is CR⁴R⁸CH₂; CH₂CR⁴R⁸; (C=O); CR⁴R⁸; CR⁴R⁸(C=O); or (C=O)CR⁴R⁸;
or when X is CR⁴R⁸, R⁸ and R⁷ together represent a bond;
U is selected from CO and CH₂, and
R⁵ is an optionally substituted bicyclic carbocyclic or heterocyclic ring system (B): 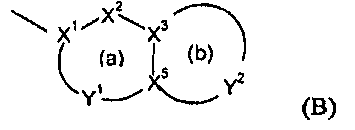 containing up to four heteroatoms in each ring in which
at least one of rings (a) and (b) is aromatic;
X¹ is C or N when part of an aromatic ring, or CR¹⁴ when part of a non-aromatic ring;
X² is N, NR¹³, O, S(O)_{X}, CO or CR¹⁴ when part of an aromatic or non-aromatic ring or may in addition be CR¹⁴R¹⁵ when part of a non-aromatic ring;
X³ and X⁵ are independently N or C;
Y¹ is a 0 to 4 atom linker group each atom of which is independently selected from N, NR¹³, O, S(O)_{X}, CO and CR¹⁴ when part of an aromatic or non-aromatic ring or may additionally be CR¹⁴R¹⁵ when part of a non-aromatic ring;
Y² is a 2 to 6 atom linker group, each atom of Y² being independently selected from N, NR¹³, O, S(O)_{X}, CO, CR¹⁴ when part of an aromatic or non-aromatic ring or may additionally be CR¹⁴R¹⁵ when part of a non-aromatic ring;
each of R¹⁴ and R¹⁵ is independently selected from: H; (C₁₋₄)alkylthio; halo; carboxy(C₁₋₄)alkyl; (C₁₋₄)alkyl; (C₁₋₄)alkoxycarbonyl; (C₁₋₄)alkylcarbonyl; (C₁₋₄) alkoxy (C₁₋₄)alkyl; hydroxy; hydroxy(C₁₋₄)alkyl; (C₁₋₄)alkoxy; nitro; cyano; carboxy; amino or aminocarbonyl optionally mono- or di-substituted by (C₁₋₄)alkyl; or
R¹⁴ and R¹⁵ may together represent oxo;
each R¹³ is independently H; trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; (C₁₋₄) alkoxycarbonyl; (C₁₋₄)alkylcarbonyl; (C₁₋₆)alkylsulphonyl; aminocarbonyl wherein the amino group is optionally mono- or di-substituted by (C₁₋₄)alkyl; and
each x is independently 0, 1 or 2.

This application also describes a method of treatment of bacterial infections in mammals, particularly in man, which method comprises the administration to a mammal in need of such treatment an effective amount of a compound of formula (I), or a pharmaceutically acceptable salt, solvate and/or N-oxide thereof.

The invention also provides the use of a compound of formula (I), or a pharmaceutically acceptable salt, solvate and/or N-oxide thereof, in the manufacture of a medicament for use in the treatment of bacterial infections in mammals.

The invention also provides a pharmaceutical composition comprising a compound of formula (I), or a pharmaceutically acceptable salt, solvate and/or N-oxide thereof, and a pharmaceutically acceptable carrier.

In particular aspects:
(1) B is CH₂ and D is a bond, Z¹ is CH and Z² is N;
(2) B is CH₂ and D is a bond, Z¹ is CH and Z² is CH;
(3) B is CH₂ and D is a bond, Z¹ is N and Z² is CH;
(4) B is a bond and D is CH₂, Z¹ is N and Z² is CH; or
(5) B is a bond and D is CH₂, Z¹ is CH and Z² is N.

In a particular aspect each R^{1a} and R^{1b} is independently hydrogen, (C₁₋₄)alkoxy, (C₁₋₄)alkylthio, (C₁₋₄)alkyl, cyano, carboxy, hydroxymethyl or halogen such as fluoro; more particularly hydrogen, methoxy, methyl, cyano, or fluoro.

In certain embodiments only one group R^{1a} or R^{1b} is other than hydrogen. In particular embodiments R^{1a} is fluoro or methoxy and R^{1b} is hydrogen.

In a particular aspect R² is hydrogen.

Particular examples of R³ include hydrogen; optionally substituted hydroxy; optionally substituted amino; halogen; (C₁₋₄) alkyl; 1-hydroxy-(C₁₋₄) alkyl; optionally substituted aminocarbonyl. More particular R³ groups are hydrogen; CONH₂; 1-hydroxyalkyl e.g. CH₂OH; optionally substituted hydroxy e.g. methoxy; optionally substituted amino; and halogen, in particular fluoro. Most particularly R³ is hydrogen, hydroxy or fluoro.

In a particular aspect, when A is (ia), n is 1. In a further aspect R³ is in the 3- or 4-position. In a more particular aspect, A is (ia), n is 1 and R³ is H or hydroxy in the 3-position, and more particularly is *cis* to the NR² group.

In a particular aspect, when A is (ii), X is CR⁴R⁸ and R⁸ is H and R⁴ is H or OH. More particularly when R⁴ is OH it is *trans* to R⁷. In a further aspect W¹ is a bond. In another aspect R⁷ is H. In an additional aspect W² and W³ are both CH₂. Where A is 3-hydroxypyrrolidin-4-ylmethyl, in a particular aspect the configuration is (3*S*,4*S*).

In certain embodiments U is CH₂.

In certain embodiments R⁵ is an aromatic heterocyclic ring (B) having 8-11 ring atoms including 2-4 heteroatoms of which at least one is N or NR¹³ in which, in particular embodiments, Y² contains 2-3 heteroatoms, one of which is S and 1-2 are N, with one N bonded to X³.

In alternative embodiments the heterocyclic ring (B) has ring (a) aromatic selected from optionally substituted benzo, pyrido, pyridazino and pyrimidino and ring (b) non aromatic and Y² has 3-5 atoms, more particularly 4 atoms, including at least one heteroatom, with O, S, CH₂ or NR¹³ bonded to X⁵ where R¹³ is other than hydrogen, and either NHCO bonded via N to X³, or O, S, CH₂ or NH bonded to X³. In a particular aspect the ring (a) contains aromatic nitrogen, and more particularly ring (a) is pyridine, pyrazine or pyrimidine.

In certain embodiments R⁵ is: 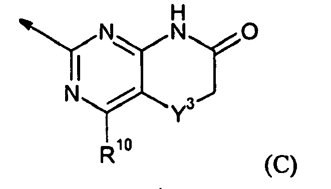 in which:
→ is the point of attachment;
Y³ is CH₂ or O; and
R¹⁰ is independently selected from hydrogen, halogen, (C₁₋₆)alkyl and (C₁₋₆) alkoxy.

More particularly R¹⁰ is selected from hydrogen, chloro, methyl and methoxy.

Examples of rings (B) include optionally substituted:

### (a) and (b) aromatic

1H-pyrrolo[2,3-b]-pyridin-2-yl, 1H-pyrrolo[3,2-b]-pyridin-2-yl, 3H-imidazo[4,5-b]-pyrid-2-yl, 3H-quinazolin-4-one-2-yl, benzimidazol-2-yl, benzo[1,2,3]-thiadiazol-5-yl, benzo[1,2,5]-oxadiazol-5-yl, benzofur-2-yl, benzothiazol-2-yl, benzo[b]thiophen-2-yl, benzoxazol-2-yl, chromen-4-one-3-yl, imidazo[1,2-a]pyridin-2-yl, imidazo-[1,2-a]-pyrimidin-2-yl, indol-2-yl, indol-6-yl, isoquinolin-3-yl, [1,8]-naphthyridine-3-yl, oxazolo[4,5-b]-pyridin-2-yl, quinolin-2-yl, quinolin-3-yl, quinoxalin-2-yl, naphthalen-2-yl, 1,3-dioxo-isoindol-2yl, benzimidazol-2-yl, 1H-benzotriazol-5-yl, 1H-indol-5-yl, 3H-benzooxazol-2-one-6-yl, 3H-benzooxazol-2-thione-6-yl, 3H-benzothiazol-2-one-5-yl, 3H-quinazolin-4-one-6-yl, benzo[1,2,3]thiadiazol-6-yl, benzo[1,2,5]thiadiazol-5-yl, benzo[1,4]oxazin-2-one-3-yl, benzothiazol-5-yl, benzothiazol-6-yl, cinnolin-3-yl, imidazo[1,2-a]pyridazin-2-yl, pyrazolo[1,5-a]pyrazin-2-yl, pyrazolo[1,5-a]pyridin-2-yl, pyrazolo[1,5-a]pyrimidin-6-yl, pyrazolo[5,1-c][1,2,4]triazin-3-yl, pyrido[1,2-a]pyrimdin-4-one-2-yl, pyrido[1,2-a]pyrimidin-4-one-3-yl, quinazolin-2-yl, quinoxalin-6-yl, thiazolo[3,2-a]pyrimidin-5-one-7-yl, thiazolo[5,4-b]pyridin-2-yl, thieno[3,2-b]pyridin-6-yl, thiazolo[5,4-b]pyridin-6-yl, thiazolo[4,5-b]pyridin-5-yl, [1,2,3]thiadiazolo[5,4-b]pyridin-6-yl, 2H-isoquinolin-1-one-3-yl, 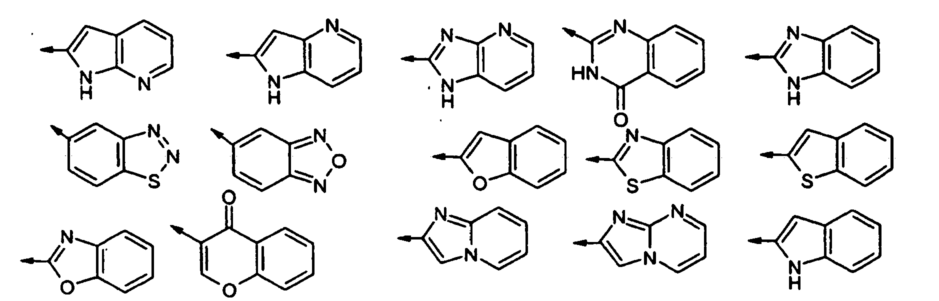 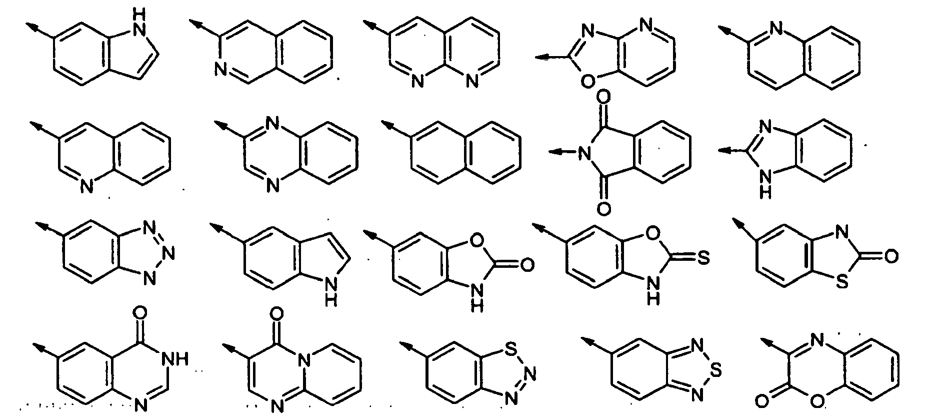 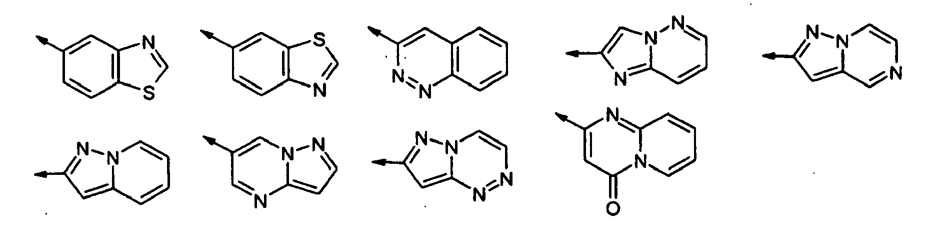 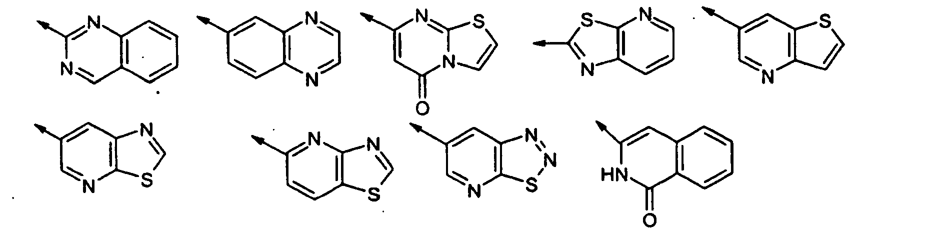 → is the point of attachment;

### (a) is non aromatic

(2S)-2,3-dihydro-1H-indol-2-yl, (2S)-2,3-dihydro-benzo[1,4]dioxine-2-yl, 3-(R,S)-3,4-dihydro-2H-benzo[1,4]thiazin-3-yl, 3-(R)-2,3-dihydro-[1,4]dioxino[2,3-b]pyridin-3-yl, 3-(S)-2,3-dihydro-[1,4]dioxino[2,3-b]pyridin-3-yl, 2,3-dihydro-benzo[1,4]dioxan-2-yl, 3-substituted-3H-quinazolin-4-one-2-yl, 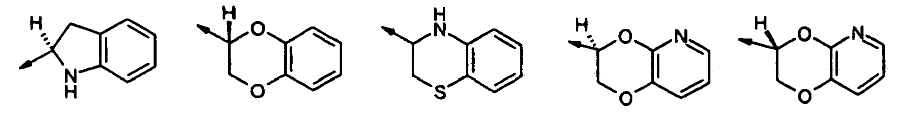 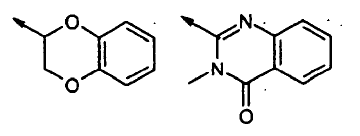 → is the point of attachment;

### (b) is non aromatic

1,1,3-trioxo-1,2,3,4-tetrahydrol *l*⁶-benzo[1,4] thiazin-6-yl, benzo[1,3]dioxol-5-yl, 2,3-dihydro-benzo[1,4]dioxin-6-yl, 2-oxo-2,3-dihydro-benzooxazol-6-yl, 3-substituted-3H-benzooxazol-2-one-6-yl, 3-substituted-3H-benzooxazole-2-thione-6-yl, 3-substituted-3H-benzothiazol-2-one-6-yl, 4H-benzo[1,4]oxazin-3-one-6-yl (3-oxo-3,4-dihydro-2H-benzo[1,4]oxazin-6-yl), 4H-benzo[1,4]thiazin-3-one-6-yl (3-oxo-3,4-dihydro-2H-benzo[1,4]thiazin-6-yl), 4H-benzo[1,4]oxazin-3-one-7-yl, 4-oxo-2,3,4,5-tetrahydro-benzo[b][1,4]thiazepine-7-yl, 5-oxo-2,3-dihydro-5H-thiazolo[3,2-a]pyrimidin-6-yl, 1H-pyrido[2,3-b][1,4]thiazin-2-one-7-yl (2-oxo-2,3-dihydro-1H-pyrido[2,3-b]thiazin-7-yl), 2,3-dihydro-1H-pyrido[2,3-b][1,4]thiazin-7-yl, 2-oxo-2,3-dihydro-1H-pyrido[3,4-b]thiazin-7-yl, 2,3-dihydro-[1,4]dioxino[2,3-b]pyridin-6-yl, 2,3-dihydro-[1,4]dioxino[2,3-c]pyridin-7-yl, 2,3-dihydro-[1,4]dioxino[2,3-b]pyridin-7-yl, 3,4-dihydro-2H-benzo[1,4]oxazin-6-yl, 3,4-dihydro-2H-benzo[1,4]thiazin-6-yl, 3-oxo-3,4-dihydro-2H-pyrido[3,2-b][1,4]oxazin-6-yl, 3,4-dihydro-2H-pyrido[3,2-b][1,4]thiazin-6-yl, 3-oxo-3,4-dihydro-2H-pyrido[3,2-b][1,4]thiazin-6-yl, 3,4-dihydro-1H-quinolin-2-one-7-yl, 3,4-dihydro-1H-quinoxalin-2-one-7-yl, 6,7-dihydro-4H-pyrazolo[1,5-a]pyrimidin-5-one-2-yl, 1,2,3,4-tetrahydro-[1,8]naphthyridin-7-yl, 2-oxo-3,4-dihydro-1*H*-[1,8]naphthyridin-6-yl, 6-oxo-6,7-dihydro-5H-8-thia-1,2,5-triaza-naphthalen-3-yl(6-oxo-6,7-dihydro-5*H-*pyridazino[3,4-b][1,4]thiazin-3-yl), 2-oxo-2,3-dihydro-1H-pyrido[3,4-b][1,4]oxazin-7-yl, 2-oxo-2,3-dihydro-1H-pyrido[2,3-b][1,4]oxazin-7-yl, 6,7-dihydro-[1,4]dioxino[2,3-d]pyrimidin-2-yl, [1,3]oxathiolo[5,4-*c*]pyridin-6-yl, 3,4-dihydro-2*H*-pyrano[2,3-*c*]pyridine-6-yl, 2,3-dihydro[1,4]oxathiino[2,3-*c*]pyridine-7-yl, 2,3-dihydro-1-benzofuran-5-yl, 6,7-dihydro[1,4]dioxino[2,3-c]pyridazin-3-yl, 6,7-dihydro[1,4]oxathiino[2,3-c]pyridazin-3-yl, 2-substituted 1*H*-pyrimido[5,4-*b*][1,4]oxazin-7(6*H*)-one, 2-substituted 5,6-dihydropyrido[2,3-*d*]pyrimidin-7(1*H*)-one, 7-oxo-1,5,6,7-tetrahydro[1,8]naphthyridin-2-yl. 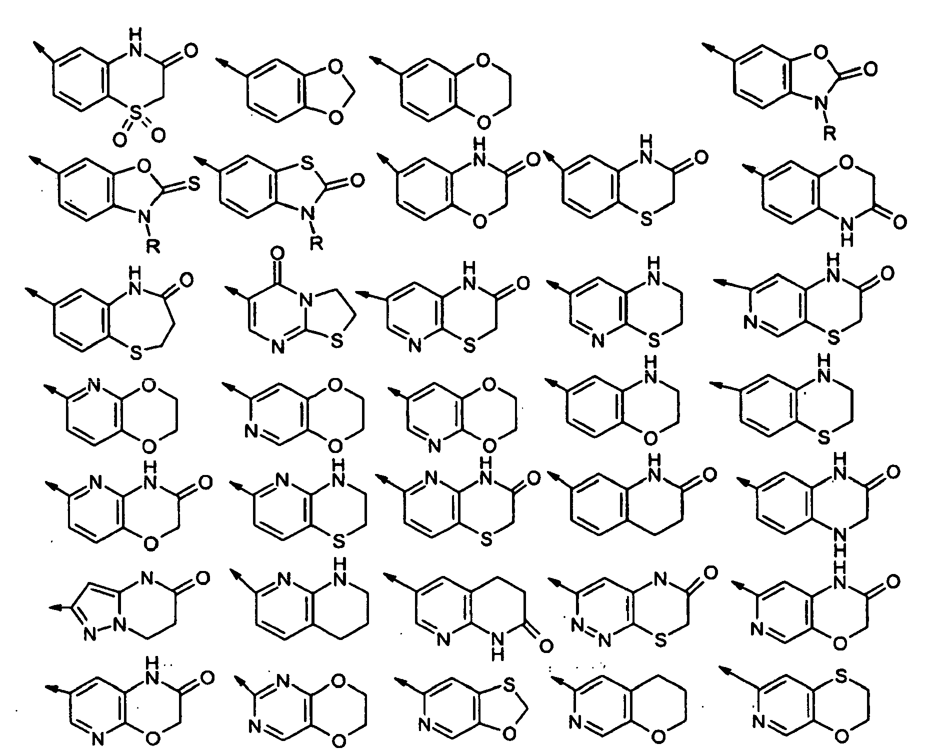 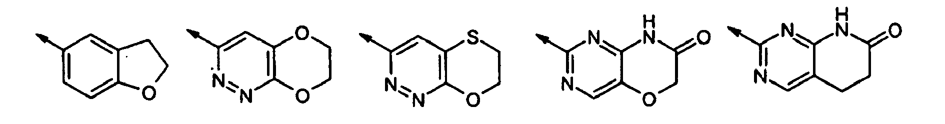 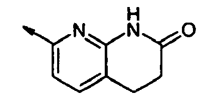 → is the point of attachment;

In some embodiments R¹³ is H if in ring (a) or in addition (C₁₋₄)alkyl such as methyl or isopropyl when in ring (b). More particularly, in ring (b) R¹³ is H when NR¹³ is bonded to X³ and (C₁₋₄)alkyl when NR¹³ is bonded to X⁵.

In futher embodiments R¹⁴ and R¹⁵ are independently selected from hydrogen, halo, hydroxy, (C₁₋₄) alkyl, (C₁₋₄)alkoxy, nitro and cyano. More particularly R¹⁵ is hydrogen.

More particularly each R¹⁴ is selected from hydrogen, chloro, fluoro, hydroxy, methyl, methoxy, nitro and cyano. Still more particularly R¹⁴ is selected from hydrogen, fluorine or nitro.

Most particularly R¹⁴ and R¹⁵ are each H.

Particular groups R⁵ include:
[1,2,3]thiadiazolo[5,4-b]pyridin-6-yl
1H-pyrrolo[2,3-b]pyridin-2-yl
2,3-dihydro-[1,4]dioxino[2,3-b]pyridin-6-yl
2,3-dihydro-[1,4]dioxino[2,3-b]pyridin-7-yl
2,3-dihydro-[1,4]dioxino[2,3-c]pyridin-7-yl
2,3-dihydro-benzo[1,4]dioxin-6-yl
2-oxo-2,3-dihydro-1H-pyrido[2,3-b][1,4]oxazin-7-yl
2-oxo-2,3-dihydro-1H-pyrido[2,3-b][1,4]thiazin-7-yl
3,4-dihydro-2H-benzo[1,4]oxazin-6-yl
3-methyl-2-oxo-2,3-dihydro-benzooxazol-6-yl
3-oxo-3,4-dihydro-2H-benzo[1,4]oxazin-6-yl
3-oxo-3,4-dihydro-2H-pyrido[3,2-b][1,4]oxazin-6-yl
3-oxo-3,4-dihydro-2H-benzo[1,4]thiazin-6-yl (4H-benzo[1,4] thiazin-3-one-6-yl)
4-oxo-4H-pyrido[1,2-a]pyrimidin-2-yl
6-nitro-benzo[1,3]dioxol-5-yl
7-fluoro-3-oxo-3,4-dihydro-2H-benzo[1,4] oxazin-6-yl
8-hydroxy-1-oxo-1,2-dihydro-isoquinolin-3-yl
8-hydroxyquinolin-2-yl
benzo[1,2,3]thiadiazol-5-yl
benzo[1,2,5]thiadiazol-5-yl
benzothiazol-5-yl
thiazolo-[5,4-b]pyridin-6-yl
3-oxo-3,4-dihydro-2*H*-pyrido[3,2-*b*][1,4]thiazin-6-yl
7-chloro-3-oxo-3,4-dihydro-2*H*-pyrido[3,2-*b*][1,4]thiazin-6-yl
7-chloro-3-oxo-3,4-dihydro-2*H*-pyrido[3,2-*b*][1,4]oxazin-6-yl
7-fluoro-3-oxo-3,4-dihydro-2*H*-pyrido[3,2-*b*][1,4]thiazin-6-yl
2-oxo-2,3-dihydro-1*H*-pyrido[3,4-*b*][1,4]thiazin-7-yl
[1,3]oxathiolo[5,4-*c*]pyridin-6-yl
3,4-dihydro-2*H*-pyrano[2,3-*c*]pyridin-6-yl
5-carbonitro-2,3-dihydro-1,4-benzodioxin-7-yl
2,3-dihydro[1,4]oxathiino[2,3-*c*]pyridin-7-yl
2,3-dihydro-1-benzofuran-5-yl
6,7-dihydro[1,4]dioxino[2,3-*c*]pyridazin-3-yl
6,7-dihydro[1,4]oxathiino[2,3-*c*]pyridazin-3-yl
2-substituted 1H-pyrimido[5,4-b][1,4]oxazin-7(6H)-one
2-substituted 4-chloro-1H-pyrimido[5,4-b][1,4]oxazin-7(6H)-one
2-substituted 5,6-dihydropyrido[2,3-d]pyrimidin-7(1H)-one
2-substituted 4-chloro-5,6-dihydropyrido[2,3-d]pyrimidin-7(1H)-one
2-substituted 4-methyl-5,6-dihydropyrido[2,3-d]pyrimidin-7(1H)-one
2-substituted 4-methyloxy-5,6-dihydropyrido[2,3-d]pyrimidin-7(1H)-one 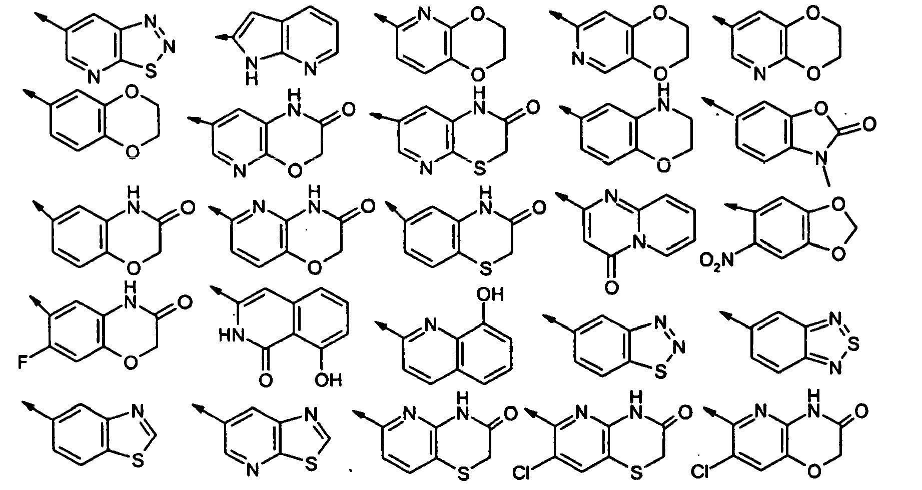 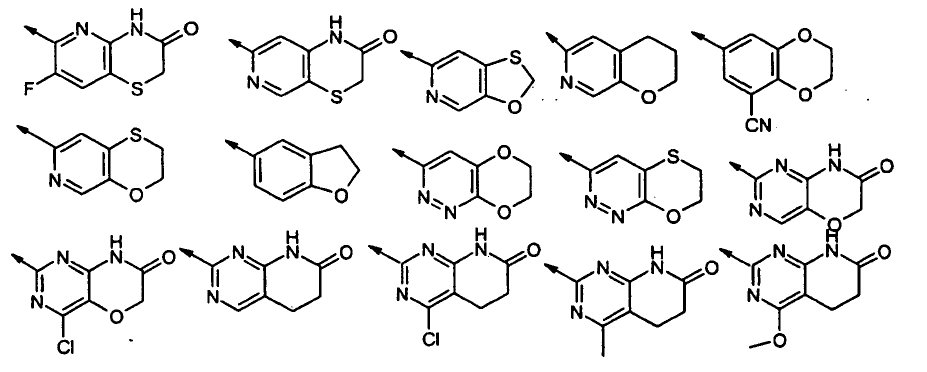 → is the point of attachment;
especially
3-oxo-3,4-dihydro-2H-pyrido[3,2-b][1,4]oxazin-6-yl
3-oxo-3,4-dihydro-2*H*-pyrido[3,2-*b*][1,4]thiazin-6-yl
6,7-dihydro[1,4]dioxino[2,3-*c*]pyridazin-3-yl
6,7-dihydro[1,4]oxathiino[2,3-*c*]pyridazin-3-yl
2,3-dihydro-[1,4]dioxino[2,3-*c*]pyridin-7-yl
[1,3]oxathiolo[5,4-*c*]pyridin-6-yl
2,3-dihydro[1,4]oxathiino[2,3-*c*]pyridin-7-yl
2-substituted 1H-pyrimido[5,4-b][1,4]oxazin-7(6H)-one
2-substituted 5,6-dihydropyrido[2,3-*d*]pyrimidin-7(1*H*)-one 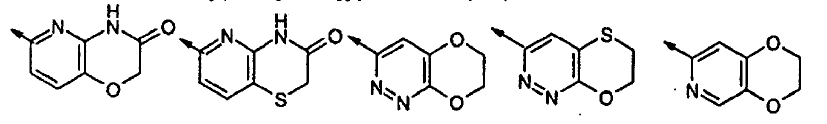 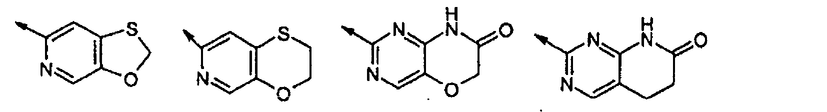 → is the point of attachment.

When used herein, the term "alkyl" includes groups having straight and branched chains, for instance, methyl, ethyl, n-propyl, iso-propyl, n-butyl, iso-butyl, sec-butyl, t-butyl, pentyl and hexyl. The term 'alkenyl' should be interpreted accordingly.

Halo or halogen includes fluoro, chloro, bromo and iodo.

Haloalkyl moieties include 1-3 halogen atoms.

Compounds within the invention contain a heterocyclyl group and may occur in two or more tautomeric forms depending on the nature of the heterocyclyl group; all such tautomeric forms are included within the scope of the invention.

Some of the compounds of this invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Furthermore, it will be understood that phrases such as "a compound of formula (I) or a pharmaceutically acceptable salt, solvate or N-oxide thereof" are intended to encompass the compound of formula (I), an N-oxide of formula (I), a pharmaceutically acceptable salt of the compound of formula (I), a solvate of formula (I), or any pharmaceutically acceptable combination of these. Thus by way of non-limiting example used here for illustrative purpose, "a compound of formula (I) or a pharmaceutically acceptable salt or solvate thereof' may include a pharmaceutically acceptable salt of a compound of formula (I) that is further present as a solvate.

Since the compounds of formula (I) are intended for use in pharmaceutical compositions it will readily be understood that in particular embodiments they are provided in substantially pure form, for example at least 60% pure, more suitably at least 75% pure and particularly at least 85%, especially at least 98% pure (% are on a weight for weight basis). Impure preparations of the compounds may be used for preparing the more pure forms used in the pharmaceutical compositions; these less pure preparations of the compounds should contain at least 1%, more suitably at least 5% and more particularly from 10 to 59% of a compound of the formula (I) or pharmaceutically acceptable salt, solvate and/or N-oxide thereof.

Particular compounds according to the invention include those mentioned in the examples and their pharmaceutically acceptable N-oxides, salts and solvates.

Pharmaceutically acceptable salts of the above-mentioned compounds of formula (I) include the acid addition or quaternary ammonium salts, for example their salts with mineral acids e.g. hydrochloric, hydrobromic, sulphuric nitric or phosphoric acids, or organic acids, e.g. acetic, fumaric, succinic, maleic, citric, benzoic, p-toluenesulphonic, methanesulphonic, naphthalenesulphonic acid or tartaric acids. Compounds of formula (I) may also be prepared as the N-oxide. The invention extends to all such derivatives.

Certain of the compounds of formula (I) may exist in the form of optical isomers, e.g. diastereoisomers and mixtures of isomers in all ratios, e.g. racemic mixtures. The invention includes all such forms, in particular the pure isomeric forms. For example the invention includes enantiomers and diastereoisomers at the attachment point of NR² and R³. The different isomeric forms may be separated or resolved one from the other by conventional methods, or any given isomer may be obtained by conventional synthetic methods or by stereospecific or asymmetric syntheses. Certain compounds of formula (I) may also exist in polymorphic forms and the invention includes such polymorphic forms.

In a further aspect of the invention there is provided a process for preparing compounds of formula (I), and pharmaceutically acceptable salts, solvates and/or N-oxides thereof, which process comprises cyclising a compound of formula (IIA): 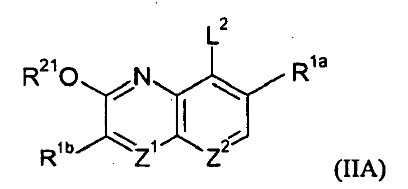 in which R²¹ is (C₁₋₆)alkyl such as methyl, L² is LCH₂CH(OH)- or CH₂=CH- where L is -A-N(R²⁰)R^{2'} or a group convertible thereto, where R²⁰ is UR⁵ or a group convertible thereto and R^{2'} is R² or a group convertible thereto, wherein Z¹, Z², A, R^{1a}, R^{1b}, R², U and R⁵ are as defined in formula (I),
to give a compound of formula (IIB): 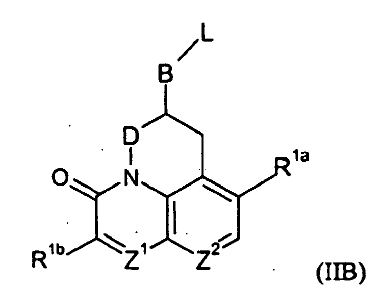 and and thereafter optionally or as necessary converting L to -A-NR²-UR⁵, interconverting any variable groups, and/or forming a pharmaceutically acceptable salt, solvate or N-oxide thereof.

Where L² is LCH₂CH(OH), the cyclisation reaction is effected by treatment of the compound of formula (IIA) with an activating agent such as trifluoromethanesulphonic anhydride, methanesulphonyl chloride, p-toluenesulphonyl chloride, methanesulfonic anhydride or p-toluene sulfonic anhydride and an organic base such as triethylamine or diisopropylethylamine. Mesylate or tosylate preparation takes place under standard conditions and the compound of formula (IIB) forms *in situ.*

Where L² is CH₂=CH- cyclisation can be effected by treatment of the olefin with m-chloroperbenzoic acid to generate the epoxide which cyclises *in situ* to give a compound of formula (IIB) in which L is OH. Alternatively treatment of the olefin with N-bromosuccinimide in water gives a compound of formula (IIB) in which L is Br.

The cyclisation produces a mixture of compounds of formula (IIB) where (i) B is CH₂ and D is a bond, and (ii) B is a bond and D is CH₂. The relative proportions of the resultant 5- and 6- membered ring products will be dependent on the particular substrates and the precise reaction conditions employed (for example the solvent or activating group). In cases where a mixture of products arise from the cyclisation step, chromatography may be used to separate the isomers.

L may be a hydroxy group which can be oxidised to the aldehyde by conventional means such as 1,1,1-tris-(acetyloxy)-1,1-dihydro-1,2-benziodooxol-3-(1H)-one for reductive alkylation with HA-N(R²⁰)R^{2'} under conventional conditions (see for examples Smith, M.B.; March, J.M. Advanced Organic Chemistry, Wiley-Interscience).

Alternatively L may be bromo which can be alkylated with HA-N(R²⁰)R^{2'} under conventional conditions.

Conveniently one of R²⁰ and R^{2'} is an N-protecting group, such as such as t-butoxycarbonyl, benzyloxycarbonyl or 9-fluorenylmethyloxycarbonyl. This may be removed by several methods well known to those skilled in the art (for examples see "Protective Groups in Organic Synthesis, T.W. Greene and P.G.M. Wuts, Wiley-Interscience, 1999), for example conventional acid hydrolysis. The invention further provides compounds of formula (IIB) in which R²⁰ is hydrogen.

The free amine of formula (IIB) in which R²⁰ is hydrogen may be converted to NR²UR⁵ by conventional means such as amide or sulphonamide formation with an acyl derivative R⁵COW or R⁵SO₂W, for compounds where U is CO or SO₂ or, where U is CH₂, by alkylation with an alkyl halide R⁵CH₂-halide in the presence of base, acylation/reduction with an acyl derivative R⁵COW or reductive alkylation with an aldehyde R⁵CHO under conventional conditions (see for examples Smith, M.B.; March, J.M. Advanced Organic Chemistry, Wiley-Interscience). The appropriate reagents containing the required R⁵ group are known compounds or may be prepared analogously to known compounds, see for example WO02/08224, WO02/50061, WO02/56882, WO02/96907, WO2003087098, WO2003010138, WO2003064421, WO2003064431, WO2004002992, WO2004002490, WO2004014361, WO2004041210, WO2004096982, WO2002050036, WO2004058144, WO2004087145, W02004/035569, WO2004/089947, WO2003082835, WO2002026723, WO06002047, WO06014580, WO06010040, WO06017326, WO06012396, WO06017468 and WO06020561, WO06132739, WO06134378, WO06137485 and EP0559285.

Where R⁵ contains an NH group, this may be protected with a suitable N-protecting group such as t-butoxycarbonyl, benzyloxycarbonyl or 9-fluorenylmethyloxycarbonyl during the coupling of the R⁵ derivative with the free amine of formula (IIB). The protecting group may be removed by conventional methods, such as by treatment with trifluoroacetic acid.

The compound of formula (IIA) may be prepared by the following Scheme 1: 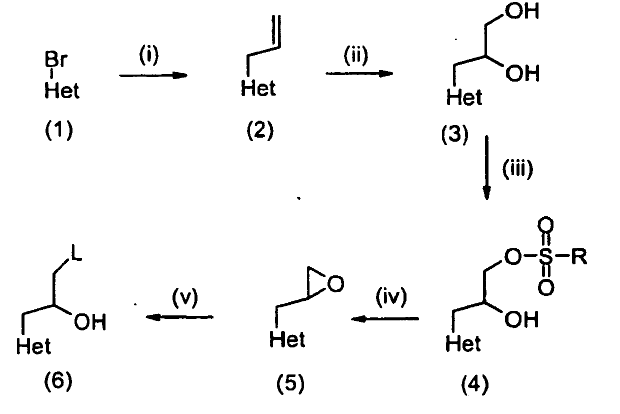 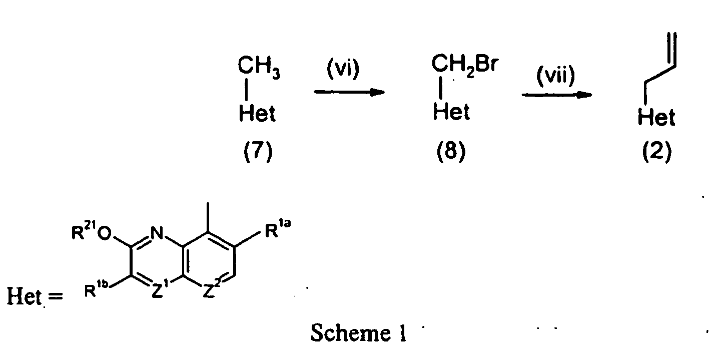
(i) Heterocyclic bromides of type (1) can be converted to allyl derivatives (2) by reaction with allyl boron reagents (see Kotha, Sambasivarao et al, Synlett (2005) 12, 1877-1880), allyl tin reagents (see Bolm, Carsten et al, Journal of Organic Chemistry (2005) 70(6), 2346-2349), allyl magnesium Grignard reagents (see Hourdin, Marie et al, Journal of Combinatorial Chemistry (2005) 7(2), 285-297) or allyl indium reagents (see Lee, Phil, Advanced Synthesis & Catalysis (2004) 346(13-15), 1641-1645). Alternatively, the bromide can be metallated and the corresponding organometallic species (magnesium (Beinhoff, Matthias, Synthesis (2003) (1), 79-90); lithium (Lau, Stephen, Canadian Journal of Chemistry (2001) 79(11) 1541-1545); copper (Inoue, Atsushi, Journal of Organic Chemistry (2001) 66(12), 4333-4339)) may be alkylated with allyl bromide to give (2).
(ii) Oxidation of allyl derivatives (2) to diols (3) may be accomplished with standard reagent systems such as osmium tetroxide/N-methyl morpholine-N-oxide (Zheng, Tao et al, Journal of the American Chemical Society (2005) 127(19), 6946-6947). It will be appreciated that such oxidations can be performed chirally using appropriate chiral oxidising systems such as AD mix alpha or beta (see Pinard, E et al, Bioorganic & Medicinal Chemistry Letters (2001), 11(16), 2173-2176). This provides compounds of formula (IIA) where L² is LCH₂CH(OH).
(iii) The primary alcohol of diols (3) can be selectively activated towards displacement by reaction with sulphonyl halides (see Wallner, Sabine R, Organic & Biomolecular Chemistry (2005) 3(14), 2652-2656) and this selectivity may be enhanced by the use of catalytic quantities of dibutyltin oxide (see Boger, Dale, Journal of the American Chemical Society (1996) 118(9), 2301-2).
(iv) The sulphonates (4) may be cyclised to give epoxides (5) most conveniently under mildly basic conditions such as potassium carbonate in methanol (Gooding, Owen, Synthetic Communications (1995), 25(8), 1155-66) although strongly basic conditions such as butyl lithium may be used (Chong, J. Michael, Tetrahedron Letters (1994), 35(39), 7197-200). As an alternative to the epoxides, the diols may be activated as cyclic suphates (see Bonini, Carlo, Tetrahedron (2005) 61(27), 6580-6589). It will be appreciated that olefins (2) may be directly oxidised to epoxides (5) using ruthenium-based systems (see Yudin, Andrei K, Journal of Organic Chemistry (2001) 66(13), 4713-4718 and references therein) or by using meta chloroperbenzoic acid (Florez-Alvarez, Jose, Tetrahedron Letters (2002) 43(1), 171-174) or by the use of dimethyldioxirane (Bhoga, Umadevi, Tetrahedron Letters (2005) 46(31), 5239-5242).
(v) The epoxides may be reacted with amine HA-N(R²⁰)R² in solvents such as DMF or ethanol in the presence of bases such as sodium or potassium carbonate, disodium hydrogen phosphate or triethylamine to give the ethanolamines (6) (compounds of formula (IIA) in which L² is LCH₂CH(OH) and L is -A-N(R²⁰)R^{2')}. It is not essential or even necessary to isolate epoxide (5) as reaction of sulphonates (4) and amines with, for example, potassium carbonate as base, can afford the ethanolamines (6) with presumed *in situ* formation and reaction of epoxides (5) (see Bonini, Carlo, Tetrahedron (2005), 61(27), 6580-6589).
(vi) Alternatively, allyl derivatives (2) may be prepared from methyl heterocycles (7) by bromination with N-bromosuccinimde with catalysis from dibenzoyl peroxide or 2,2'-azodiisobutyronitrile (see Aguirre, Gabriela et al, Bioorganic & Medicinal Chemistry (2005) 13(23), 6324-6335) to give bromomethyl derivatives (8).
(vii) The bromomethyl derivatives (8) can be converted to the allyl analogues (2) by a variety of reagents, including vinyltributyltin (see Crawforth, Catherine et al, Tetrahedron Letters (2004) 45(3), 461-465), vinylmagnesium bromide with copper (I) iodide catalysis (see Esumi, Tomoyuki et al, Bioorganic & Medicinal Chemistry Letters (2004) 14(10), 2621-2625) and organoboron reagents (for a related example see Langle, Sandrine et al, Tetrahedron Letters (2003) 44(52), 9255-9258)

Intermediates such (1) and (7) may be prepared by the following schemes.

For the quinoxalinone system (Z¹=N), Scheme 2 may be employed to give a methyl derivative (7): 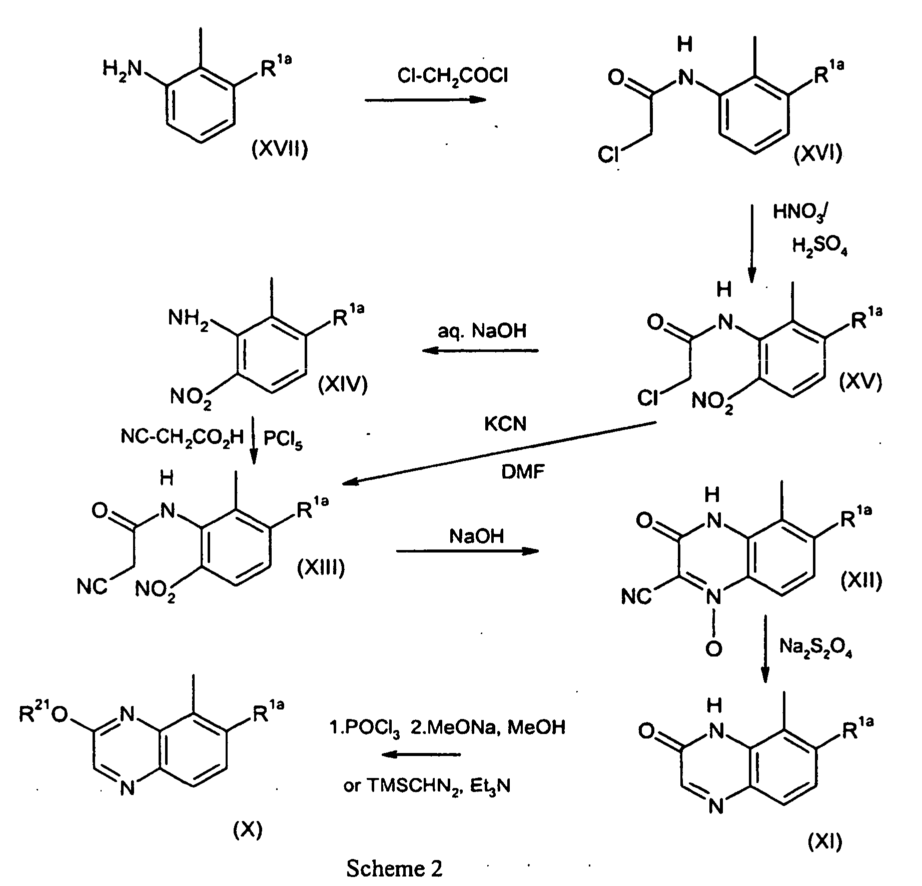 NBS = N-bromosuccinimide
TMSCl = chlorotrimethylsilane
TMSCHN₂ = (trimethylsilyl)diazomethane

Aniline (XVII) is converted by acylation to the chloroacetamide (XVI), which is nitrated to give (XV) and then hydrolysed to give the nitroaniline (XIV). This is converted into the cyanoacetamide (XIII) by treatment with cyanoacetic acid and phosphorus pentachloride (by the method of S.T.Hazeldine et al, J. Med. Chem., (2002) 44, 1758). Alternatively, the nitro-chloroacetamide (XV) may be treated with potassium cyanide to give cyanoacetamide (XIII) directly. Cyclisation with sodium hydroxide in pyridine gives a cyanoquinoxalinone-N-oxide (XII) which is reduced by sodium dithionite with loss of the cyano group to give a compound of formula (XI). This is first chlorinated with phosphorus oxychloride and then treated with sodium methoxide to give (X) or (XI) can be methylated with (trimethylsilyl)diazomethane in the presence of triethylamine . This route is particularly suitable for R^{1a}=F.

For the quinolinone system where Z¹ and Z² are both carbon, Scheme 3 may be employed to give a methyl derivative (7):

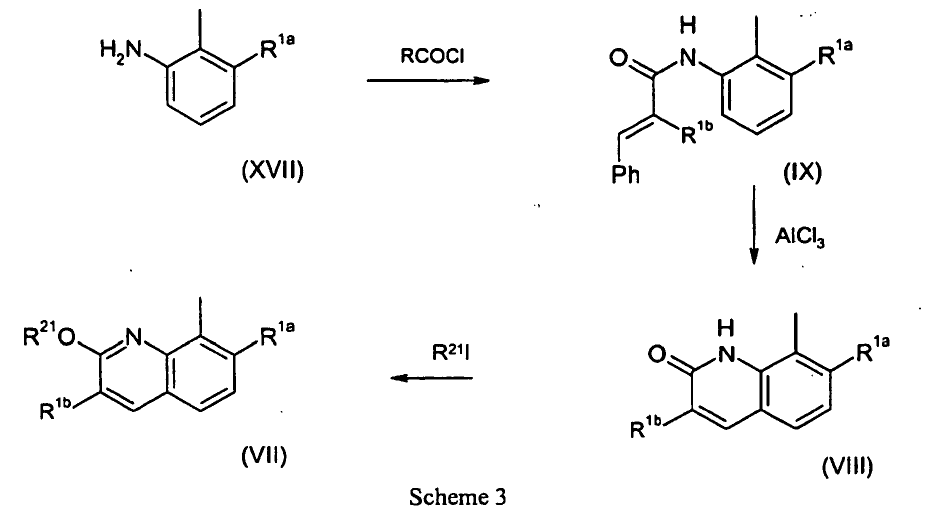

The aniline (XVII) is converted to the cinnamide (XI), which is cyclised with aluminium chloride (with loss of the phenyl moiety - See M.C. Elliot et al. J. Med. Chem. 47 (22) ,5405-5417 (2004), S.R. Inglis et al. Synlett, 5, 898-900 (2004), to give (VIII). This is selectively O-alkylated with e.g. methyl iodide to give (VII).

For the quinolinone system where Z¹ and Z² are both carbon, Scheme 4 may be employed to give a bromo derivative (1):

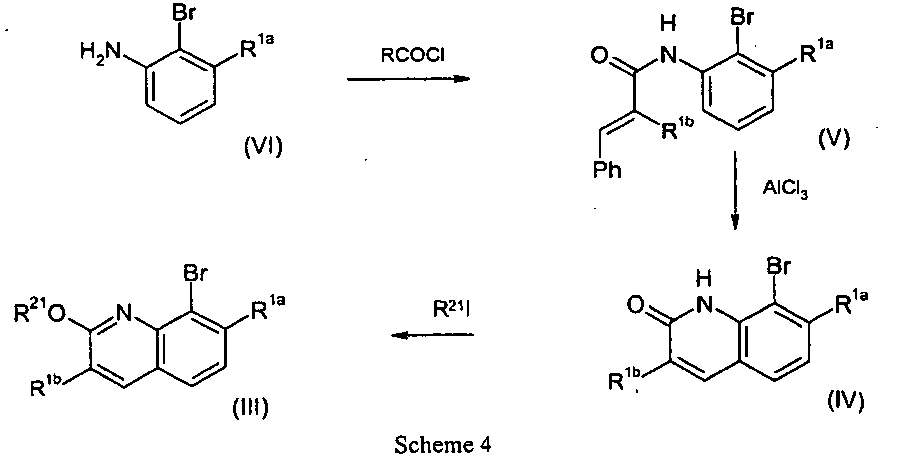

Aniline (VI) can be reacted with cinnamoyl chloride to give (V) which can subsequently be cyclised (for an example of this procedure see Cottet, F.; Marull, M.; Lefebvre, O.; Schlosser, M European Journal of Organic Chemistry (2003), 8, 1559) to give (IV). This can be converted into the bromo-quinoline (III) by o-methylation under standard conditions (see for examples Smith, M.B.; March, J.M. Advanced Organic Chemistry, Wiley-Interscience).

The naphthyridinone bromide (1) where Z¹ and Z² are both N is known in the literature, see for example WO 2004058144.

For the naphthyridone bromide (1) where Z¹ and Z² are both N and R^{1a} is fluorine, Scheme 5 may be employed:

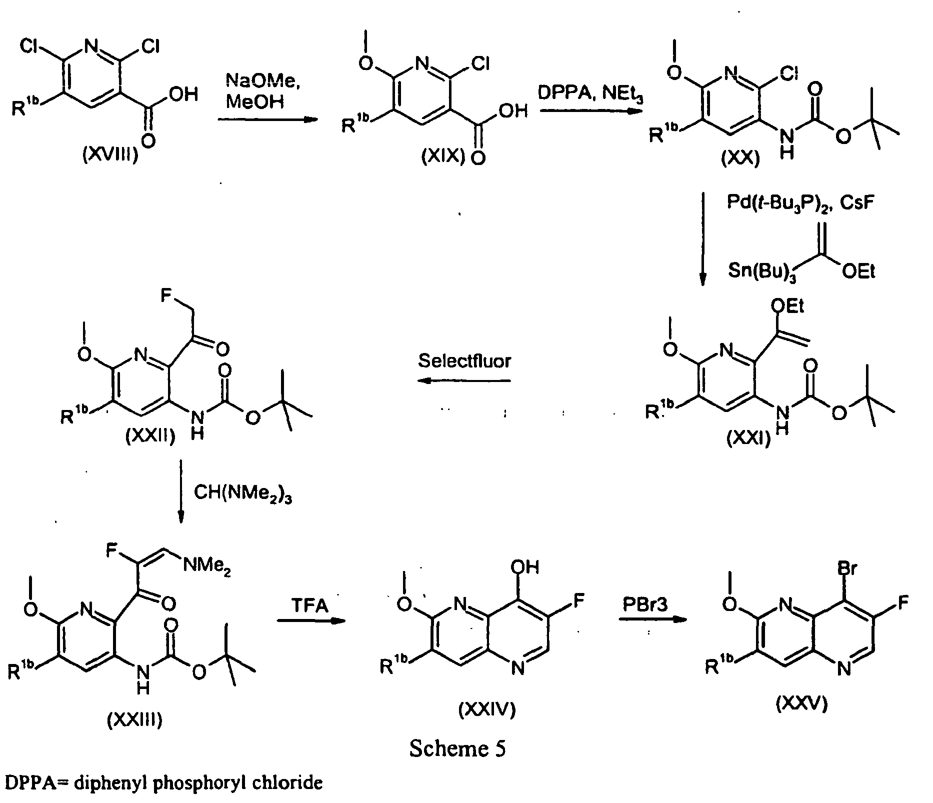

Carboxylic acid (XVIII) is converted to methoxy intermediate (XIX) and then to the protected aminopyridine (XX) via a Curtius type rearrangement under standard conditions (see for examples Smith, M.B.; March, J.M. Advanced Organic Chemistry, Wiley-Interscience). Enol ether (XXI) is formed via a Stille coupling using the procedure of Fu et al (for a reference see Littke, A.F.; Schwartx, L.; Fu, G.C. J. Am. Chem. Soc. 2002, 124, 6343). Reaction of (XXI) with Selectfluor® 1-chloromethyl-4-fluoro-1,4diazoniabicyclo[2.2.2]octane gives fluoro-ketone (XXII) (see for examples Lal, G. S.; Pez, G.P.; Syvret, R.G. Chem. Rev, 1996, 96, 1737). Conversion to the enamino-ketone (XXIII), deprotection to give (XIV) and finally bromination gives intermediate (XXV) under standard conditions (see for examples Smith, M.B.; March, J.M. Advanced Organic Chemistry, Wiley-Interscience). This route is particularly suitable for R^{1b}=F.

Interconversions of Z¹, Z², R^{1a}, R^{1b}, R², A and R⁵ are conventional. In compounds which contain an optionally protected hydroxy group, suitable conventional hydroxy protecting groups which may be removed without disrupting the remainder of the molecule include acyl and alkylsilyl groups. N-protecting groups are removed by conventional methods.

Interconversion of R^{1a} and R^{1b} groups may be carried out conventionally, on compounds of formula (I), (IIA) or (IIB). For example R^{1a} or R^{1b} methoxy is convertible to R^{1a} or R^{1b} hydroxy by treatment with lithium and diphenylphosphine (general method described in Ireland et al, J. Amer. Chem. Soc., 1973, 7829) or HBr. Alkylation of the hydroxy group with a suitable alkyl derivative bearing a leaving group such as halide, yields R^{1a} or R^{1b} substituted alkoxy. R^{1a} halogen is convertible to other R^{1a} by conventional means, for example to hydroxy, alkylthiol (via thiol) and amino using metal catalysed coupling reactions, for example using copper as reviewed in Synlett (2003), 15, 2428-2439 and Angewandte Chemie, International Edition, 2003, 42(44), 5400-5449. R^{1b} halo such as bromo may be introduced by the method of M. A. Alonso et al, Tetrahedron 2003, 59(16), 2821. R^{1a} or R^{1b} halo such as bromo may be converted to cyano by treatment with copper (I) cyanide in N,N-dimethylformamide. R^{1a} or R^{1b} carboxy may be obtained by conventional hydrolysis of R^{1a} or R^{1b} cyano, and the carboxy converted to hydroxymethyl by conventional reduction.

Compounds of formula HA-N(R²⁰)R^{2'} are known compounds or may be prepared analogously to known compounds, see for example WO2004/035569, WO2004/089947, WO02/08224, WO02/50061, WO02/56882, WO02/96907, WO2003087098, WO2003010138, WO2003064421, WO2003064431, WO2004002992, WO2004002490, WO2004014361, WO2004041210,WO2004096982, WO2002050036, WO2004058144, WO2004087145, WO2003082835, WO2002026723, WO06002047, W006014580, WO06134378 and WO06137485.

As shown in Scheme 6, the hydroxy-aminomethylpyrrolidines of formula (xiii) (HA-NH(R²⁰), A is (ii), X is CR⁴R⁸, W¹ is a bond, W² and W³ are both CH₂, R⁴ and R⁷ are H and R⁸ is OH) can be prepared from doubly protected chiral intermediate (xvi), separated by preparative HPLC. The benzyloxycarbonyl protecting group is removed by hydrogenation to give (xv) and the amino function converted to a trifluoroacetamide (xiv). The t-butoxycarbonyl (Boc) protecting group is removed with HCl to give the pyrrolidine hydrochloride salt (xiii).

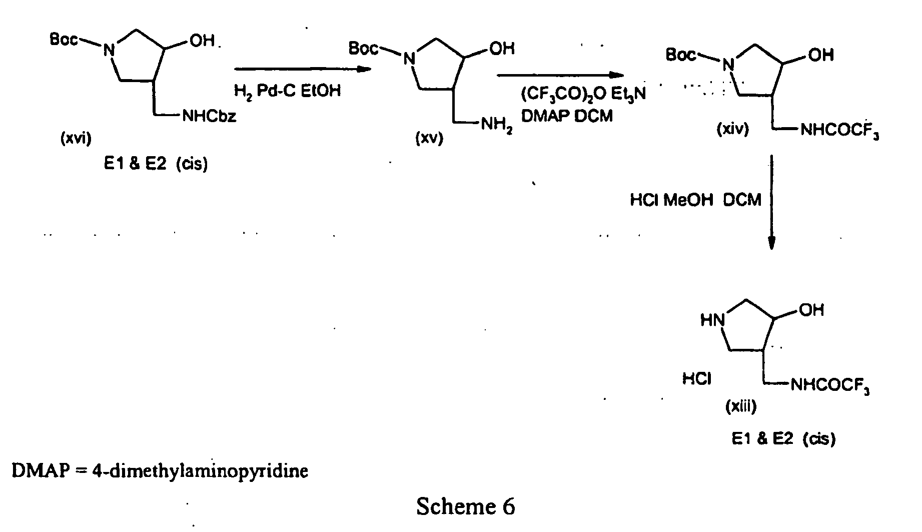

The intermediate (xvi) may be prepared by the general method of Scheme 7:

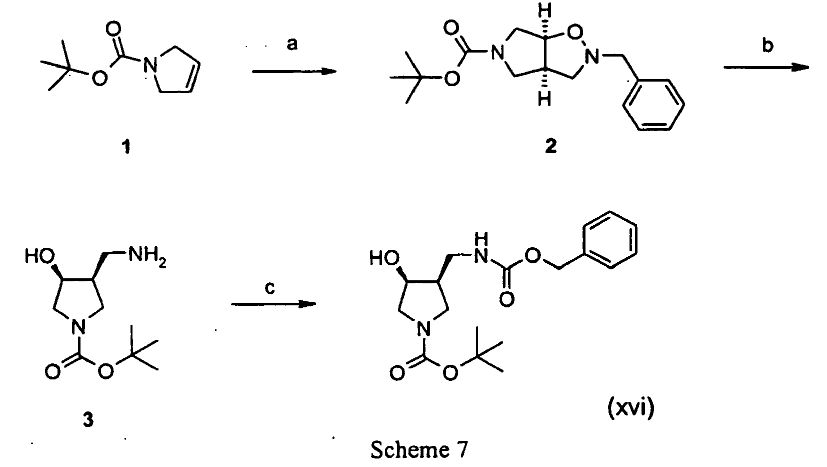

Reagents and conditions: (a) *N*-Hydroxybenzylamine hydrochloride, paraformaldehyde, toluene, EtOH, 80°C; (b) Pd(OH)₂, H₂ (50psi), MeOH, room temperature; (c) Benzyloxycarbonyl-succinimide, Et₃N, dichloromethane, room temperature.

In Scheme 8 the aminomethylpyrrolidine of formula (xvii) (HA-NH(R²⁰), A is (ii), X is CR⁴R⁸, W¹ is a bond, W² and W³ are both CH₂, R⁴, R⁷ and R⁸ are all H) can be prepared from commercially available Boc-protected aminomethylpyrrolidine, and converted to the trifluoroacetamide.

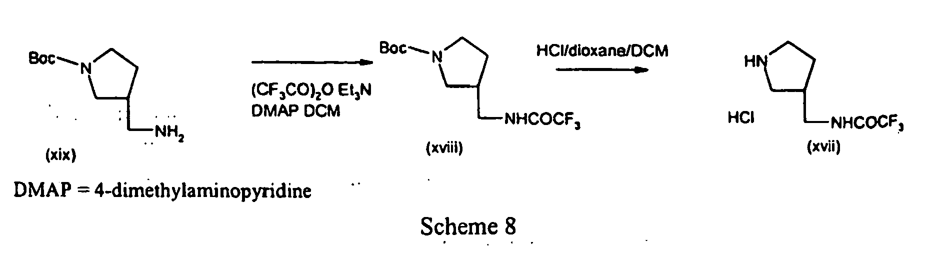

The aminomethylmorpholine intermediate of formula (xxi) (HA-NH(R²⁰), A is (ii), X is O, W¹, W² and W³ are each CH₂) may be prepared from a chiral dichlorobenzyl intermediate (xxiii) (WO2003082835) (Scheme 9) by first protecting the amino function with a Boc-protecting group (xxii), removing the dichlorobenzyl group by hydrogenation to give (xxi), protecting the morpholine N-atom with a benzyloxycarbonyl group (to allow purification by chromatography) (xx), and hydrogenation to afford the required morpholine derivative (xxi).

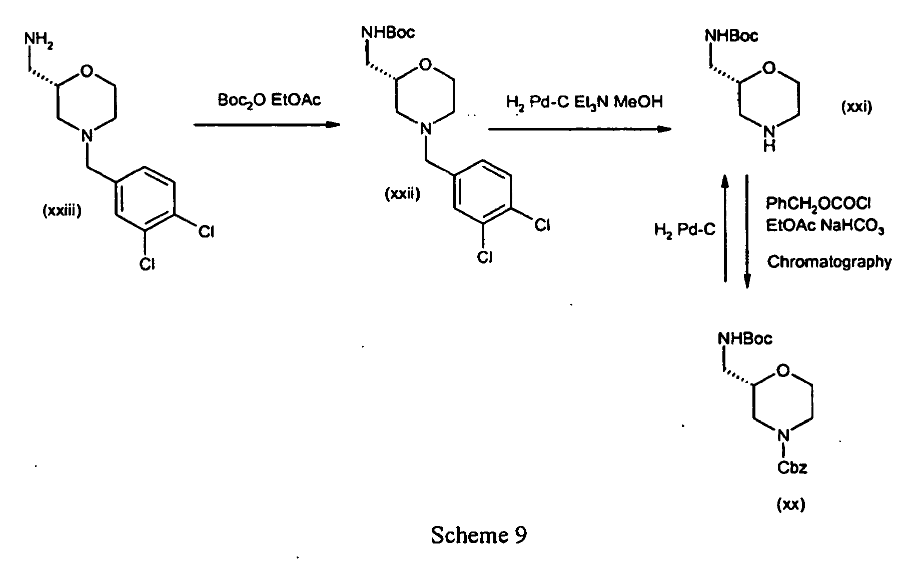

A method to prepare the pyrimidinyloxazinone unit R⁵ (C), where Y³ = O, R¹⁰ = H) is illustrated in Scheme 10.

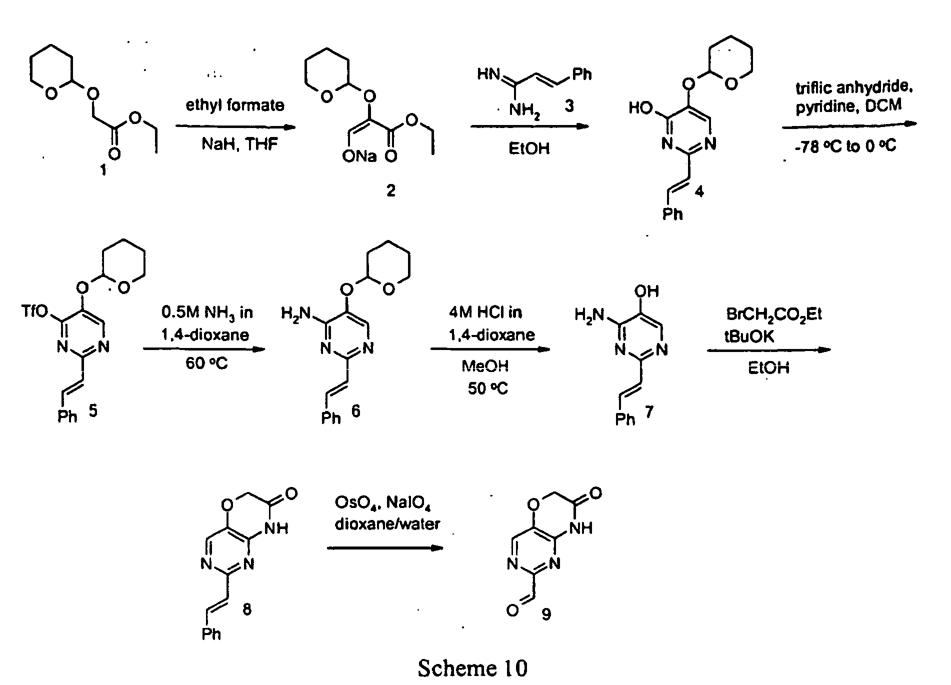

A suitably protected ethyl glycolate (THP-protected in this example, **1)** is formylated using ethyl formate and a base such as NaH in THF or diethyl ether. The intermediate formyl enolate **2** is then directly reacted with an amidine, in this case the (2E)-3-phenyl-2-propenimidamide **3,** giving the pyrimidinone **4.** Pyrimidinone **4** is converted to a trifluoromethansulfonate ester **(5)** which is then reacted with ammonia in a suitable solvent, such as 1,4-dioxane, providing amine **6.** The amino alcohol **7** is then obtained by removing the THP-protecting group of **6** with acid in methanol. Treatment of 7 with a base and an ester of a halo-acetate in an alcohol solvent such as absolute ethanol, provides the bicyclic intermediate **8** directly. This transformation may be accomplished using a base such as potassium tert-butoxide and the alkylating agent ethyl bromoacetate. An amine base such as triethylamine may also be employed as an alternative to the alkoxide base illustrated herein (for similar examples see N.V. Sazonov and T.S. Safonova, Khimiya Geterotsiklicheskikh Soedinenii, 1971, 1285-1288). The final aldehyde intermediate **9** is then obtained via oxidative cleavage of the phenylethenyl side chain. One method to achieve this is by reacting **8** with NaIO₄, in a mixture of 1,4-dioxane-water, with a catalytic amount of OsO₄. Other methods, such as ozonolysis, may also be suitable to achieve the desired transformation.

Pyrimidine dihydropyridone aldehyde (R⁵ (C) where Y³ = CH₂ and R¹⁰ = Cl) may be prepared as illustrated in Scheme 11.

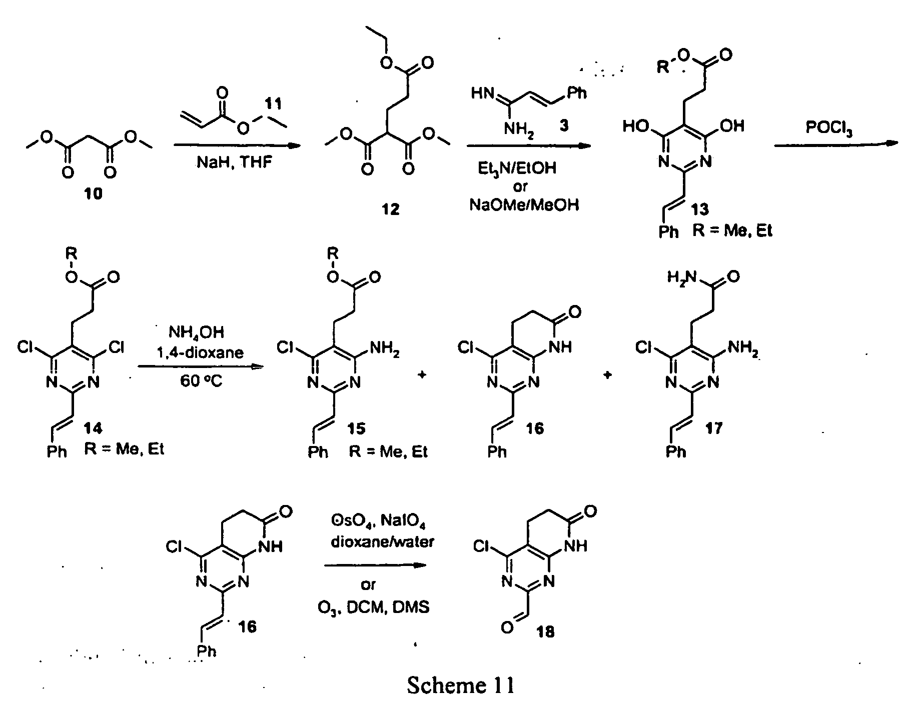

By reacting the anion of dimethyl malonate **(10)** with ethyl acrylate **(11),** the triester **12** is obtained. Condensing **12** with (2E)-3-phenyl-2-propenimidamide **(3),** in the presence of a base, leads to the dihydroxypyrimidine **13.** Triethylamine in EtOH can be used to carry out this transformation, however the preferred conditions utilize NaOMe in MeOH. It should be noted that under these latter conditions the methyl ester of **13** (R = Me) is obtained whereas the ethyl ester is preserved using the former conditions. Either ester form, methyl or ethyl, can be used to carry out the remaining steps of the synthesis. Treating **13** with POCl₃ provides the dichloropyrimidine **14.** Heating **14** in a sealed tube in the presence of NH₄OH usually yields a mixture of the components **15, 16,** and **17** with **15** and **16** predominating. Subsequently, intermediate **15** can be converted to **16** by treating with K₂CO₃ in MeOH. In addition, **17** can be recycled to **15** (R = Et) by treatment with ethanolic HCl. The preparation of the aldehyde **18** is then completed via oxidative cleavage of the olefin side chain of **16** using either OsO₄ and NaIO₄, or by ozonolysis.

Scheme 12 illustrates one convenient method to remove the chlorine substituent found in **18** in order to obtain the des-chloro aldehyde **20** (R⁵ (C) where Y³ = CH₂ and R¹⁰ = H). This can be achieved by first protecting the aldehyde group of **18** by forming the dimethyl acetal using p-toluene sulfonic acid (p-TsOH) and MeOH, providing **19.** The chlorine is then be removed by hydrogenation using Pd-C catalysis under an atmosphere of H₂. Treatment with aqueous acid, such as TFA and water, once again liberates the aldehyde group, thus providing **20.**

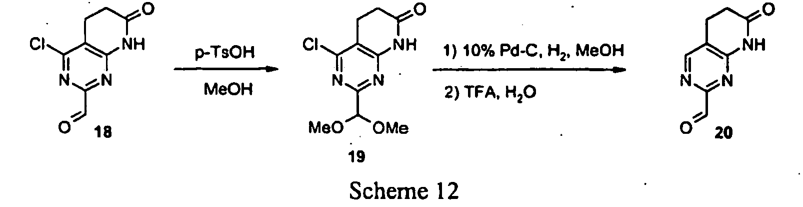

Scheme 13 illustrates a method to prepare analogs incorporating alternative substituents at the 4-position on the pyrimidine ring, for example for R⁵ (C) where Y³ = CH₂ and R¹⁰ = OMe or Me. These analogs can be prepared from the previously described intermediate **16** using a variety of well known methods. Illustrated in Scheme 13 is the preparation of the 4-methoxy and the 4-methyl derivatives, however similar or other methods may be employed to incorporate a wide range of substituents. As shown below, **16** can be treated with NaOMe in refluxing methanol to provide the methoxy-containing intermediate **21A.** The methyl group can be prepared from **16** via a Pd-mediated reaction with methyl boronic acid, thus affording **21B.** The aldehyde functional group is once again liberated by oxidative cleavage of the olefin side chain using methods such as ozonolysis, or by reaction with OsO₄ and NaIO₄, to provide **22A** and **22B.**

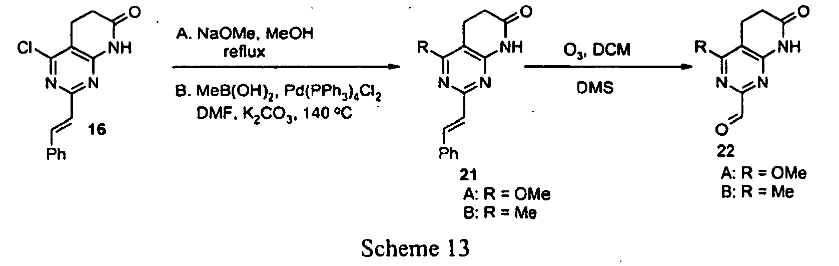

The pyrimidine oxazinone aldehyde unit needed to prepare examples of R⁵ (C) where Y³ = O and R¹⁰ = Cl, is shown in Scheme 14 starting from dimethyl diazomalonate **(23),** prepared according to Peace, Carman, Wulfman, Synthesis, 658-661, (1971). Reaction of **23** with ethyl glycolate under rhodium catalysis provides the substituted malonate **24.** The pyrimidine ring system is constructed through the reaction **of 24** with (2E)-3-phenyl-2-propenimidamide **(3),** and sodium methoxide to give **25.** Intermediate **25** is isolated as the carboxylic acid as the methyl ester is hydrolyzed under the sodium methoxide reaction conditions. Treatment of **25** with POCl₃ followed by the addition of MeOH provides dichloride-methyl ester **26.** Exchanging one of the chlorines with ammonia can be accomplished by treating **26** with NH₄OH, also providing the primary amide, which is then converted to the ethyl ester **27** with HCl and EtOH. Formation of the oxazinone ring can be carried out by treating **27** with a base such as K₂CO₃ in a polar solvent such as DMF. Heating is usually required to complete the conversion to the bicyclic system **28**. Conversion to the aldehyde can be achieved by oxidative cleavage of the 2-phenylethenyl side chain. In this particular example, the side chain of **28** is reacted with OsO₄ and NaIO₄ to give aldehyde **29.**

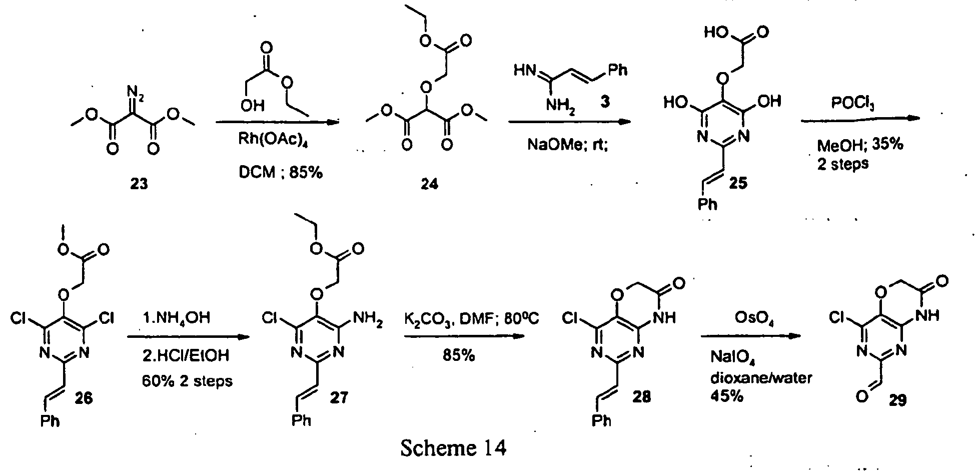

Further details for the preparation of compounds of formula (I) are found in the examples.

The antibacterial compounds according to the invention may be formulated for administration in any convenient way for use in human or veterinary medicine, by analogy with other antibacterials.

The pharmaceutical compositions of the invention include those in a form adapted for oral, topical or parenteral use and may be used for the treatment of bacterial infection in mammals including humans.

The composition may be formulated for administration by any route. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl *p*-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1 % by weight, preferably from 10-60% by weight, of the active material, depending on the method of administration. Where the compositions comprise dosage units, each unit will preferably contain from 50-1000 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (I) or a pharmaceutically acceptable salt, solvate and/or N-oxide thereof is administered in the above-mentioned dosage range.

The compound of formula (I) may be the sole therapeutic agent in the compositions of the invention or a combination with other antibacterials. If the other antibacterial is a β-lactam then a β-lactamase inhibitor may also be employed.

Compounds of formula (I) may be used in the treatment of bacterial infections caused by a wide range of organisms including both Gram-negative and Gram-positive organisms, such as upper and/or lower respiratory tract infections, skin and soft tissue infections and/or urinary tract infections. Some compounds of formula (I) may be active against more than one organism. This may be determined by the methods described herein.

The following examples illustrate the preparation of certain compounds of formula (I) and the activity of certain compounds of formula (I) against various bacterial organisms.

### Examples and Experimental

### General

Abbreviations in the examples:
LC-MS = Liquid chromatography mass spectrometry.
HPLC = High Performance Liquid Chromatography
Rt = retention time

Certain reagents are also abbreveiated herein. DMF refers to N,N-dimethylformamide, TFA refers to trifluoroacetic acid, THF refers to tetrahydrofuran and DCM refers to dichloromethane. NBS refers to N-bromosuccinimide.

Proton nuclear magnetic resonance (¹H NMR) spectra were recorded at 400, or 250 MHz, and Chemical shifts are reported in parts per million (δ) downfield from the internal standard tetramethylsilane (TMS). Abbreviations for NMR data are as follows: s = singlet, d = doublet, t = triplet, q = quartet, m = multiplet, dd = doublet of doublets, dt = doublet of triplets, app = apparent, br = broad. CDCl₃ is deuteriochloroform, DMSO-d₆ is hexadeuteriodimethylsulfoxide, and CD₃OD is tetradeuteriomethanol. Mass spectra were obtained using electrospray (ES) ionization techniques. All temperatures are reported in degrees Celsius.

MP-carbonate resin refers to macroporous triethylammonium methylpolystyrene carbonate (Argonaut Technologies). Amberlyst®A21 is a weakly basic, macroreticular resin with alkyl amine functionality, ®Registered trademark of Rohm & Haas Co. SCX is an ion exchange column containing strong cation exchange resin ( benzene sulfonic acid) supplied by Varian, USA.

Chiralpak AS-H columns comprise of a chiral adsorbent based on amylose tris [(S)- alpha methylbenzylcarbamate] coated onto 5um silica gel (21mm ID x 250mm L, Chiral Technologies, Inc). Measured retention times are dependent on the precise conditions of the chromatographic procedures. Where quoted below in the Examples they are indicative of the order of elution. Chiralpak IA column comprise of silica for preparative column (5um particle size, 21mm ID x 250mm L ) immobilized with Amylose tris (3,5-dimethylphenylcarbamate).

AD mix alpha is prepared by mixing potassium osmate (K₂OsO₄.2H₂O) (0.52g), (3a,9R,3"'a,4"'b,9"'R)-9,9'-[1,4-phthalazinediylbis(oxy)]bis[6'-(methyloxy)-10,11-dihydrocinchonan] [(DHQ)₂PHAL] (5.52g), then adding potassium ferricyanide [K₃Fe(CN)₆] (700g) and powdered potassium carbonate (294g). This mixture is stirred in a blender for 30 minutes. This provides approximately 1kg of AD mix alpha, which is commercially available from Aldrich. See K. Barry Sharpless et al, J. Org. Chem., 1992, 57 (10), 2771. AD mix beta is the corresponding mixture prepared with (9*S*,*9*"'*S*)-9,9'-[1,4-phthalazinediylbis(oxy)]bis[6'-(methyloxy)-10,11-dihydrocinchonan] [(DHQD)2PHAL]. Where AD mix alpha and beta is referred to, this is a 1:1 mixture of the alpha and beta mix.

Reactions involving metal hydrides including lithium hydride, lithium aluminium hydride, di-isobutylaluminium hydride, sodium hydride, sodium borohydride, sodium triacetoxyborohydride, (polystyrylmethyl)trimethylammonium cyanoborohydride are carried out under argon or other inert gas.

As will be understood by the skilled chemist, references to preparations carried out in a similar manner to, or by the general method of, other preparations, may encompass variations in routine parameters such as time; temperature, workup conditions, minor changes in reagent amounts etc.

### Examples 1 and 2 5-({4-[(2,3-Dihydro[1,4]dioxino[2,3-c]pyridin-7-ylmethyl)amino]-1-piperidinyl}methyl)-3-fluoro-4,5-dihydro-7H-pyrrolo[3,2,1-de]-1,5-naphthyridin-7-one hydrochloride, Enantiomers 1 and 2

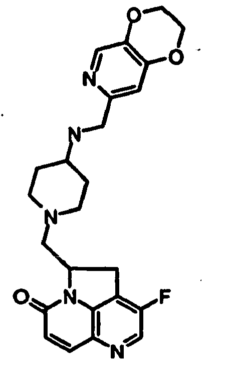

### (a) 7-Fluoro-2-(methyloxy)-8-(2-propen-1-yl)-1,5-naphthyridine

A suspension of 8-bromo-7-fluoro-2-(methyloxy)-1,5-naphthyridine (20g, 77.8 mmol),(for a synthesis, see WO2004058144, Example 53(g)) in degassed 1,4-dioxane (200 ml) was treated under argon with tris (dibenzylideneacetone)dipalladium(O) (0.71g, 0.8 mmol), bis (tri-tert-butylphosphine)palladium(O) (0.8g, 1.6 mmol), allyltributylstannane (24.7 ml, 80 mmol) and cesium fluoride (26 mg, 0.2 mmol) and the mixture heated at 70°C for 1 hour. The mixture was filtered through a plug of Keiselguhr, washing with ethyl acetate (200 ml). The filtrate was treated with half saturated brine (200 ml) and the phases separated. The aqueous phase was further extracted with ethyl acetate (2 x 100 ml). The combined organic extracts were dried and evaporated affording a brown oil (44g). This material was chromatographed eluting with with 0-100% ethyl acetate in hexane affording a yellow oil (17g, 100%).
MS (+ve ion electrospray) m/z 219 (MH+).

### (b) (2R/S)-3-[3-Fluoro-6-(methyloxy)-1,5-naphthyridin-4-yl]-1,2-propanediol

A solution of 7-fluoro-2-(methyloxy)-8-(2-propen-1-yl)-1,5-naphthyridine (17g, 78 mmol) in tert-butanol (500 ml) was treated with water (500 ml) and the cloudy suspension cooled in an ice bath. An equal mixture of AD mix alpha and beta (50g of each) was added portionwise over 45 minutes. The mixture was stirred at ambient temperature for 18 hours. More AD mix alpha and beta (20g of each) was added and the mixture stirred for a further 5 days. The phases were separated and the aqueous phase was extracted with 10% methanol in DCM (4 x 250 ml). The combined organic extracts were not homogenous and a second phase separation was carried out. The organic phase was dried (sodium sulphate) and evaporated affording a white solid (18g, 92%).
MS (+ve ion electrospray) m/z 253 (MH+).

### (c) (2R/S)-3-[3-Fluoro-6-(methyloxy)-1,5-naphthyridin-4-yl]-2-hydroxypropyl 4-methylbenzenesulfonate

A suspension of (2R/S)-3-[3-fluoro-6-(methyloxy)-1,5-naphthyridin-4-yl]-1,2-propanediol (18g, 71 mmol) in DCM (400 ml), THF (400 ml) and DMF (400 ml) was treated at 15°C with triethylamine (15 ml, 107 mmol), dibutyltin oxide (0.9g, 3.5 mmol) and para-toluenesulphonyl chloride (13.6g, 71 mmol). After 12 hours the mixture was treated with saturated aqueous sodium bicarbonate solution (300 ml) and the phases separated. The aqueous phase was further extracted with 10% methanol in dichloromethane (3 x 200 ml). The combined organic extracts were dried (sodium sulphate) and evaporated affording a yellow oil. Chromatography eluting with 0-2% methanol in DCM afforded some pure material which was isolated by trituration with ether followed by filtration affording a solid (18.4g). The filtrate was combined with the impure column fractions and evaporated. Chromatography eluting with 0-10% methanol in DCM afforded some further material which was isolated by trituration with ether followed by filtration affording a solid (3g). Combined yield = 21.4g (74%).
MS (+ve ion electrospray) m/z 407 (MH+).

### (d) 1,1-Dimethylethyl(1-{(2R/S)-3-[3-fluoro-6-(methyloxy)-1,5-naphthyridin-4-yl]-2-hydroxypropyl}-4-piperidinyl)carbamate

A suspension of (2R/S)-3-[3-fluoro-6-(methyloxy)-1,5-naphthyridin-4-yl]-2-hydroxypropyl 4-methylbenzenesulfonate (21.3g, 52.5 mmol), 1,1-dimethylethyl 4-piperidinylcarbamate (21 g, 105 mmol) in ethanol/DMF (250 ml/65 ml) and sodium carbonate (16.7g, 156 mmol) was heated to 40°C under argon for 2 days. The mixture was filtered through Keiselguhr and evaporated. The residue was partitioned between DCM and water (100 ml each) and the aqueous phase further extracted with DCM (50 ml). The combined extracts were added to a silica gel column which was subject to chromatography eluting with 0-2% methanol in ethyl acetate. Impure fractions were combined and rechromatographed eluting with 0-20% methanol in ethyl acetate. All pure fractions were combined and evaporated to afford a foam (9.7g, 42%).
MS (+ve ion electrospray) m/z 435 (MH+).

### (e) 1,1-Dimethylethyl (1-{[(5R/S)-3-fluoro-7-oxo-4,5-dihydro-7H-pyrrolo[3,2,1-de]-1,5-naphthyridin-5-yl]methyl}-4-piperidinyl)carbamate and 1,1-dimethylethyl {1-[(5R/S)-3-fluoro-8-oxo-5,6-dihydro-4H,8H-pyrido[3,2,1-de]-1,5-naphthyridin-5-yl]-4-piperidinyl}carbamate

A solution of 1,1-dimethylethyl (1-{(2R/S)-3-[3-fluoro-6-(methyloxy)-1,5-naphthyridin-4-yl]-2-hydroxypropyl}-4-piperidinyl)carbamate (2.0g, 4.6 mmol) in DCM (100 ml) was cooled to 0°C (ice-salt bath) under argon and treated with pyridine (0.82 ml, 10.1 mmol) then over 10 minutes with trifluoromethanesulphonic anhydride (0.93 ml, 5.5 mmol). The mixture was allowed to come to room temperature overnight, then recooled to 4°C and treated with a second portion of trifluoromethanesulphonic anhydride (0.19 ml, 1.1 mmol). Again, the mixture was allowed to come to room temperature overnight. The dark reaction mixture was poured into water (500 ml). The phases were separated and the aqueous phase further extracted with DCM (4 X 50 ml). The combined organic extracts were dried and evaporated affording a dark foam (3g). Chromatography eluting with 0-100% ethyl acetate in hexane then 0-50% methanol in ethyl acetate afforded an approximately equal mixture of starting material and 1,1-dimethylethyl {1-[(5R/S)-3-fluoro-8-oxo-5,6-dihydro-4H,8H-pyrido[3,2,1-de]-1,5-naphthyridin-5-yl]-4-piperidinyl}carbamate (451 mg) then 1,1-dimethylethyl (1-{[(5R/S)-3-fluoro-7-oxo-4,5-dihydro-7H-pyrrolo[3,2,1-de]-1,5-naphthyridin-5-yl]methyl}-4-piperidinyl)carbamate (487 mg, 26%).
MS (+ve ion electrospray) m/z 403 (MH+).

The above mixture of starting material and 1,1-dimethylethyl {1-[(SR/S)-3-fluoro-8-oxo-5,6-dihydro-4H,8H-pyrido[3,2,1-de]-1,5-naphthyridin-5-yl]-4-piperidinyl}carbamate (451 mg) was dissolved in dimethylsulphoxide and treated with acetonitrile. On standing 1,1-dimethylethyl {1-[(SR/S)-3-fluoro-8-oxo-5,6-dihydro-4H,8H-pyrido[3,2,1-de]-1,5-naphthyridin-5-yl]-4-piperidinyl}carbarnate crystallised out and was isolated by filtration and drying in vacuo as a white crystalline solid (35 mg). MS (+ve ion electrospray) m/z 403 (MH+).

### (f) (5R/S)-5-[(4-Amino-1-piperidinyl)methyl]-3-fluoro-4,5-dihydro-7H-pyrrolo[3,2,1-de]-1,5-naphthyridin-7-one

A solution of 1,1-dimethylethyl (1-{[(SR/S)-3-fluoro-7-oxo-4,5-dihydro-7H-pyrrolo[3,2,1-de]-1,5-naphthyridin-5-yl]methyl}-4-piperidinyl)carbamate (300 mg, 0.75 mmol) in TFA/DCM (3 ml/3 ml) was stirred for 1 hour then evaporated. The residue was dissolved in 1/1 DCM/methanol and treated with MP-carbonate resin (until a moistened pH indicator strip indicated ca pH8). Filtration, washing with methanol and evaporation afforded a brown oily solid (175 mg, 78%).
MS (+ve ion electrospray) m/z 303 (MH+).

### (g) Title compounds

A solution of (5R/S)-5-[(4-amino-1-piperidinyl)methyl]-3-fluoro-4,5-dihydro-7H-pyrrolo[3,2,1-de]-1,5-naphthyridin-7-one (66 mg, 0.22 mmol) and 2,3-dihydro[1,4]dioxino[2,3-c]pyridine-7-carboxaldehyde (for a synthesis see WO2004058144, Example 2(c) or WO03/087098, Example 19(d)) (36 mg, 0.22 mmol) in chloroform/methanol (2.5 ml/0.25 ml) was treated with sodium triacetoxyborohydride (140 mg, 6.6 mmol) and stirred under argon for 2 hours. The mixture was treated with saturated aqueous sodium bicarbonate solution (5 ml) and 10% methanol in DCM (5 ml) and the phases separated. The aqueous phase was further extracted with 10% methanol in dichloromethane (3 x 5 ml). The combined organic extracts were dried (sodium sulphate) and evaporated affording a yellow oil. Chromatography eluting with 0-30% methanol in DCM afforded the free bases of the title compounds (54 mg, 55%).
δH (CD₃OD, 250 MHz) 1.20-1.50 (2H, m), 1.75-1.95 (2H, m), 2.10-2.30 (2H, m), 2.42-2.60 (1H, m), 2.60-2.75 (2H, m), 3.00-3.15 (2H, m), 3.50-3.70 (2H, m), 3.80 (2H, s), 4.25-4.40 (4H, m), 5.10-5.20 (1H, m), 6.75 (1H, d), 6.95 (1H, s), 7.95 (1H, d), 8.00 (1H, s), 8.40 (1H, s)
MS (+ve ion electrospray) m/z 452 (MH+).

This material was converted to the corresponding dihydrochloride salt (54 mg). Chromatography of a portion (14 mg) of this material on a Chiralpak AS-H column eluting with 85:15:0.1 acetonitrile:methanol:isopropylamine afforded 4.8 mg of each of the separate free bases of the title compounds (Rt Enantiomer 1, 4.6 minutes, Rt Enantiomer 2, 8.7 minutes). Each free base was then converted to the separate title compounds with one equivalent of hydrochloric acid.

### Examples 3 and 4 5-({4-[(6,7-Dihydro[1,4]dioxino[2,3-c]pyridazin-3-ylmethyl)amino]-1-piperidinyl}methyl)-3-fluoro-4,5-dihydro-7H-pyrrolo[3,2,1-de]-1,5-naphthyridin-7-one hydrochloride, Enantiomers 1 and 2

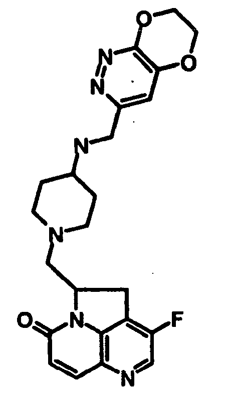

### (a) 3,4,6-Trichloropyridazine

This was prepared by a slight variation on the method of Kasnar et al, Nucleosides & Nucleotides (1994), 13(1-3), 459-79.

Hydrazine sulphate salt (51 g) was suspended in water (250ml), heated to reflux and bromomaleic anhydride (90.38 g) was added dropwise . The mixture was heated at reflux for 4 hours then cooled to room temperature. The reaction was repeated with 29g hydrazine sulphate, 53g bromomaleic anhydride and 130ml water. The precipitates were collected by filtration, washed with water and acetone and dried as a combined batch in vacuo to afford 4-bromo-1,2-dihydro-3,6-pyridazinedione as a white solid (113 g).

The solid in two batches was treated with phosphorus oxychloride (2x200 ml) and heated to reflux for 3.5 hours. The mixture was cooled, evaporated and azeotroped with toluene. The residue was partitioned between dichloromethane and saturated aqueous sodium bicarbonate solution and extracted with DCM twice more. The organic extracts were dried and evaporated. This residue was re-dissolved in dichloromethane, and chromatographed on silica gel (300 g) (DCM as eluent) to give a white solid (101.5 g, 87%).
(LC/MS analysis showed ca 20-30% impurity, isomers of bromo-dichloropyridazine).
MS (+ve ion electrospray) m/z 184/185/186 (MH+), trichloropyridazine.
MS (+ve ion electrospray) m/z 228/229/231 (MH+), bromo-dichloropyridazine.

### (b) 2-[(3,6-Dichloro-4-pyridazinyl)oxy]ethanol

A solution of ethylene glycol (55 ml) in tetrahydrofuran (200 ml) was treated at around 0°C (ice bath cooling) with sodium hydride (60% dispersion in oil, 5.9 g) over 40 minutes. After the addition was complete, 3,4,6-trichloropyridazine (27 g) containing isomers of bromo-dichloropyridazine as impurity was added portionwise and washed in with more dry THF (50ml) and the mixture was stirred at 0°C for 1 hour and then at room temperature overnight. The mixture was concentrated (to 1/3 volume) then diluted with aqueous sodium bicarbonate solution and extracted with chloroform (5x) and ethyl acetate (3x). The combined organic extracts were washed with water, dried over sodium sulphate and evaporated and the solids filtered off and washed with CHCl₃ (x3) and dried in a vacuum oven overnight at 40°C affording a white solid (25.5 g, 83%), containing some bromo-derivative (10-15%).
MS (+ve ion electrospray) m/z 209/211 (MH+).
MS (+ve ion electrospray) m/z 255/7 (MH+), bromo-derivative.

### (c) 3-Chloro-6,7-dihydro[1,4]dioxino[2,3-c]pyridazine

A solution of 2-[(3,6-dichloro-4-pyridazinyl)oxy]ethanol (5.5g) in tetrahydrofuran (4.5 litres) was treated with sodium hydride (60% dispersion in oil, 8g) and heated at 80 deg C for 72 hours. The reaction mixture was quenched with wet tetrahydrofuran then ice and evaporated to dryness. The residue was chromatographed on silica eluting with 0-100% ethyl acetate in hexane affording a white solid (2.5g, 55%) containing some bromo species (5%).
MS (+ve ion electrospray) m/z 173 (MH+).

### (d) 3-Ethenyl-6,7-dihydro[1,4]dioxino[2,3-c]pyridazine

A solution of 3-chloro-6,7-dihydro[1,4]dioxino[2,3-c]pyridazine (2.5g, 14.5 mmol) in dimethoxyethane (75 ml) was degassed under argon then tetrakis(triphenylphosphine)palladium (0) (500 mg), potassium carbonate (1.9g), 2,4,6-trivinylcyclotriboroxane pyridine complex *(commercially available from Astatech Ltd)* and water (10 ml) were added. The mixture was heated at 95°C for 16 hours. The mixture was treated with aqueous sodium bicarbonate solution and evaporated to dryness. The residue was chromatographed on silica eluting with 0-100% ethyl acetate in hexane affording a white solid (1.7g, 70%).
MS (+ve ion electrospray) m/z 165 (MH+).

### (e) 6,7-Dihydro[1,4]dioxino[2,3-c]pyridazine-3-carbaldehyde

A solution of 3-ethenyl-6,7-dihydro[1,4]dioxino[2,3-c]pyridazine (1.4g) in 1,4-dioxane/water (100 ml/35 ml) was treated with an aqueous solution of osmium tetroxide (4% w/v, 8 ml) and sodium periodate (3.9g). After 5 hours the mixture was evaporated onto silica and chromatographed eluting with 0-100% ethyl acetate in hexane affording a white solid (820 mg, 60%).
MS (+ve ion electrospray) m/z 165 (MH+).

### (f) Title compounds

A solution of (5R/S)-5-[(4-amino-1-piperidinyl)methyl]-3-fluoro-4,5-dihydro-7H-pyrrolo[3,2,1-de]-1,5-naphthyridin-7-one (58 mg, 0.19 mmol) and 6,7-dihydro[1,4]dioxino[2,3-c]pyridazine-3-carbaldehyde (32 mg, 0.19 mmol) in chloroform/methanol (2.5 ml/0.25 ml) was treated with sodium triacetoxyborohydride (120 mg, 5.7 mmol) and stirred under argon for 2 hours. The mixture was treated with saturated aqueous sodium bicarbonate solution (5 ml) and 10% methanol in DCM (5 ml) and the phases separated. The aqueous phase was further extracted with 10% methanol in dichloromethane (3 x 5 ml). The combined organic extracts were dried (sodium sulphate) and evaporated. Chromatography eluting with 0-30% methanol in DCM afforded the free bases of the title compounds (43 mg, 50%).
δH (CD₃OD, 250 MHz) 1.20-1.50 (2H, m), 1.75-1.95 (2H, m), 2.10-2.30 (2H, m), 2.42-2.55 (1H, m), 2.60-2.75 (2H, m), 3.00-3.15 (2H, m), 3.50-3.70 (2H, m), 3.90 (2H, s), 4.40-4.55 (4H, m), 5.10-5.20 (1H, m), 6.78 (1H, d), 7.25 (1H, s), 7.95 (1H, d), 8.40 (1H, s)
MS (+ve ion electrospray) m/z 453 (MH+).

This material was converted to the corresponding dihydrochloride salt (45 mg) Chromatography of a portion (18 mg) of this material on a Chiralpak AS-H column eluting with 90:10:0.1 acetonitrile:methanol:isopropylamine afforded 6.0 mg of each of the separate free bases of the title compounds (Rt Enantiomer 1, 3.9 minutes, Rt Enantiomer 2, 11.3 minutes). Each free base was then converted to the separate title compounds with one equivalent of hydrochloric acid.

### Examples 5 and 6 3-Fluoro-5-({4-[([1,3]oxathiolo[5,4-c]pyridin-6-ylmethyl)amino]-1-piperidinyl}methyl)-4,5-dihydro-7H-pyrrolo[3,2,1-de]-1,5-naphthyridin-7-one hydrochloride, Enantiomers 1 and 2

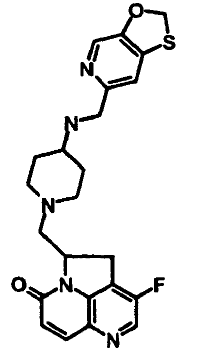

A solution of (5R/S)-5-[(4-amino-1-piperidinyl)methyl]-3-fluoro-4,5-dihydro-7H-pyrrolo[3,2,1-de]-1,5-naphthyridin-7-one (58 mg, 0.19 mmol) and [1,3]oxathiolo[5,4-c]pyridine-6-carbaldehyde (for a synthesis, see WO2004058144, Example 61) (32 mg, 0.19 mmol) in chloroform/methanol (2.5 ml/0.25 ml) was treated with sodium triacetoxyborohydride (120 mg, 5.7 mmol) and stirred under argon for 2 hours. The mixture was treated with saturated aqueous sodium bicarbonate solution (5 ml) and 10% methanol in DCM (5 ml) and the phases separated. The aqueous phase was further extracted with 10% methanol in dichloromethane (3 x 5 ml). The combined organic extracts were dried (sodium sulphate) and evaporated. Chromatography eluting with 0-30% methanol in DCM afforded the free bases of the title compounds (46 mg, 53%). δH (CD₃OD, 250 MHz) 1.20-1.50 (2H, m), 1.75-1.95 (2H, m), 2.10-2.30 (2H, m), 2.40-2.55 (1H, m), 2.60-2.75 (2H, m), 3.00-3.15 (2H, m), 3.50-3.70 (2H, m), 3.75 (2H, s), 5.10-5.20 (1H, m), 5.80 (2H, s), 6.78 (1H, d),7.35 (1H, s), 7.80 (1H, s), 7.95 (1H, d), 8.40 (1H, s)
MS (+ve ion electrospray) m/z 454 (MH+).

This material was converted to the corresponding dihydrochloride salt (50 mg) Chromatography of a portion (12 mg) of this material on a Chiralpak AS-H column eluting with 90:10:0.1 acetonitrile:methanol:isopropylamine afforded 4.6 mg of each of the separate free bases of the title compounds (Rt Enantiomer 1, 5.4 minutes, Rt Enantiomer 2, 10.7 minutes). Each free base was then converted to the separate title compounds with one equivalent of hydrochloric acid.

### Examples 7 and 8 5-({4-[(2,3-Dihydro[1,4]oxathiino[2,3-c]pyridin-7-ylmethyl)amino]-1-piperidinyl}methyl)-3-fluoro-4,5-dihydro-7H-pyrrolo[3,2,1-de]-1,5-naphthyridin-7-one hydrochloride, Enantiomers 1 and 2

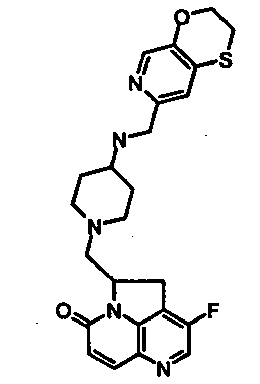

A solution of (5R/S)-5-[(4-amino-1-piperidinyl)methyl]-3-fluoro-4,5-dihydro-7H-pyrrolo[3,2,1-de]-1,5-naphthyridin-7-one (57 mg, 0.19 mmol) and 2,3-dihydro[1,4]oxathiino[2,3-c]pyridine-7-carbaldehyde (34 mg, 0.19 mmol) (for a synthesis, see WO2004058144, Example 60(i)) in chloroform/methanol (2.5 ml/0.25 ml) was treated with sodium triacetoxyborohydride (120 mg, 5.7 mmol) and stirred under argon for 2 hours. The mixture was treated with saturated aqueous sodium bicarbonate solution (5 ml) and 10% methanol in DCM (5 ml) and the phases separated. The aqueous phase was further extracted with 10% methanol in dichloromethane (3 x 5 ml). The combined organic extracts were dried (sodium sulphate) and evaporated. Chromatography eluting with 0-30% methanol in DCM afforded the free bases of the title compounds (90 mg, 100%).
δH (CD₃OD, 250 MHz) 1.30-1.50 (2H, m), 2.00-2.25 (2H, m), 2.50-2.70 (2H, m), 3.20-3.40 (4H, m), 3.60-4.00 (5H, m), 4.35 (2H, s), 4.50 (2H, t), 5.70 (1H, m), 6.95 (1H, d),7.35 (1H, s); 8.10 (2H, m), (1H, d), 8.50 (1H, s)
MS (+ve ion electrospray) m/z 468 (MH+).

This material was converted to the corresponding dihydrochloride salt (69 mg). Chromatography of a portion (14 mg) of this material on a Chiralpak AS-H column eluting with 85:15:0.1 acetonitrile:methanol:isopropylamine afforded 3.8 mg of each of the separate free bases of the title compounds (Rt Enantiomer 1, 5.2 minutes, Rt Enantiomer 2, 11.1 minutes). Each free base was then converted to the separate title compounds with one equivalent of hydrochloric acid.

### Examples 9 and 10 3-Fluoro-5-[(4-{[(3-oxo-3,4-dihydro-2H-pyrido[3,2-b][1,4]thiazin-6-yl)methyl]amino}-1-piperidinyl)methyl]-4,5-dihydro-7H-pyrrolo[3,2,1-de]-1,5-naphthyridin-7-one hydrochloride, Enantiomers 1 and 2

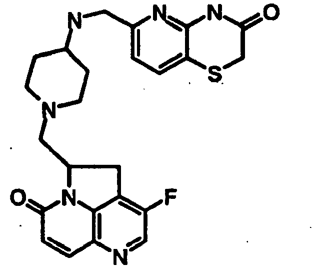

A solution of (5R/S)-5-[(4-amino-1-piperidinyl)methyl]-3-fluoro-4,5-dihydro-7H-pyrrolo[3,2,1-de]-1,5-naphthyridin-7-one (57 mg, 0.19 mmol) and 3-oxo-3,4-dihydro-2H-pyrido[3,2-b][1,4]thiazine-6-carboxaldehyde (37 mg, 0.19 mmol) (for a synthesis, see W02004058144, Example7(d)) in chloroform/methanol (2.5 ml/0.25 ml) was treated with sodium triacetoxyborohydride (120 mg, 5.7 mmol) and stirred under argon for 2 hours. The mixture was treated with saturated aqueous sodium bicarbonate solution (5 ml) and 10% methanol in DCM (5 ml) and the phases separated. The aqueous phase was further extracted with 10% methanol in dichloromethane (3 x 5 ml). The combined organic extracts were dried (sodium sulphate) and evaporated. Chromatography eluting with 0-30% methanol in DCM afforded the free bases of the title compounds (44 mg, 48%).
δH (CD₃OD, 250 MHz) 1.15-1.40 (2H, m), 1.70-1.95 (2H, m), 2.05-2.25 (2H, m), 2.40-2.50 (1H, m), 2.60-2.70 (2H, m), 2.95-3.10 (2H, m), 3.45-3.65 (4H, m), 3.75 (2H, s), 5.05-5.15 (1H, m), 6.75 (1H, d),7.05 (1H, d), 7.70 (1H, d), 7.95 (1H, d), 8.40 (1H, s) MS (+ve ion electrospray) m/z 481 (MH+).

This material was converted to the corresponding dihydrochloride salt (43 mg) Chromatography of a portion (12 mg) of this material on a Chiralpak AS-H column eluting with 85:15:0.1 acetonitrile:methanol:isopropylamine afforded 3.1 mg of each of the separate free bases of the title compounds (Rt Enantiomer 1, 5.8 minutes, Rt Enantiomer 2, 9.1 minutes). Each free base was then converted to the separate title compounds with one equivalent of hydrochloric acid.

### Example 11 (5R/S)-3-Fluoro-5-[(4-{[(3-oxo-3,4-dihydro-2H-pyrido[3,2-b][1,4]oxazin-6-yl)methyl]amino}-1-piperidinyl)methyl]-4,5-dihydro-7H-pyrrolo[3,2,1-de]-1,5-naphthyridin-7-one dihydrochloride

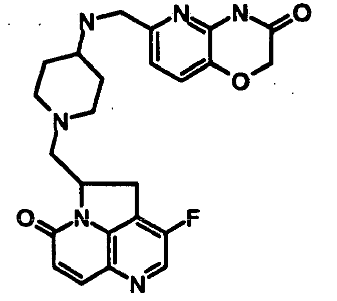

A solution of (5R/S)-5-[(4-amino-1-piperidinyl)methyl]-3-fluoro-4,5-dihydro-7H-pyrrolo[3,2,1-de]-1,5-naphthyridin-7-one (57 mg, 0.19 mmol) and 3-oxo-3,4-dihydro-2H-pyrido[3,2-b][1,4]oxazine-6-carboxaldehyde (for a synthesis, see W02004058144, Example 1(1)) (34mg, 0.19 mmol) in chloroform/methanol (2.5 ml/0.25 ml) was treated with sodium triacetoxyborohydride (120 mg, 5.7 mmol) and stirred under argon for 2 hours. The mixture was treated with saturated aqueous sodium bicarbonate solution (5 ml) and 10% methanol in DCM (5 ml) and the phases separated. The aqueous phase was further extracted with 10% methanol in dichloromethane (3 x 5 ml). The combined organic extracts were dried (sodium sulphate) and evaporated. Chromatography eluting with 0-30% methanol in DCM afforded the free base of the title compound (51 mg, 57%). δH (DMSO-d₆, 250 MHz) 1.10-1.35 (2H, m), 1.70-1.90 (2H, m), 2.00-2.22 (2H, m), 2.40-2.70 (3H, m), 2.90-3.00 (2H, m), 3.40-3.65 (4H, m), 3.70 (2H, s), 4.62 (2H, s), 5.05-5.15 (1H, m), 6.75 (1H, d),7.00 (1H, d), 7.30 (1H, d), 7.95 (1H, d), 8.45 (1H, s) MS (+ve ion electrospray) m/z 465 (MH+).

A solution of the above material was treated with 1M hydrochloric acid in methanol (0.3 ml) and evaporated to dryness affording the dihydrochloride salt.

### Examples 12 and 13 2-({4-[(6,7-Dihydro[1,4]dioxino[2,3-c]pyridazin-3-ylmethyl)amino]-1-piperidinyl}methyl)-9-fluoro-1,2-dihydro-4H-pyrrolo[3,2,1-ij]quinolin-4-one hydrochloride, Enantiomers 1 and 2

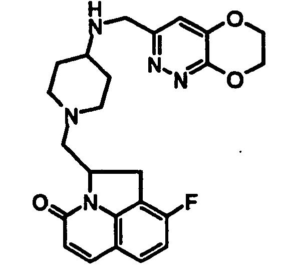

### (a) (2R/S)-1,1-Dimethylethyl {1-[(9-fluoro-4-oxo-1,2-dihydro-4H-pyrrolo[3,2,1-ij]quinolin-2-yl)methyl]-4-piperidinyl}carbamate

### Method A

(1) (2E)-N-(3-Fluoro-2-methylphenyl)-3-phenyl-2-propenamide A solution of cinnamyl chloride (100 g, 610 mmol) in ethyl acetate (400 ml) was added to a vigorously-stirred mixture of 3-fluoro-2-methylaniline (75 g, 400 mmol), saturated aqueous sodium bicarbonate (850 ml), ice (ca 100 g) and ethyl acetate (400 ml). After 1 hour the mixture was concentrated on a rotary evaporator (removing most of the ethyl acetate) and filtered, washing with water. The resulting white solid was dried *in vacuo* (∼160 g, 100%).
MS (+ve ion electrospray) m/z 256 (H+).

### (2) 7-Fluoro-8-methyl-2(1H)-quinolinone

A solution of (2E)-N-(3-fluoro-2-methylphenyl)-3-phenyl-2-propenamide (75 g, 305 mmol) in chlorobenzene (400 ml) was treated slowly with aluminium trichloride (163 g, 1.2 mol) over 10 minutes, with the temperature < 30°C. The reaction was heated to 75°C (internal temperature) for 1 hour. The mixture was allowed to cool (ca 40°C), then added to excess ice with vigorous stirring and left to stand at room temperature overnight. The resulting oily precipitate was isolated by filtration and washing with water. Drying *in vacuo* afforded a light brown solid (42.5g, 79%).
MS (+ve ion electrospray) m/z 178 (MH+).

### (3) 7-Fluoro-8-methyl-2-(methyloxy)quinoline

Crude 7-fluoro-8-methyl-2(1H)-quinolinone (46 g, 260 mmol) was suspended in DMSO (300 ml), warmed to 35°C, then treated with potassium t-butoxide (32g, 286 mmol), under argon (the internal temperature rose to 45°C). After 15 minutes methyl iodide (21 ml, 48 g, 338 mmol) was added over 2 minutes. (The internal temperature rose to 60°C).The mixture was added to water (2 litres) and extracted with hexane (1.5 litres). The hexane extract was further washed with brine, dried over sdium sulphate, and filtered through a short plug of silica (approx. 80 g), eluting with 1:1 hexane:dichloromethane (500 ml). Evaporation afforded an oil (36.8g, 74%).
MS (+ve ion electrospray) m/z 192 (MH+).

### (4) 8-(Bromomethyl)-7-fluoro-2-(methyloxy)quinoline

A solution of 7-fluoro-8-methyl-2-(methyloxy)quinoline (36.7 g, 192 mmol) in trifluoromethylbenzene (500 ml) was treated with N-bromosuccinimide (37.6 g, 211 mmol) and benzoyl peroxide (243 mg, 1 mmol) and heated at 70°C while irradiating with a 120 Watt tungsten lamp for 1 hour. The cooled mixture was filtered, washed with dichloromethane, and the combined organic fractions were washed with saturated aqueous sodium bicarbonate solution then dried. The solution was filtered through a plug of silica and evaporated affording a pale yellow solid (51.4 g, 99%).
MS (+ve ion electrospray) m/z 271 (MH+).

### (5) 7-Fluoro-2-(methyloxy)-8-(2-propen-1-yl)quinoline

A solution of 8-(bromomethyl)-7-fluoro-2-(methyloxy)quinoline (18.25 g; 67.6 mmol) in dimethoxyethane (340 ml) and water (51 ml) was degassed with a stream of argon for 10 minutes, then potassium carbonate (8.8 g), [1,1'-bis(diphenylphosphino)ferrocene]dichloro-palladium(II): DCM complex (1.12 g; 1.53 mmol) and 2,4,6-trivinylcyclotriboroxane pyridine complex (14.7 g) were added and the mixture was heated under reflux overnight. More 2,4,6-trivinylcyclotriboroxane pyridine complex (7.3 g) was added and heating was continued overnight. The mixture was evaporated to dryness and azeotroped with dry toluene and the residue was chromatographed on silica gel, eluting with DCM, then 5% ethyl acetate /hexane gave impure product (about 50% pure by LC-MS and NMR) (5.5 g).
LC-MS (+ve ion electrospray) m/z 218 (MH+).
Further elution with methanol gave a 1-{[7-fluoro-2-(methyloxy)-8-quinolinyl]methyl}pyridinium species [MS (+ve ion electrospray) m/z 269 (MH+) (12 g), which on treatment with 2,4,6-trivinylcyclotriboroxane pyridine complex (as above, for 72 hours followed by evaporation, treatment with sodium bicarbonate and extraction with chloroform) gave further product (3.4 g; ca. 80% pure by LC-MS), after chromatography on silica gel (eluting with 2% methanol-DCM).

### (6) (2R/S)-3-[7-Fluoro-2-(methyloxy)-8-quinolinyl]-1,2-propanediol

Impure 7-fluoro-2-(methyloxy)-8-(2-propen-1-yl)quinoline (ca. 50% pure; 4.1 g) in 2-butanol (50 ml) and water (50 ml) was stirred with AD mix α/β (1:1 mixture) (28.4 g) at room temperature for 72 hours. Water was added to dissolve the solid and the clear top layer (butanol) was separated and washed with a little water. The combined aqueous layers were extracted with 10% methanol-chloroform, dried (sodium sulphate), added to the butanol layer and evaporated. The product was chromatographed on silica gel, eluting with 0-15% methanol-DCM, to afford the product (3.0 g) as an oil (88% pure by LC-MS).
LC-MS (+ve ion electrospray) m/z 252 (MH+).

### (7) (2R/S)-3-[7-Fluoro-2-(methyloxy)-8-quinolinyl]-2-hydroxypropyl 4-methylbenzenesulfonate

(2R/S)-3-[7-Fluoro-2-(methyloxy)-8-quinolinyl]-1,2-propanediol (2.18 g; 8.7 mmol)) in DCM (30 ml) and triethylamine (1.22 ml) was treated with tosyl chloride (1.82 g; 9.6 mmol) and dibutyltin(IV) oxide (108 mg; 5mol. %), and the mixture was stirred at room temperature for 4.5 hr. Further tosyl chloride (150 mg) was added and the mixture was stirred for a further 30 minutes, then sodium bicarbonate solution was added and the mixture was extracted (3x) with DCM, dried (sodium sulphate) and evaporated to dryness to give the product, which was used immediately in the next step.
LC-MS (+ve ion electrospray) m/z 406 (MH+).

### (8) (2R/S)-1,1-Dimethylethyl (1-{3-[7-fluoro-2-(methyloxy)-8-quinolinyl]-2-hydroxypropyl} -4-piperidinyl)carbamate

(2R/S)-3-[7-Fluoro-2-(methyloxy)-8-quinolinyl]-2-hydroxypropyl 4-methylbenzenesulfonate (assume 8.7 mmol) and 1,1-dimethylethyl 4-piperidinylcarbamate (7.0 g, 35 mmol) in ethanol/DMF (5 ml/l ml) and disodium hydrogenphosphate (5.0 g, 35 mmol) were heated under reflux for 18 hours and cooled. Water and sodium bicarbonate was added and the mixture was extracted (3x) with DCM, dried (sodium sulphate), evaporated and chromatographed on silica gel, eluting with 0-100% ethyl acetate-hexane to afford the product as an oil (ca. 75% pure, 1.05 g). LC-MS (+ve ion electrospray) m/z 434 (MH+).

### (9) Title compound

A solution of (2R/S)-1,1-dimethylethyl(1-{3-[7-fluoro-2-(methyloxy)-8-quinolinyl]-2-hydroxypropyl}-4-piperidinyl)carbamate (1.1 g; ca. 75% pure) (2.54 mmol), methanesulfonic anhydride (0.575 g; 3.3 mmol) and diisopropylethylamine (0.86 ml; 5.1 mmol) was heated at 88°C for 3 hours and cooled. Sodium carbonate solution was added and the mixture was extracted with chloroform and then DCM (x2), dried (sodium sulphate), evaporated and chromatographed on silica gel, eluting with 0-100% ethyls acetate-hexane to afford the product. Several impure fractions were re-chromatographed, as above, to give further material. (Total yield 0.58 g).
LC-MS (+ve ion electrospray) m/z 402 (MH+).

### Method B

### (10) (2R/S)-9-Fluoro-2-(hydroxymethyl)-1,2-dihydro-4H-pyrrolo[3,2,1-ij]quinolin-4-one

A solution of 7-fluoro-2-(methyloxy)-8-(2-propen-1-yl)quinoline (ca. 50% pure, 2.0 g; ca. 4.6 mmol) in chloroform was treated with m-chloroperbenzoic acid (ca. 60% pure with water; 1.6 g) and the solution was stirred at room temperature overnight. More m-chloroperbenzoic acid (0.8 g) was added and the solution was stirred at room temperature for 72 hours. MP Carbonate resin (12 g) and excess solid sodium metabisulphite was added and the mixture was stirred at room temperature for 3 hours. It was filtered, evaporated to one third volume and chromatographed on silica gel, eluting with 0-2% methanol-DCM, then 1:1 ethyl acetate-DCM to afford a 57:25 mixture (0.85 g) of 9-fluoro-2-(hydroxymethyl)-1,2-dihydro-4*H*-pyrrolo[3,2,1-*ij*]quinolin-4-one (less polar, longer retention time on a reverse phase LC-MS system) and an isomer, believed to be 10-fluoro-2-hydroxy-2,3-dihydro-1*H*,5*H*-pyrido[3,2,1-*ij*]quinolin-5-one (more polar, shorter retention time on a reverse phase LC-MS system).
LC-MS (+ve ion electrospray) m/z 220 (MH+).

### (11) 9-Fluoro-4-oxo-1,2-dihydro-4H-pyrrolo[3,2,1-ij]quinoline-2-carbaldehyde

A 57:25 mixture (80% pure; 0.75 g; 3.42 mmol) of (2R/S)-9-fluoro-2-(hydroxymethyl)-1,2-dihydro-4*H*-pyrrolo[3,2,1-*ij*]quinolin-4-one and and 10-fluoro-2-hydroxy-2,3-dihydro-1*H*,5*H*-pyrido[3,2,1-*ij*]quinolin-5-one in dry DCM (40 ml) was stirred with 1,1,1-tris-(acetyloxy)-1,1-dihydro-1,2-benziodooxol-3-(1H)-one (Dess-Martin periodinane) (1.74 g; 4.1 mmol), solid sodium bicarbonate (2.87 g; 3.4 mmol) and 3A molecular sieves at room temperature for 2 hours. Methanol (5 drops) was added and the mixture was filtered, evaporated and divided into two equal portions. One portion was chromatographed on silica gel, eluting with 0-5% methanol-DCM, to afford a fraction (70 mg) containing impure 9-fluoro-4-oxo-1,2-dihydro-4*H*-pyrrolo[3,2,1-*ij*]quinoline-2-carbaldehyde (70 mg).
MS (+ve ion electrospray) m/z 218 (MH+).
δH (CDCl₃), (400 MHz) 9.95 (1H, s, CHO)

### (12) Title compound

Impure 9-fluoro-4-oxo-1,2-dihydro-4H-pyrrolo[3,2.1-ij]quinoline-2-carbaldehyde (assume 200 mg 0.922 mmol), and 1,1-dimethylethyl 4-piperidinylcarbamate (370 mg; 1.85 mmol) were heated in chloroform (3 ml) and dry methanol (3 ml) under reflux with 3A molecular sieves for 1 hour. The mixture was cooled and sodium triacetoxyborohydride (586 mg; 2.76 mmol) was added and the mixture was stirred at room temperature for 72 hours. Sodium carbonate solution was added and the mixture was extracted (3x) with DCM, dried (sodium sulphate), evaporated and chromatographed on silica gel, eluting with 0-15% methanol-DCM, to give an oil (27 mg).
MS (+ve ion electrospray) m/z 402 (MH+).

### (b) (2R/S)-[(4-Amino-1-piperidinyl)methyl]-9-fluoro-1,2-dihydro-4H-pyrrolo[3,2,1-ij]quinolin-4-one

A solution of (2R/S)-1,1-dimethylethyl {1-[(9-fluoro-4-oxo-1,2-dihydro-4H-pyrroio[3,2,1-ij]quinolin-2-yl)methyl]-4-piperidinyl}carbamate (0.70 g) in dry DCM (10 ml) and dry methanol (10 ml) was treated with 4M hydrogen chloride in 1,4-dioxane (15 ml) and the solution was stirred for 3 hours at room temperature, then evaporated to dryness and azeotroped with toluene/methanol. It was dissolved in methanol (10 ml) and DCM (10 ml) and stirred with excess MP-carbonate resin (7.0 g) at room temperature overnight, filtered, and evaporated to give a solid (760 mg) (contains some inorganics/resin). (95% pure by LC-MS).
LC-MS (+ve ion electrospray) m/z 302 (MH+).

### (c) Title compounds

A solution of (2R/S)-[(4-amino-1-piperidinyl)methyl]-9-fluoro-1,2-dihydro-4H-pyrrolo[3,2,1-ij]quinolin-4-one (ca. 67% pure) (110 mg, assume 73 mg pure material, 0.242 mmol) and 6,7-dihydro[1,4]dioxino[2,3-c]pyridazine-3-carbaldehyde (for a preparation see Example 3(e)) (46 mg, 0.28 mmol) in chloroform/methanol (3 ml/3 ml) was stirred at room temperature under argon overnight with excess 3A molecular sieves. The mixture was treated with sodium triacetoxyborohydride (154 mg, 0.7 mmol) and stirred for 5 hours at room temperature. Further 6,7-dihydro[1,4]dioxino[2,3-c]pyridazine-3-carbaldehyde (15 mg) was added and the mixture was stirred at room temperature overnight. The mixture was treated with saturated aqueous sodium carbonate solution and 10% methanol in chloroform and the phases separated. The aqueous phase was further extracted (3x) with 10% methanol in chloroform. The combined organic extracts were dried (sodium sulphate) and evaporated. Chromatography eluting with 0-15% methanol in DCM afforded the free bases of the title compounds as the racemate (50 mg).
LC-MS (+ve ion electrospray) m/z 452-(M+).
δH (CDCl₃), (400 MHz) 1.30-1.50 (2H, m), 1.75-1:95 (2H, m), 2.15-2.30 (2H, m), 2.40-2.60 (2H, m), 2.67 (1H, d), 3.05 (1H, d), 3.11 (1H, dd), 3.40-3.55 (3H, m), 4.00 (2H, s), 4.38 (2H, m), 4.51 (2H, m), 5.08 (1H, m), 6.55 (1H, d), 6.88 (1H, t), 7.05 (1H, s), 7.35 (1H, m), 7.65 (1H, d)
LC-MS (+ve ion electrospray) m/z 452 (MH+).

The racemate free base in methanol/chloroform was treated with excess 4M HCl in 1,4-dioxane, evaporated, and the resulting solid washed with ether to give the title compounds (dihydrochloride) as a racemic mixture. LC-MS (+ve ion electrospray) m/z 452 (MH+).

Racemic title compound dihydrochloride (54 mg) was separated by chiral preparative HPLC on a Chiralpak AS-H column (21x250mm L) eluting with 90:10:0.1 acetonitrile/methanol/isopropylamine mixture at 20ml/min and monitoring by UV at 254nM, to provide Enantiomer I (free base), 22 mg (>99% ee; retention time 6.1 min) and Enantiomer 2 (free base), 19 mg (>99% ee; retention time 12.8 min).

The free bases were converted to mono hydrochloride salts by dissolving in methanol, treatment with 1 equivalent of 6.0 M HCl, and evaporating to dryness.

### Examples 14 and 15 2-({4-[(2,3-Dihydro[1,4]dioxino[2,3-c]pyridin-7-ylmethyl)amino]-1-piperidinyl}methyl)-9-fluoro-1,2-dihydro-4H-pyrrolo[3,2,1-ij]quinolin-4-one hydrochloride, Enantiomers 1 and 2,

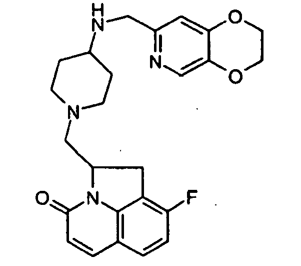

### (a) N-(2-Bromo-3-fluorophenyl)-3-phenyl-2-propenamide

To a solution of 2-bromo-3-fluoroaniline (5.29g, 27.8mmol) in acetone (12ml) was added potassium carbonate (5.75g, 41.8mmol) followed by water (15ml) and the stirred mixture cooled to 0°C. Cinnamoyl chloride (4.63g, 27.8mmol) was added portionwise over 15min then the mixture stirred for a further 2h. After this time the reaction mixture was poured onto ice/water and the resulting precipitate collected and dried to yield a white solid (8.1 g).
LC-MS: m/z 320/322 (MH)⁺.

### (b) 8-Bromo-7-fluoro-2(1H)-quinolinone

To a stirred suspension of *N*-(2-bromo-3-fluorophenyl)-3-phenyl-2-propenamide (8.0g, 25mmol) in chlorobenzene (40ml) was

added aluminium chloride (20.0g, 150mmol) portionwise over about 5min. The mixture was then heated to 120°C for 1.5h cooled to ∼50°C and added slowly onto ice. A pink oil/solid separated out and the mixture was allowed to stand overnight. The mixture was extracted with ethyl acetate and the organics dried and concentrated to a reddish solid (4.9g) which was largely the title compound. The solids were washed with hexane and dried to yield a pink solid (2.35g).
LC-MS: m/z 242/244 (MH)⁺.

### (c) 8-Bromo-7-fluoro-2-(methyloxy)quinoline

To a solution of 8-bromo-7-fluoro-2(1*H*)-quinolinone (2.0g, 8.3mmol) in DMF (30ml) was added potassium carbonate (2.28g, 16.6mmol) followed by methyl iodide (1.0ml, 9.9mmol) and the mixture stirred for 3h. The mixture was separated between ethyl acetate and water and the organics isolated, dried and concentrated to provide a dark solid (2.1g).
LC-MS: m/z 257 (MH)⁺.

### (d) 7-Fluoro-2-(methyloxy)-8-(2-propen-1-yl)quinoline

To a solution of 8-bromo-7-flluoro-2-(methyloxy)quinoline (1.51g, 5.9mmol) in 1,4-dioxane (20ml) was added allyltributylstannane (1.9ml), 5.9mmol) followed by cesium fluoride (1.9g, 12.9mmol). The mixture was degassed thoroughly then tris(dibenzylideneacetone)dipalladium(0) (0.06g, 0.06mmol) and bis(tri-t-butylphosphine)palladium(0) (0.06g, 0.12mmol) added and the mixture degassed again. The mixture was heated at 75°C for 3h then allowed to cool and separated between ethyl acetate and brine. The organics were isolated, dried and concentrated to a red oil. Chromatography (100g silica SPE, gradient elution hexane/dichloromethane 0% to 15%) provided the title compound contaminated with some tin residues (1.72g).
LC-MS: m/z 218 (MH)⁺.

### (e) (2R/S)-2-(Bromornethyl)-9-fluoro-1,2-dihydro-4H-pyrrolo[3,2,1-ij]quinolin-4-one

To a solution of 7-fluoro-2-(methyloxy)-8-(2-propen-1-yl)quinoline (0.5g, 2.3mmol) in DMF (5ml) was added water (0.3ml) followed by NBS (0.41g, 2.3mmol) and the solution stirred for 2h. The reaction mixture was separated between ethyl acetate and brine and the organics isolated, washed with brine, dried and concentrated. Chromatography of the residues (70g silica SPE, gradient elution with dichloromethane/hexane 2:1 to dichloromethane then dichloromethane/ethyl acetate 1:0 to 4:1) to yield a mixture of 2-(bromomethyl)-9-fluoro-1,2-dihydro-4*H*-pyrrolo[3,2,1-*ij*]quinolin-4-one and 2-bromo-10-fluoro-2,3-dihydro-1*H*,5*H*-pyrido[3,2,1-*ij*]quinolin-5-one in a 4:1 ratio (0.27g).
LC-MS: m/z 282/284 (MH)⁺.

### (f) 1,1-Dimethylethyl (1-{[(2R/S)-9-fluoro-4-oxo-1,2-dihydro-4H-pyrrolo[3,2,1-ij]quinolin-2-yl]methyl}-4-piperidinyl}carbamate

To a solution of 2-(bromomethyl)-9-fluoro-1,2-dihydro-4H-pyrrolo[3,2,1-*ij*]quinolin-4-one and 2-bromo-10-fluoro-2,3-dihydro-1*H*,5*H-*pyrido[3,2,1-*ij*]quinolin-5-one in a 4:1 ratio (0.25g, 0.9mmol) in acetonitrile (20ml) was added 1,1-dimethylethyl 4-piperidinylcarbamate (0.18g, 0.9mmol) and potassium carbonate (0.124g, 0.9mmol). The mixture was heated at 50°C for 24h whereupon 1c/ms monitoring of the reaction showed only 50% conversion. Therefore a further equivalent of 1,1-dimethylethyl 4-piperidinylcarbamate (0.18g, 0.9mmol) was added together with potassium carbonate (0.124g, 0.9mmol) and heated for a further 24h at 50°C followed by heating at 70°C for 2h. After cooling the solution was concentrated and the residues separated between ethyl acetate and water. The organics were separated, dried and concentrated. Chromatography of the residues (20g silica SPE, gradient elution with hexane/ethyl acetate 40%-100%) provided a yellow solid (0.15g).
LC-MS: m/z 402 (MH)⁺.

### (g) (2R/S)-2-[(4-Amino-1-piperidinyl)methyl]-9-fluoro-1,2-dihydro-4H-pyfrolo[3,2,1-ij]quinolin-4-one hydrochloride

To a solution of 1,1-dimethylethyl (1-{[(2R/S)-9-fluoro-4-oxo-1,2-dihydro-4*H-*pyrrolo[3,2,1-ij]quinolin-2-yl]methyl}-4-piperidinyl)carbamate (0.14g, 0.35mmol) in dichloromethane (1ml) was added HCl in 1,4dioxane (1ml, 4M solution) followed by methanol (0.5ml). The solution was stirred for 3h then concentrated to provide a cream solid (0.14g).
LC-MS: m/z 302 (MH)⁺.

### (h) Title compounds

To a suspension of (2R/S)-2-[(4-amino-1-piperidinyl)methyl]-9-fluoro-1,2-dihydro-4*H*-pyrrolo[3,2,1-*ij*]quinolin-4-one hydrochloride (0.12g, 0.32mmol) in dichloromethane (5ml) was added triethylamine (0.11ml, 0.8mmol) whereupon all the solids dissolved. To the solution was added 2,3-dihydro[1,4]dioxino[2,3-*c*]pyridine-7-carbaldehyde (for a synthesis see WO2004058144, Example 2(c) or WO03/087098, Example 19(d)) (0.053g, 0.32mmol) followed by sodium triacetoxyborohydride (0.20g, 0.96mmol) and the solution stirred for 4h. The reaction mixture was separated between dichloromethane and saturated sodium bicarbonate solution. The organics were separated and washed with brine, dried (MgSO₄) and concentrated to provide the free bases of the title compounds (racemate) as a clear oil (0.14 g, 100%).
δH (CDCl₃), (400 MHz) 1.30-1.50 (2H, m), 1.75-1.95 (2H, m), 2.15-2.30 (2H, m), 2.40-2.55 (2H, m), 2.65 (1H, d), 3.05 (1H, d), 3.11 (1H, dd), 3.40-3.55 (3H, m), 3.75 (2H, s), 4.25 (2H, m), 4.35 (2H, m), 5.08 (1H, m), 6.55 (1H, d), 6.8 (1H, s), 6.85 (1H, t), 7.35 (1H, m); 7.65 (1H, d), 8.1 (1H, s).
LC-MS: m/z 451 (MH)⁺.

Chromatography of a portion (80 mg) of this material on a Chiralpak AS-H column eluting with 90:10:0.1 acetonitrile:methanol:isopropylamine afforded 19 mg of the free base of Enantiomer 1 isomer (retention time 5.0 minute), followed by 23 mg of the free base of Enantiomer 2 isomer (retention time 13.2 minutes).

The free bases were converted to hydrochloride salts by dissolving in methanol, treatment with 1 equivalent of 6.0 M HCl, and evaporating to dryness.

### Examples 16 and 17 2-({4-[(6,7-Dihydro[1,4]oxathiino[2,3-c]pyridazin-3-ylmethyl)amino]-1-piperidinyl}methyl)-9-fluoro-1,2-dihydro-4H-pyrrolo[3,2,1-ij]quinolin-4-one hydrochloride, Enantiomers 1 and 2

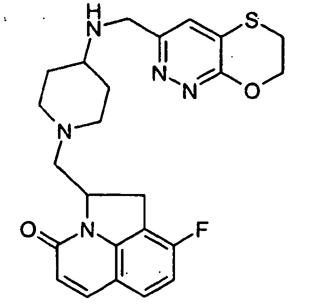

### (a) 2-[(3,6-Dichloro-4-pyridazinyl)thio]ethanol

A solution of 3,4,6-trichloropyridazine (25 g) in tetrahydrofuran (200 ml) and triethylamine (19 ml) was treated at 0°C (ice bath cooling) with 2-mercaptoethanol (8.33 ml) over 5 minutes. After the addition was complete, the mixture was stirred at room temperature for 72 hours. The mixture was stirred with aqueous sodium bicarbonate solution and dichloromethane and the solid was collected, washed with water, ether and pentane and dried in vacuo, giving (22.9 g). The combined aqueous and organic fraction was evaporated to half volume giving further solid, which was washed and dried as above (5.0 g). The total yield of solid (27.9 g; 91 %) contained some bromo-derivative (5-10%) by NMR.

### (b) 3-Chloro-6,7-dihydro[1,4]oxathiino[2,3-c]pyridazine

A solution of 2-[(3,6-dichloro-4-pyridazinyl)thio]ethanol(13 g) (previously dried at 50°C in vacuo) in dry 1,4-dioxane (250 ml) was treated with lithium hydride (3 g) in portions and heated at 105 -110°C for 24 hours. The reaction mixture was cooled and quenched with iced-water. The solution was taken to pH 10 - 11 with 5M hydrochloric acid and evaporated. Water was added and the mixture was extracted 4x with dichloromethane, dried (sodium sulphate), evaporated, and chromatographed on silica gel, eluting with 0-100% ethyl acetate-hexane, to afford a white solid (1.61 g) (containing ca. 10% of a bromo species).
MS (+ve ion electrospray) m/z 189/91 (Cl MH+); 233/5 (Br MH+)
δH (CDCl₃, 400MHz) 3.23 (2H, m), 4.67 (2H, m), 7.26 (1H, s) (for major chloro-compound).

### (c) 3-Ethenyl-6,7-dihydro[1,4]oxathiino[2,3-c]pyridazine

A solution of 3-chloro-6,7-dihydro[1,4]oxathiino[2,3-c]pyridazine(1.0 g) in dimethoxyethane (25 ml) was degassed under argon then tetrakis(triphenylphosphine)palladium (0) (135 mg), potassium carbonate (0.695 g), triethenylboroxin pyridine complex (0.8 g) and water (3.7 ml) were added. The mixture was heated overnight at 105°C. More triethenylboroxin pyridine complex (0.4 g) and tetrakis(triphenylphosphine)palladium (0) (30 mg) were added and heating was continued for 24 hours. The mixture was cooled, treated with aqueous sodium bicarbonate solution, extracted (4x) with dichloromethane, dried (sodium sulphate), evaporated and chromatographed on silica gel (70 g), eluting with 0-100% ethyl acetate - hexane, affording a solid (0.56 g) (87% pure by LC-MS).
MS (+ve ion electrospray) m/z 181 (MH+).

### (d) 6,7-Dihydro[1,4]oxathiino[2,3-c]pyridazine-3-carbaldehyde

A solution of 3-ethenyl-6,7-dihydro[1,4]oxathiino[2,3-c]pyridazine(320 mg) in 1,4-dioxane/water (20 ml/5 ml) was treated with an aqueous solution of osmium tetroxide (4% w/v, 2 ml) and sodium periodate (1.08 g), initially stirred in an ice-bath, then allowed to warm to room temperature. After 2.5 hours the mixture was evaporated to dryness and dissolved in 1,4-dioxane and chloroform. Silica gel was added and the mixture was evaporated to dryness, added to a silica column (50 g) and chromatographed, eluting with 0-100% ethyl acetate in hexane, to afford a white solid (116 mg, 36%). MS (+ve ion electrospray) m/z 183 (MH+).

### (e) Title compounds

A solution of (2R/S)-[(4-amino-1-piperidinyl)methyl]-9-fluoro-1,2-dihydro-4H-pyrrolo[3,2,1-ij]quinolin-4-one (for a preparation see Example 12(b)) (ca. 67% pure) (110 mg, assume 73 mg pure material; 0.242 mmol) and 6,7-dihydro[1,4]oxathiino[2,3-c]pyridazine-3-carbaldehyde (ca. 80% pure) (55 mg, assume 44 mg pure material; 0.243 mmol) in methanol (2 ml) and chloroform (2 ml) with 3A sieves was stirred overnight. Sodium triacetoxyborohydride (150 mg, 0.70 mmol) was added and stirred for 8 hours. Further sodium triacetoxyborohydride was added and stirring was continued overnight. The mixture was basified with sodium carbonate solution and extracted with 10% methanol in DCM (3x), and the extracts were dried and evaporated. Chromatography on silica gel eluting with 0-10% methanol in DCM gave the racemic free base (29 mg). δH (CDCl₃), (250 MHz) 1.30-1.50 (2H, m), 1.55-1.95 (2H, m), 2.10-2.35 (2H, m), 2.35-2.60 (2H, m), 2.70 (1H, d), 3.00-3.30 (4H, m), 3.35-3.60 (2H, m), 3.97 (2H, s),4.60-4.75 (2H, m), 5.10 (1H, m), 6.57 (1H, d), 6.89 (1H, t), 7.34 (2H, m), 7.66 (1H, d).

The enantiomers were separated by chiral preparative HPLC on a Chiralpak AS-H column (21 x250mm L) eluting with 90:10:0.1 acetonitrile/methanol/isopropylamine mixture at 20ml/min and monitoring by UV at 254nM. 24 mg of racemate was processed to provide Enantiomer 1 (7 mg, >99% ee; retention time 3.9 min) and Enantiomer 2 (7 mg, >99% ee; retention time 10.9 min).

The free bases were dissolved in methanol and treated with one equivalent of 6.0 M HCl, and evaporated to give the title compounds (each 8 mg).
MS (+ve ion electrospray) m/z 468 (MH+).

### Examples 18 and 19 5-({4-[(6,7-Dihydro[1,4]dioxino[2,3-c]pyridazin-3-ylmethyl)amino]-1-piperidinyl}methyl)-7-fluoro-5,6-dihydro-3H-pyrrolo[1,2,3-de]quinoxalin-3-one hydrochloride, Enantiomers 1 and 2

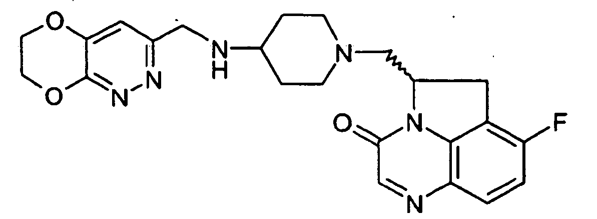

### (a) 2-Chloro-N-(3-fluoro-2-methylphenyl)acetamide.

A solution of chloroacetyl chloride (38.4 ml, 480 mmol) in ethyl acetate (100 ml) was added to a vigorously-stirred mixture of3-fluoro-2-methylaniline (60g, 480 mmol), saturated aq. sodium bicarbonate (400 ml), ice (ca 100g) and ethyl acetate (400 ml). After 1 hour the mixture was concentrated on a rotary evaporator (removing most of the ethyl acetate) and filtered, washing with water. The resulting white solid was dried in vacuo (94.4g, 98%).
LC-MS (+ve ion electrospray): *m*/*z* 202/204 [MH⁺]

### (b) 2-Chloro-N-(3-fluoro-2-methyl-6-nitrophenyl)acetamide.

A solution of 2-chloro-*N*-(3-fluoro-2-methylphenyl)acetamide (47.1g, 243 mmol) in conc. sulphuric acid (220 ml) was treated at ca. -20°C (ethanol/water/CO₂ bath) with conc. nitric acid (69%, 15.2 ml, 19g, 235 mmol) over 30 minutes, keeping internal temperature <10°C. The reaction was complete at the end of the addition. The mixture was added to ice with vigorous stirring then left to stand overnight. Filtration, washing of the solid with water and drying afforded the product (54.6g, 95%).
LC-MS (-ve ion electrospray): *m*/*z* 245/247 [(M-H⁺)⁻]

### (c) 3-Fluoro-2-methyl-6-nitroaniline.

2-Chloro-*N*-(3-fluoro-2-methyl-6-nitrophenyl)acetamide (prepared from 47.2g of 2-chloro-*N*-(3-fluoro-2-methylphenyl)acetamide, ca. 234 mmol) was suspended in water (220 ml) then treated with aqueous sodium hydroxide solution (12.5M, 110 ml, 1.37 mol). Tetrahydrofuran (110 ml) was added, then the mixture was heated to reflux for 5 hours, then concentrated on a rotary evaporator (removing most of the tetrahydrofuran). The yellow solid was filtered off and washed with cold water until washing were non-alkaline. Drying in vacuo afforded a yellow solid (32.3g, 81% over 2 steps).
LC-MS (+ve ion electrospray): *m*/*z* 171 [MH⁺]

### (d) 2-Cyano-N-(3-fluoro-2-methyl-6-nitrophenyl)acetamide.

### Method A

To a solution of 3-fluoro-2-methyl-6-nitroaniline (10.0g, 58.8 mmol) and cyanoacetic acid (10.1g, 117.5 mmol) in dry toluene (500ml) was added phosphorus pentachloride (25.8g, 117.5 mmol) portionwise with stirring. The mixture was then heated at 120-125°C while passing air over the mixture. After 2.5h, an extra 1g of cyanoacetic acid and 2.6g phosphorus pentachloride were added and heating continued for 1h. After cooling, toluene was evaporated and the residue was dissolved in ethyl acetate and washed with brine, brine/sodium bicarbonate (twice) and brine, dried and evaporated. The crude product was recrystallised from ethanol, washed with ethanol/hexane (1:1) and dried to give 10.72g (77%), which contained some 6-fluoro-5-methyl-3-oxo-3,4-dihydro-2-quinoxalinecarbonitrile 1-oxide (lower-running yellow spot on TLC, 1:1 ethyl acetate /hexane).

### Method B

2-Chloro-*N*-(3-fluoro-2-methyl-6-nitrophenyl)acetamide (2.7g, 11 mmol) in dimethylformamide (20ml) was treated with potassium cyanide (1.08g, 16.6 mmol) and the mixture was stirred at room temperature for 24h. The dimethylformamide was evaporated and the residue was dissolved in ethyl acetate/water. The aqueous phase was extracted several times with ethyl acetate and the organic extracts were dried and evaporated. The crude product was recrystallised from ethanol/petrol (40-60°C) to give a pale brown solid (1.44g, 55%).
LC-MS (-ve ion electrospray): *m*/*z* 236 [(M-H⁺)⁻]

### (e) 6-Fluoro-5-methyl-3-oxo-3,4-dihydro-2-quinoxalinecarbonitrile 1-oxide

2-Cyano-*N*-(3-fluoro-2-methyl-6-nitrophenyl)acetamide (11.17g, 45 mmol) was partially dissolved in pyridine (50ml) and 1M aq. sodium hydroxide (46ml) was added, giving a clear solution. The mixture was stirred at room temperature for 24h, then water was added to dissolve all solids and the mixture was filtered, washing through with water. The filtrate (∼ 300ml) was acidified with 5M HCl to pH6. The precipitate was filtered off, washed with water and dried to give 8.13g of product. The filtrate was acidified to pH1 and extracted three times with ethyl acetate. The extracts were dried and evaporated and the residue was azeotroped with toluene to give 2.28g product containing some of the acetamide starting material.
Total yield 10.41 g, containing approx. 7% acetamide.
LC-MS (+ve ion electrospray): *m*/*z* 220 [MH⁺]

### (f) 7-Fluoro-8-methyl-2(1H)-quinoxalinone.

A mixture of 6-fluoro-5-methyl-3-oxo-3,4-dihydro-2-quinoxalinecarbonitrile 1-oxide (15.9g, 71.8 mmol) and sodium dithionite (36.7g, 179.6 mmol) in ethanol (200ml) and water (400ml) was heated under reflux for 1h, with a flow of argon over the top of the condenser leading to a bleach-filled Drechsel bottle to trap HCN. The cooled mixture was carefully acidified to pH1 with 5M HCl and the mixture was stirred for 45 min at room temperature. Sodium hydroxide (50% aq.) was then added to give pH11-12 and the mixture was evaporated to remove approx. 500ml. The residue was acidified to pH 6 with 5M HCl (caution: cyanide still present!) and the precipitate was filtered off, washed with water and dried to give a solid (10.37g, 81%). Extraction of the aqueous liquor with 10% methanol/dichloromethane and evaporation of the extracts gave a further small amount of product (0.8g). Total yield 11.2g, 88%.
LC-MS (+ve ion electrospray): *m*/*z* 179 [MH⁺]

### (g) 2-Chloro-7-fluoro-8-methylquinoxaline.

7-Fluoro-8-methyl-2(1*H*)-quinoxalinone (5.75g, 32.3 mmol) in phosphorus oxychloride (30ml) was heated under reflux for 2h. Phosphorus oxychloride was removed by evaporation, and the residue was basified with aq. sodium bicarbonate and extracted several times with ethyl acetate. The extracts were dried and evaporated, and the residue was dissolved in dichloromethane and passed quickly through a short column of silica (20g), washing through with more dichloromethane. Removal of solvent gave a light brown solid (4.13g, 65%).
LC-MS (+ve ion electrospray): *m*/*z* 197/199 [MH⁺]

### (h) 7-Fluoro-8-methyl-2-(methyloxy)quinoxaline.

2-Chloro-7-fluoro-8-methylquinoxaline (4.1g, 20.9 mmol) was suspended in dry methanol and a 25% solution of sodium methoxide in methanol (4.73ml, 21.9 mmol) was added. The mixture was heated under reflux for 2h, then evaporated. The residue was dissolved in dichloromethane and water and the phases were separated. The aqueous phase was extracted twice with dichloromethane, and combined organic fractions were dried and evaporated to give a light brown solid (3.84g, 96%).
LC-MS (+ve ion electrospray): *m*/*z* 193 [MH⁺]

### (i) 8-Bromomethyl-7-fluoro-2-(methyloxy)quinoxaline.

7-Fluoro-8-methyl-2-(methyloxy)quinoxaline (50g, 260 mmol) was dissolved in dry chloroform (1L). *N*-Bromosuccinimide (52g, 292 mmol) and benzoyl peroxide (70%, 0.42g) were added and the mixture was heated under reflux, illuminated with a 120W lamp, for 2h. After cooling, the mixture was washed with water. The aqueous phase was re-extracted twice with dichloromethane and the combined organic fractions were washed with water, dried and evaporated to give an off-white solid (72.1g, 100%).
LC-MS (+ve ion electrospray): *m*/*z* 271/273 [MH⁺]

### (j) 7-Fluoro-2-(methyloxy)-8-(2-propen-1-yl)quinoxaline

A solution of 8-bromomethyl-7-fluoro-2-(methyloxy)quinoxaline(25g, 92.2 mmol) in 1,4-dioxane (450mL) and water (60mL) was flushed with argon, then 2,4,6-trivinylcyclotriboroxane pyridine complex (15g, 62.1 mmol), [1,1'-bis(diphenylphosphino)ferrocene]dichloropalladium(Il) dichloromethane complex (1.52g) and potassium carbonate (12g, 185 mmol) were added. The mixture was heated under reflux, under argon, for approx. 72h. After cooling, water and ethyl acetate were added and the phases were separated.The aqueous phase was extracted twice with ethyl acetate and the combined organic fractions were dried and evaporated. Chromatography on silica, eluting with 0-20% ether/hexane gave the product (8.23g, 41 %), containing a few mole percent of the 8-methyl analogue.
LC-MS (+ve ion electrospray): *m*/*z* 219 [MH⁺]

### (k) (2R/S)-3-[6-Fluoro-3-(methyloxy)-5-quinoxalinyl]-1,2-propanediol

To a solution of 7-fluoro-2-(methyloxy)-8-(2-propen-1-yl)quinoxaline (8.23g, 37.75 mmol), in tert-butanol (250mL) and water (250mL) was added a 1:1 mixture of AD mix alpha and beta (50g). The mixture was stirred overnight at room temperature, another portion of 10g of AD mix alpha and beta was added and stirring was continued for three days. Sodium sulphite (60g) was added and the mixture was stirred for 30min., then the phases were separated. The aqueous phase was extracted twice with 10% methanol/dichloromethane and the combined organic fractions were dried and evaporated to give the product (9.57g, 100%).
LC-MS (+ve ion electrospray): *m*/*z* 253 [MH⁺]

### (1) (2R/S)-3-[6-Fluoro-3-(methyloxy)-5-quinoxalinyl]-2-hydroxypropyl 4-methylbenzenesulfonate

(2R/S)-3-[6-Fluoro-3-(methyloxy)-5-quinoxalinyl]-1,2-propanediol (9.57g, 37.75 mmol) was partially dissolved in anhydrous tetrahydrofuran/dichloromethane (1:1, 400mL). Triethylamine (7.85mL), 56.6 mmol) and dibutyltin(IV) oxide (0.47g, 1.9 mmol) were added, followed by 4-methylbenzensulfonyl chloride (7.16g, 37.75 mmol). The mixture was stirred at room temperature overnight, then aqueous sodium bicarbonate was added and the phases were separated. The aqueous phase was extracted with dichloromethane and the organic fractions were washed with brine, dried and evaporated to give the product (15.4g, approx. 100%), containing some residual triethylamine and a few percent of 7-fluoro-2-(methyloxy)-8-(2-oxiranylmethyl)quinoxaline.
LC-MS (+ve ion electrospray): *m*/*z* 407 [MH⁺]

### (m) 1,1-Dimethylethyl (1-{(2R/S)-3-[6-fluoro-3-(methyloxy)-5-quinoxalinyl]-2-hydroxypropyl}-4-piperidinyl)carbamate

A mixture of (2R/S)-3-[6-fluoro-3-(methyloxy)-5-quinoxalinyl]-2-hydroxypropyl 4-methylbenzenesulfonate (15.4g, 37.75 mmol), 1,1-dimethylethyl 4-piperidinylcarbamate (15.1g, 75.5 mmol) and sodium carbonate (12.0g, 113.25 mmol) in ethanol (180mL) and dimethylformamide (45mL) was heated at 40°C for approx. 64h. The mixture was evaporated and the residue was dissolved in brine and ethyl acetate. The phases were separated and the aqueous phase was extracted twice with ethyl acetate. Organic fractions were dried and evaporated, and the residue was chromatographed on silica, eluting with 0-20% methanol/ethyl acetate to give the product (8.05g, 49%). LC-MS (+ve ion electrospray): *m*/*z* 435 [MH⁺]

### (n) 1,1-Dimethylethyl (1-{[(5R/S)-7-fluoro-3-oxo-5,6-dihydro-3H-pyrrolo[1,2,3-de]quinoxalin-5-yl]methyl}-4-piperidinyl)carbamate and 1,1-dimethylethyl {1-[(6R/S)-8-fluoro-3-oxo-6,7-dihydro-3H,5H-pyrido[1,2,3-de]quinoxalin-6-yl]-4-piperidinyl carbamate

To a solution of 1,1-dimethylethyl (1-{(2R/S)-3-[6-fluoro-3-(methyloxy)-5-quinoxalinyl]-2-hydroxypropyl}-4-piperidinyl)carbamate (7.02g, 16.15 mmol) in dry chloroform (80mL) was added methanesulfonic anhydride (3.37g, 18.5 mmol) and di*iso*propylethylamine (6.32mL, 36.2 mmol). The mixture was stirred for 0.5h at room temperature, and then heated under reflux for 24h. After cooling, water was added and the phases were separated. The aqueous phase was extracted twice with dichloromethane, and the organic fractions were dried and evaporated. Chromatography on silica eluted with 50-100% ethyl acetate/hexane gave the title isomeric products 1,1-dimethylethyl (1-{[(5R/S)-7-fluoro-3-oxo-5,6-dihydro-3*H*-pyrrolo[1,2,3-*de*]quinoxalin-5-yl]methyl}-4-piperidinyl)carbamate (eluted first, 3.55g, 55%), and 1,1-dimethylethyl {1-[(6R/S)-8-fluoro-3-oxo-6,7-dihydro-3H,5H-pyrido[1,2,3-*de*]quinoxalin-6-yl]-4-piperidinyl}carbamate (eluted second, 0.84g, 13%).
LC-MS (+ve ion electrospray): *m*/*z* 403 [MH⁺]

### (o) (5R/S)-5-[(4-Amino-1-piperidinyl)methyl]-7-fluoro-5,6-dihydro-3H-pyrrolo[1,2,3-de]quinoxalin-3-one

To a solution of 1,1-dimethylethyl (1-{[(5R/S)-7-fluoro-3-oxo-5,6-dihydro-3*H-*pyrrolo[1,2,3-*de*]quinoxalin-5-yl]methyl}-4-piperidinyl)carbamate (0.21g, 0.52 mmol) in dichloromethane (4mL) and methanol (2.5mL) was added hydrogen chloride in 1,4-dioxane (4M, 4mL). The mixture was stirred for 1.75h then evaporated to dryness. The residue was dissolved in 10% methanol/dichloromethane (20mL) and stirred with MP-carbonate resin (0.77g, 2.69 mmol/g) for 1h. The resin was filtered off, washed a few times with 10% methanol/dichloromethane and methanol alternately, and the filtrate was evaporated to give the free amine (0.19g, approx. 83% pure).
LC-MS (+ve ion electrospray): *m*/*z* 303 [MH⁺]

### (p) Title compounds

(5R/S)-5-[(4-Amino-1-piperidinyl)methyl]-7-fluoro-5,6-dihydro-3*H-*pyrrolo[1,2,3-*de*]quinoxalin-3-one (approx. 83% pure, 0.19g, 0.52 mmol) and 6,7-dihydro[1,4]dioxino[2,3-*c*]pyridazine-3-carbaldehyde (for a preparation see Example 3(e)) (96mg, 0.58 mmol) were dissolved in dry chloroform and methanol (1:1, 14mL) and heated under reflux overnight with 3A molecular sieves. After cooling, sodium triacetoxyborohydride (0.44g, 2.08 mmol) was added and the mixture was stirred for 8h at room temperature. Aqueous sodium bicarbonate was added to basify and the phases were separated. The aqueous phase was extracted several times with dichloromethane and 10% methanol/dichloromethane, and the organic fractions were dried and evaporated. Chromatography on silica, eluting with 0-10% methanol/dichloromethane gave the title compounds as free bases (0.144g, 61 %).
δH (CDCl₃, 250MHz) 1.38 (2H, m), 1.88 (2H, m), 2.24 (2H, m), 2.50 (2H, m), 2.69 ( 1H, br. d), 3.00 (1H, br. d), 3.13 (1H, dd), 3.48 (1H, m, part. obscured by solvent)), 3.99 (2H, s), 4.37 (2H, m), 4.51 (2H, m), 5.08 (1H, m), 6.99 (1H, t), 7.02 (1H,s), 7.65 (1H, dd), 8.15 (1H, s)
LC-MS (+ve ion electrospray): *m*/*z* 453 [MH⁺]

The free bases in dichloromethane/methanol were treated with 0.4M hydrogen chloride in 1,4-dioxane (1.6mL), and the solvent was evaporated to give the racemic dihydrochloride salt (167mg).

Racemic 5-({4-[(6,7-dihydro[1,4]dioxino[2,3-c]pyridazin-3-ylmethyl)amino]-1-piperidinyl}methyl)-7-fluoro-5,6-dihydro-3H-pyrrolo[1,2,3-de]quinoxalin-3-one dihydrochloride (148mg) was separated by preparative HPLC on a 21x 250mm Chiralpak AS-H® column, 2 injections, eluting with acetonitrile/methanol/isopropylamine (90/10/0.1, 20ml/min, UV monitoring at 254nm) to give the two enantiomers E1 (retention time 3.6min.) and E2 (retention time 5.1min.) of the free base of the title compounds.

The separate enantiomers in methanol were both treated with one equivalent of aqueous hydrochloric acid, and the solvent was evaporated to give the title hydrochloride salts.

### Example 18B 5-({4-[(6,7-Dihydro[1,4]dioxino[2,3-c]pyridazin-3-ylmethyl)amino]-1-piperidinyl}methyl)-7-fluoro-5,6-dihydro-3H-pyrrolo[1,2,3-de]quinoxalin-3-one fumarate, Enantiomer 1

A solution of the free base (0.84g) in dichloromethane/methanol was treated with a solution of fumaric acid (216 mg) in methanol. The mixture was evaporated and the resulting solid triturated with ether and dried (1.05g).

### Example 19B 5-({4-[(6,7-Dihydro[1,4]dioxino[2,3-c]pyridazin-3-ylmethyl)amino]-1-piperidinyl}methyl)-7-fluoro-5,6-dihydro-3H-pyrrolo[1,2,3-de]quinoxalin-3-one di-trifluoracetate, Enantiomer 2

The free base (29 mg) was purified on a reverse phase C18 column eluting with aqueous acetonitrile containing 1% TFA. Removal of solvents afforded a solid (30 mg).

### Example 19C 5-({4-[(6,7-Dihydro[1,4]dioxino[2,3-c]pyridazin-3-ylmethyl)amino]-1-piperidinyl}methyl)-7-fluoro-5,6-dihydro-3H-pyrrolo[1,2,3-de]quinoxalin-3-one di-fumarate, Enantiomer 2

A solution of the free base (1.1g) in dichloromethane/methanol was treated with a solution of fumaric acid (281 mg) in methanol. The mixture was evaporated and the resulting solid triturated with ether and dried (1.24g).

### Example 19D 5-({4-[(6,7-Dihydro[1,4]dioxino[2,3-c]pyridazin-3-ylmethyl)amino]-1-piperidinyl}methyl)-7-fluoro-5,6-dihydro-3H-pyrrolo[1,2,3-de]quinoxalin-3-one benzoate, Enantiomer 2

The free base (900 mg) was dissolved in methanol (50 ml) and treated with benzoic acid (243 mg) then evaporated to dryness affording an off-white solid (1.17g).

### Example 20 (6R/S)-6-{4-[(2,3-Dihydro[1,4]dioxino[2,3-c]pyridin-7-ylmethyl)amino]-1-piperidinyl}-8-fluoro-6,7-dihydro-3H,5H-pyrido[1,2,3-de]quinoxalin-3-one dihydrochloride

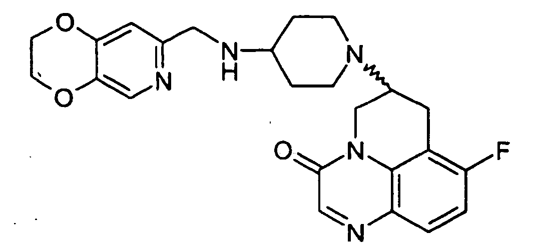

### (a) (6R/S)-6-(4-amino-1-piperidinyl)-8-fluoro-6,7-dihydro-3H,5H-pyrido[1,2,3-de]quinoxalin-3-one

1,1-Dimethylethyl {1-[(6R/S)-8-fluoro-3-oxo-6,7-dihydro-3H,5H-pyrido[1,2,3-de]quinoxalin-6-yl]-4-piperidinyl}carbamate (0.88g, 2.19 mmol) was dissolved in dichloromethane (16mL) and methanol (10 mL) and treated with 4M hydrogen chloride I 1,4-dioxane (16 mL). After stirring for 1.5h at room temperature, the mixture was evaporated. The residue was partially dissolved in 10% methanol/dichloromethane (50 mL) and stirred with MP-carbonate resin (2.69 mmol/g, 3.27g) for approx. 2h. The resin was filtered off, washed several times with 10% methanol/dichloromethane and methanol alternately, and the filtrate was evaporated. The crude product was chromatographed on silica, eluting with 20-50% methanol/dichloromethane to give the amine (0.51g, 77%). LC-MS (+ve ion electrospray): m/z 302 [MH+]

### (b) Title compound

A solution of (6R/S)-6-(4-amino-1-piperidinyl)-8-fluoro-6,7-dihydro-3H,5H-pyrido[1,2,3-de]quinoxalin-3-one (0.10g, 0.33 mmol) and 2,3-dihydro[1,4]dioxino[2,3-c]pyridine-7-carboxaldehyde (for a synthesis see WO2004058144, Example 2(c) or WO03/087098, Example 19(d)) (55 mg, 0.33 mmol) in dry 1:1 chloroform/methanol (10 ml) was heated under reflux with 3A molecular sieves overnight. After cooling, sodium triacetoxyborohydride (0.28g, 1.32 mmol) was added and the mixture was stirred at room temperature for 8h, then allowed to stand overnight. Aqueous sodium bicarbonate was added to basify, and the phases were separated. The aqueous phase was extracted three times with 10% methanol/dichloromethane, and the organic fractions were dried and evaporated. Chromatography on silica, eluting with 0-20% methanol/dichloromethane, gave the free base (131mg, 88%).
δH (CDCl₃, 250MHz) 1.44 (2H, m), 1.92 (2H, m), 2.39 (2H, m), 2.53 ( 1H, m), 2.70-3.10 (4H, complex m), 3.22 (1H, d), 3.63 (1H, dd), 3.81 (2H, s), 4.30 (4H, m), 4.65 (1H, d), 6.82 (1H,s), 7.05 (1H, t), 7.70 (1H, dd), 8.11 (1H, s), 8.23 (1H, s).
LC-MS (+ve ion electrospray): m/z 452 [MH+]

The free base (31 mg) in dichloromethane/methanol was treated with excess 0.4M hydrogen chloride in 1,4-dioxane (0.34mL), and the solvent was evaporated to give the title dihydrochloride salt (35mg). The racemic material (100 mg) was partially resolved on Chiralpak IA using acetonitrile:THF:isopropylamine. The enriched second eluting enantiomer (E2) was then further resolved on Chiralpak IA using CH₃OH:CH₃CN:isopropanol:isopropylamine to give 28 mg of free base which was converted to the mono hydrochoride salt (30 mg ) with 1 equivalent of hydrochloric acid.

### Examples 21 and 22 6-{4-[(6,7-Dihydro[1,4]dioxino[2,3-c]pyridazin-3-ylmethyl)amino]-1-piperidinyl}-8-fluoro-6,7-dihydro-3H,5H-pyrido[1,2,3-de]quinoxalin-3-one hydrochloride, Enantiomers 1 and 2

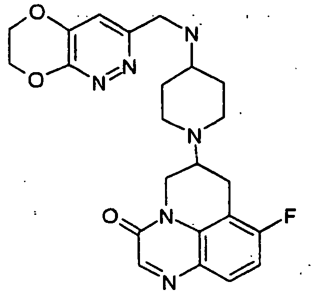

A solution of (6R/S)-6-(4-amino-1-piperidinyl)-8-fluoro-6,7-dihydro-3H,5H-pyrido[1,2,3-de]quinoxalin-3-one (for a preparation see Example 20) (0.10g, 0.33 mmol) and 6,7-dihydro[1,4]dioxino[2,3-c]pyridazine-3-carbaldehyde (for a preparation see Example 3(e)) (55 mg, 0.33 mmol) in dry 1:1 chloroform/methanol (10 ml) was heated under reflux with 3A molecular sieves overnight. After cooling, sodium triacetoxyborohydride (0.28g, 1.32 mmol) was added and the mixture was stirred at room temperature for 8h, then allowed to stand overnight. Aqueous sodium bicarbonate was added to basify, and the phases were separated. The aqueous phase was extracted three times with 10% methanol/dichloromethane, and the organic fractions were dried and evaporated. Chromatography on silica, eluting with 0-20% methanol/dichloromethane, gave the free base (118mg, 79%).
δH (CDCl₃, 250MHz) 1.41 (2H, m), 1.94 (2H, br. d), 2.38 (2H, q), 2.55 ( 1H, m), 2.70-3.10 (4H, complex m), 3.21 (1H, d), 3.67 (1H, dd), 4.01 (2H, s), 4.37 (2H, m), 4.51 (2H, m), 4.62 (1H, d), 7.04 (1H,s) overlapping 7.05 (1H, t), 7.70 (1H, dd), 8.23 (1H, s).
LC-MS (+ve ion electrospray): m/z 453 [MH+]

The free base (28mg) in dichloromethane/methanol was treated with 0.4M hydrogen chloride in 1,4-dioxane (0.31mL), and the solvent was evaporated to give the dihydrochloride salt (33mg).

Racemic 6-{4-[(6,7-Dihydro[1,4]dioxino[2,3-c]pyridazin-3-ylmethyl)amino]-1-piperidinyl}-8-fluoro-6,7-dihydro-3H,5H-pyrido[1,2,3-de]quinoxalin-3-one (90mg) was separated by preparative HPLC on a 2 1 x 250mm Chiralpak AS-H® column, 4 injections, eluting with acetonitrile/methanol/isopropylamine (98/2/0.1, 20ml/min, UV monitoring at 254nm) to give the two enantiomers E1 (retention time 6.9min.) and E2 (retention time 7.5min.).

The separate isomers in methanol were both treated with one equivalent of aqueous hydrochloric acid, and the solvent was evaporated to give the hydrochloride salts.

### Example 23 (5R/S)-5-({4-[(6,7-Dihydro[1,4]dioxino[2,3-c]pyridazin-3-ylmethyl)amino]-1-piperidinyl}methyl)-7-(methyloxy)-5,6-dihydro-3H-pyrrolo[1,2,3-de]quinoxalin-3-one dihydrochloride

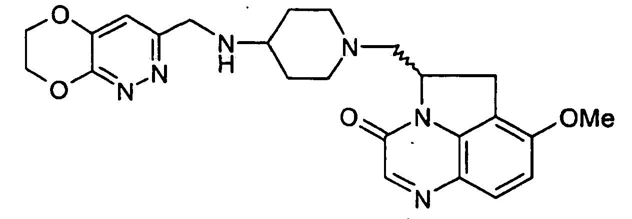

### (a) (5R/S)-5-[(4-Amino-1-piperidinyl)methyl]-7-(methyloxy)-5,6-dihydro-3H-pyrrolo[1,2,3-de]quinoxalin-3-one

A solution of (5R/S)-5-[(4-amino-1-piperidinyl)methyl]-7-fluoro-5,6-dihydro-3H-pyrrolo[1,2,3-de]quinoxalin-3-one (for a preparation see Example 18(o)) (0.30g, 1 mmol) in dry methanol (5mL) was treated with sodium methoxide (25% in methanol, 0.65mL, 3 mmol) and heated under reflux overnight. The cooled mixture was treated with aqueous ammonium chloride (6 drops) and evaporated to dryness. The residue was extracted three times with 10% methanol/dichloromethane, the extracts were filtered and evaporated. The crude product was chromatographed on silica, eluting with 0-20% (2M ammonia in methanol)/dichloromethane to give the amine (0.20g, 64%).
LC-MS (+ve ion electrospray): m/z 315 [MH+]

### (b) Title compound

A solution of (5R/S)-5-[(4-amino-1-piperidinyl)methyl]-7-(methyloxy)-5,6-dihydro-3H-pyrrolo[1,2,3-de]quinoxalin-3-one (0.20g, 0.64 mmol) and 6,7-dihydro[1,4]dioxino[2,3-c]pyridazine-3-carbaldehyde (for a preparation see Example 3(e)) (116 mg, 0.70 mmol) in dry 1:1 chloroform/methanol (16 ml) was heated under reflux with 3A molecular sieves overnight. After cooling, sodium triacetoxyborohydride (0.54g, 2.55 mmol) was added and the mixture was stirred at room temperature overnight. Aqueous sodium bicarbonate was added to basify, and the phases were separated. The aqueous phase was extracted several times with 10% methanol/dichloromethane, and the organic fractions were dried and evaporated. Chromatography on silica, eluting with 0-20% methanol/dichloromethane, gave the free base (185mg, 62%).
δH (CDCl₃, 250MHz) 1.40 (2H, m), 1.88 (2H, m, part. obscured by water), 2.24 (2H, m), 2.48 (1H, m), 2.52 (1H, m), 2.72 ( 1H, br. d), 3.02 (1H, br. d), 3.17 (1H, dd), 3.39 (1H, m), 3.96 (3H, s), 4.00 (2H, s), 4.38 (2H, m), 4.51 (2H, m), 5.04 (1H, m), 6.87 (1H, d), 7.03 (1H,s), 7.63 (1H, d), 8.05 (1H, s).
LC-MS (+ve ion electrospray): *m*/*z* 465 [MH⁺]

The free base in chloroform/dichloromethane/methanol was treated with excess 0.4M hydrogen chloride in 1,4-dioxane (1.99mL), and the solvent was evaporated to give the dihydrochloride salt (213mg).

### Examples 24 (5R/S)-5-{4-[(2,3-Dihydro[1,4]dioxino[2,3-c]pyridin-7-ylmethyl)amino]-1-piperidinyl}-3-fluoro-5,6-dihydro-4H,8H-pyrido[3,2,1-de]-1,5-naphthyridin-8-one dihydrochloride

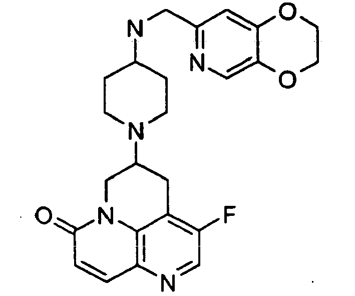

A solution of 1,1-dimethylethyl {1-[(5R/S)-3-fluoro-8-oxo-5,6-dihydro-4H,8H-pyrido[3,2,1-*de*]-1,5-naphthyridin-5-yl]-4-piperidinyl}carbamate (for a preparation see Example 1(e)) (35 mg, 0.09 mmol) was dissolved in dichloromethane and trifluoroacetic acid (1 ml/1 ml) and stirred for 1 hour then evaporated . The residue was dissolved in dichloromethane/methanol and treated with MP-carbonate resin until a moistened pH paper indicated pH 8.. The mixture was filtered, washing with methanol and evaporated affording an oil. Analysis indicated a 4:1 mixture of the anticipated (5R/S)-5-(4-amino-1-piperidinyl)-3-fluoro-5,6-dihydro-4H,8H-pyrido[3,2,1-*de*]-1,5-naphthyridin-8-one and 3-fluoro-6H,8H-pyrido[3,2,1-*de*]-1,5-naphthyridin-8-one. This material was dissolved in chloroform/methanol (1 ml/0.1 ml) and treated with 2,3-dihydro[1,4]dioxino[2,3-c]pyridine-7-carboxaldehyde (for a synthesis see WO2004058144, Example 2(c) or WO03/087098, Example 19(d)) (14 mg, 0.09 mmol) and sodium triacetoxyborohydride (55 mg, 0.3 mmol). After 2 hours the mixture was added to a silica column and chromatographed eluting with 0-30% methanol in dichloromethane affording the monoacetate salt of the free base of the title compound as an oil (30 mg).
δH (CD₃OD, 250 MHz) 1.50-1.70 (2H, m), 1.95 (3H, s), 2.10-2.30 (2H, m), 2.35-2.50 (2H, m), 3.05-3.25 (4H, m), 4.05-4.20 (3H, m), 4.25-4.40 (7H, m), 6.85 (1H, d), 7.00 (1H, s), 7.95 (1H, d), 8.10 (1H, s), 8.45 (1H, s)

This material was treated with excess hydrochloric acid in methanol and evaporated to afford the dihydrochloride salt (ca 30 mg).
MS (+ve ion electrospray) m/z 452 (MH+).

### Examples 25 and 26 5-({4-[(2,3-Dihydro[1,4]dioxino[2,3-c]pyridin-7-ylmethyl)amino]-1-piperidinyl}methyl)-3-(methyloxy)-4,5-dihydro-7H-pyrrolo[3,2,1-de]-1,5-naphthyridin-7-one hydrochloride, Enantiomers 1 and 2

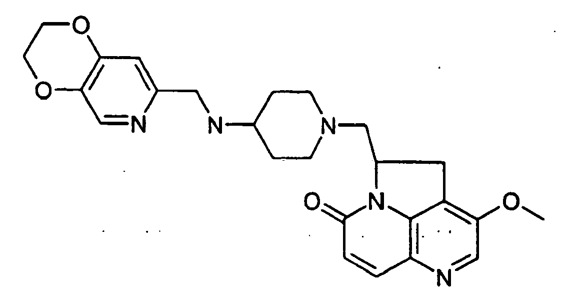

### (a) 5-[(4-Amino-1-piperidinyl)methyl]-3-(methyloxy)-4,5-dihydro-7H-pyrrolo[3,2,1-de]-1,5-naphthyridin-7-one

A solution of (5R/S)-5-[(4-amino-1-piperidinyl)methyl]-3-fluoro-4,5-dihydro-7H-pyrrolo[3,2,1-de]-1,5-naphthyridin-7-one (for a preparation see Example 1(f)) (332 mg, 1.1 mmol) in methanol (25 ml) was treated with sodium methoxide (25% solution in methanol, 1.0 ml, 4.6 mmol) and heated to 60°C for 6 hours. More sodium methoxide (25% solution in methanol, 1.0 ml, 4.6 mmol) was added and the mixture heated at 60°C for 17 hours. The mixture was cooled in an ice bath and taken to ca pH8 with saturated aqueous ammonium chloride then evaporated to dryness. The residue was suspended in methanol and added to an SCX column, washing off with 2M methanolic ammonia. Evaporation afforded an orange gum (389 mg,).
MS (ES+) m/z 315 (MH⁺).

### (b) Title compounds

A solution of 5-[(4-amino-1-piperidinyl)methyl]-3-(methyloxy)-4,5-dihydro-7H-pyrrolo[3,2,1-de]-1,5-naphthyridin-7-one (170 mg) and 2,3-dihydro[1,4]dioxino[2,3-c]pyridine-7-carboxaldehyde (for a synthesis see WO2004058144, Example 2(c) or WO03/087098, Example 19(d)) (89 mg) in dichloromethane/methanol (10 ml/2 ml) was treated with sodium triacetoxyborohydride (343 mg, 0.52 mmol) and stirred for 20 hours then treated with saturated aqueous sodium bicarbonate solution. The phases were separated and the aqueous phase further extracted three times with 10% methanol in dichloromethane. The combined extracts were washed with brine, dried and evaporated. Chromatography eluting with 0-30% methanol in dichloromethane afforded the free base of the title compounds as a yellow gum (121 mg). The free base (20 mg) was dissolved in chloroform (0.33 ml) and treated with I M hydrochloric acid in ether (0.5 ml) followed by ether (1 ml). Removal of solvent gave the product (23 mg).
¹H NMR (400MHz) δ (CDCl₃) 1.30-1.45 (2H, m), 1.75-1.95 (2H, m), 2.18 (1H, t), 2.25 (1H, t), 2.50 (2H, m), 2.65 (1H, d), 3.05 (1H, d), 3.15 (1H, dd), 3.45 (1H, dd), 3.55 (1H, dd), 3.80 (2H, s), 4.08 (3H, s) , 4.30 (2H, m), 4.38 (2H, m), 5.05 (1H, m), 6.68 (1H, d), 6.80 (1H, s), 7.82 (1H, d), 8.10 (1H, s), 8.22 (1H, s),
MS (ES+) m/z 464 (MH⁺).

Chromatography of a portion (78 mg) of this material (free base) on a Chiralpak AS-H column eluting with a water/acetonitrile gradient (1% isopropylamine present) afforded separate enantiomers (Rt Enantiomer 1, 2.4 minutes, alpha_{D} +133 (c = 0.5 in methanol), Rt Enantiomer 2, 3.9 minutes, alpha_{D} -143 (c = 0.9 in methanol)). Each material was then converted to the separate title compounds with one equivalent of hydrochloric acid (25 mg E1 and 28 mg E2).

### Example 27 5-({4-[(2,3-Dihydro[1,4]dioxino[2,3-c]pyridin-7-ylmethyl)amino]-1-piperidinyl}methyl)-7-oxo-4,5-dihydro-7H-pyrrolo[3,2,1-de]-1,5-naphthyridine-3-carbonitrile diformate

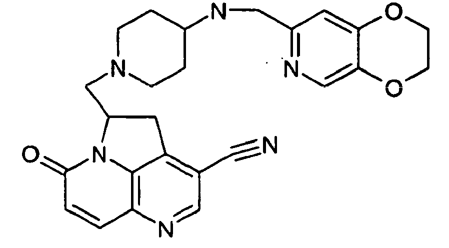

### (a) 6-(Methyloxy)-4-(2-propen-1-yl)-1,5-naphthyridine-3-carbonitrile

A solution of 4-bromo-6-(methyloxy)-1,5-naphthyridine-3-carbonitrile (prepared from 4-bromo-6-(methyloxy)-1,5-naphthyridine-3-carboxylic acid (WO2004058144, Example 53(d)) *via* conversion to 4-bromo-6-(methyloxy)-1,5-naphthyridine-3-carboxamide with oxalyl bromide then ammonia and then treatment of 4-bromo-6-(methyloxy)-1,5-naphthyridine-3-carboxamide with trifluoromethane sulfonic anhydride and triethylamine to give 4-bromo-6-(methyloxy)-1,5-naphthyridine-3-carbonitrile) (200mg, 0.76 mmol) in degassed 1,4-dioxane (2 ml) was treated with tris(dibenzylideneacetone)dipalladium(0), (6.9 mg, 0.008 mmol), bis(tri-tert-butylphosphine)palladium(0) (7.8 mg, 0.015 mmol), allyl tributylstannane (0.2 ml, 0.76 mmol) and cesium fluoride (253mg, 1.7 mmol). The reaction mixture was heated at 70°C under argon for 2 hours, cooled and partitioned between water and ethyl acetate. The aqueous was extracted further with ethyl acetate and the combined organic phases were dried, evaporated and chromatographed (15% ethyl acetate/hexane) to afford the desired compound (156 mg; 92%).
MS (ES+) m/z 226 (MH⁺).

### (b) 4-(2,3-Dihydroxypropyl)-6-(methyloxy)-1,5-naphthyridine-3-carbonitrile

A solution of 6-(methyloxy)-4-(2-propen-1-yl)-1,5-naphthyridine-3-carbonitrile (60mg, 0.0.27 mmol) in 1:1 THF:water (1 ml) was treated with sodium chlorate (43 mg, 0.4 mmol)and then osmium tetroxide (0.01ml, 4% aqueous solution, 0.00134 mmol) The reaction mixture was stirred for 6h, then evaporated, treated with acetonitrile, sonicated for 5 minutes and then filtered.. The filtrate was evaporated and the residue chromatographed (80% ethyl acetate/hexane) to afford the desired compound as a white solid (39 mg; 56%).
MS (ES+) m/z 260 (MH⁺).

### (c) 1,1-Dimethylethyl (1-{3-[3-cyano-6-(methyloxy)-1,5-naphthyridin-4-yl]-2-hydroxypropyl}-4-piperidinyl)carbamate

A solution of 4-(2,3-dihydroxypropyl)-6-(methyloxy)-1,5-naphthyridine-3-carbonitrile (3 g, 11.6 mmol) in DCM (150 ml) at 0°C under argon was treated with triethylamine (3.22ml, 23.2 mmol) and para-toluenesulfonic anhydride (5.28g, 16.2 mmol) and stirred at 0°C for 1hour. Ethanol was added and the reaction stirred for a further 0.5 hour, reaction was evaporated, treated with ethanol and evaporated again. The residue was suspended in ethanol (100ml) and treated with 1,1-dimethylethyl 4-piperidinylcarbamate (4.6 g, 23 mmol) and sodium carbonate (3.87g, 34.8 mmol) and then stirred at 60°C under argon for 24 hours. The reaction was then partitioned between water and DCM, and the aqueous phase extracted twice more with DCM. The combined organic phases were dried, evaporated and chromatographed (99:1:0.1 DCM:Methanol: 0.88 ammonia) to afford the desired compound (2.92g; 57%).
MS (ES+) m/z 442 (MH⁺).

### (d) 1,1-Dimethylethyl {1-[(3-cyano-7-oxo-4,5-dihydro-7H-pyrrolo[3,2,1-de]-1,5-naphthyridin-4-yl)methyl]-4-piperidinyl}carbamate

A solution of 1,1-dimethylethyl (1-{3-[3-cyano-6-(methyloxy)-1,5-naphthyridin-4-yl]-2-hydroxypropyl}-4-piperidinyl)carbamate (2.15 g, 4.87 mmol) in DCM (40 ml) at 0°C under argon was treated with sodium carbonate (2.12g, 20 mmol) and methanesulfonic anhydride (1.74g, 10 mmol) and stirred for 4 hours. More sodium carbonate (2.12g, 20 mmol) and methanesulfonic anhydride (1.74g, 10 mmol) were added and the reaction was allowed to stir overnight. The solvent was then evaporated, then the residue dissolved in chloroform (40 ml) and treated with sodium carbonate (2.12g, 20 mmol) and methanesulfonic anhydride (1.74g, 10 mmol). The reaction mixture was stirred for 6 hours, then partitioned between water and DCM, and the aqueous phase separated twice more with DCM. The combined organic phases were dried, evaporated and chromatographed (98:2:0.2 DCM:methanol: 0.88 ammonia) to afford a mixture of the desired compound and starting material (1.7g). Some of this material (1.26g) was treated with sodium carbonate (6.36g) and methanesulfonic anhydride (5.22g) in DCM (20 ml) at 0°C under argon and stirred for 4 hours and then worked up again as above and purified by MDAP (Mass Directed Auto Purification) to give the desired product (65 mg).
MS (ES+) m/z 410 (MH⁺).

### (e) 4-[(4-Amino-1-piperidinyl)methyl]-7-oxo-4,5-dihydro-7H-pyrrolo[3,2,1-de]-1,5-naphthyridine-3-carbonitrile

A solution of 1,1-dimethylethyl {1-[(3-cyano-7-oxo-4,5-dihydro-7*H-*pyrrolo[3,2,1-*de*]-1,5-naphthyridin-4-yl)methyl]-4-piperidinyl}carbamate (84 mg) in DCM (4 ml) was treated with trifluoroacetic acid and stirred for 1 hour. The reaction mixture was then evaporated to dryness then redissolved and passed through a column of Amberlyst A-21 basic resin, eluting with methanol. The fractions containing the desired compound were then evaporated and chromatographed (99:1:0.1 DCM:Methanol: 0.88 ammonia) to afford the desired compound (41mg; 64%).
MS (ES+) m/z 310 (MH⁺).

### (f) Title compound

A solution of 4-[(4-amino-1-piperidinyl)methyl]-7-oxo-4,5-dihydro-7*H-*pyrrolo[3,2,1-*de*]-1,5-naphthyridine-3-carbonitrile (41mg, 0.113 mmol) and 2,3-dihydro[1,4]dioxino[2,3-*c*]pyridine-7-carboxaldehyde (22mg, 0.113mmol)(for a synthesis see WO2004058144, Example 2(c) or WO03/087098, Example 19(d)) in 1:1 methanol:chloroform (2 ml) with 3A molecular sieves was heated at 65°C for 4 hours, cooled in ice and treated with sodium triacetoxyborohydride (56 mg, 0.266 mmol) and stirred overnight. The reaction was then partitioned between water and 20% methanol:DCM, and the aqueous phase extracted twice more with 20% methanol:DCM. The combined organic phases were dried, evaporated and purified by MDAP (Mass Directed Auto Purification), evaporated and triturated with diethyl ether to afford the desired compound (40 mg; 54%).
¹H NMR (400MHz) δ(mixture of CD30D:CDCl₃) 1.69 (m, 2H), 2.05 (m, 2H), 2.30 (m, 2H), 2.60 (m, 1H), 2.80 (m, 1H), 3.0 (m, 1H), 3.15 (m, 2H), 3.72 (m, 2H), 4.12(s, 2H) , 4.48 (m, 4H), 5.12 (m, 1H), 7.01 (m, 2H), 8.02 (d, 1H), 8.30 (s, 1H), 8.41 (br s, 2H), 8.71 (s, 1H)
MS (ES+) m/z 459(MH⁺).

### Examples 28 and 29 5-({4-[(2,3-Dihydro[1,4]dioxino[2,3-c]pyridin-7-ylmethyl)amino]-1-piperidinyl}methyl)-7-fluoro-5,6-dihydro-3H-pyrrolo[1,2,3-de]quinoxalin-3-one benzoate, Enantiomers E1 and E2

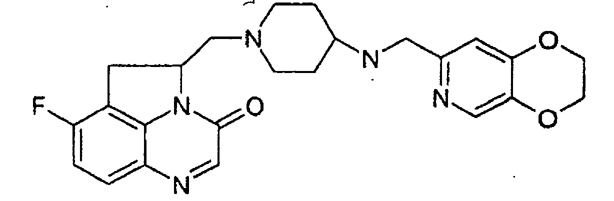

A mixture of (5R/S)-5-[(4-amino-1-piperidinyl)methyl]-7-fluoro-5,6-dihydro-3*H-*pyrrolo[1,2,3-*de*]quinoxalin-3-one (for a preparation see Example 18(o)) (200 mg, 0.66 mmol), 2,3-dihydro[1,4]dioxino[2,3-c]pyridine-7-carboxaldehyde (for a synthesis see W02004058144, Example 2(c) or WO03/087098, Example 19(d)) (130 mg, 0.8 mmol), triethylamine (0.2 ml), in methanol/chloroform (10 ml/10 ml) was heated to reflux overnight in the presence of 3A molecular sieves. The mixture was treated at room temperature with sodium triacetoxyborohydride (367 mg) and stirred for 7 hours then treated with saturated aqueous sodium bicarbonate solution. The phases were separated and the aqueous phase further extracted several times with 10% methanol in dichloromethane. The combined extracts were dried and evaporated affording a yellow solid (320 mg). Chromatography eluting with 0-20% methanol in dichloromethane afforded the free base of the title compounds as a yellow gum (87 mg, 30%).
¹H NMR (250 MHz, CDCl₃) 1.42 (2H, m), 1.89 (2H, br t), 2.10-2.35 (2H, m), 2.50 (2H, m), 2.70 (1H, d), 3.01 (1H, dd), 3.12 (1H, dd), 3.49 (2H, m), 3.80 (2H, s), 4.30 (4H, m), 5.08 (1H, m), 6.82 (1H, s), 6.99 (1H, t), 7.65 (1H, dd), 8.10 (1H, s), 8.15 (1H, s)
MS (ES+) m/z 452 (MH⁺).

This material was converted to the corresponding fumarate (98 mg) by adding 1 equivalent of fumaric acid to a dichloromethane solution of the free base then evaporating. Chromatography of a portion (70 mg) of this material on a Chiralpak AS-H column eluting with a water/acetonitrile gradient (1% TFA present) afforded separate enantiomers (Rt Enantiomer 1, 5.6 min minutes; Rt Enantiomer 2, 10.6 minutes, baseline separation). Each material was then converted to the separate title compounds with one equivalent of benzoic acid (24 mg E1 and 23 mg E2).

### Examples 30 and 31 7-Fluoro-5-[(4-{[(3-oxo-3,4-dihydro-2H-pyrido[3,2-b][1,4]oxazin-6-yl)methyl]amino}-1-piperidinyl)methyl]-5,6-dihydro-3H-pyrrolo[1,2,3-de]quinoxalin-3-one, Enantomers E1 and E2

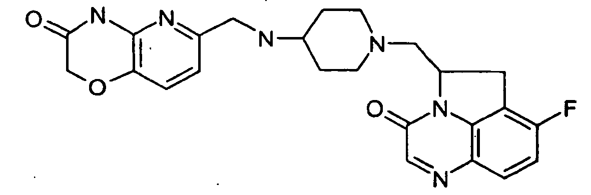

A mixture of (5R/S)-5-[(4-amino-1-piperidinyl)methyl]-7-fluoro-5,6-dihydro-3*H-*pyrrolo[1,2,3-*de*]quinoxalin-3-one (for a preparation see Example 18(o)) (50 mg, 0.17 mmol) and 3-oxo-3,4-dihydro-2H-pyrido[3,2-b][1,4]oxazine-6-carboxaldehyde (for a synthesis, see WO2004058144, Example 1(l)) (35 mg, 0.2 mmol) in chloroform/methanol (3 ml/3 ml) was heated under reflux with 3A molecular sieves overnight. The mixture was treated at room temperature with sodium triacetoxyborohydride (110 mg, 0.52 mmol) and stirred for 24 hours then treated with saturated aqueous sodium bicarbonate solution. The phases were separated and the aqueous phase further extracted three times with 10% methanol in dichloromethane. The combined extracts were dried and evaporated. Chromatography eluting with 0-20% methanol in dichloromethane afforded the free base of the title compounds as a yellow gum (55 mg, 71%).
¹H NMR (250 MHz, CDCl₃) 1.45 (2H, m), 1.89 (2H, br t), 2.15-2.35 (2H, m), 2.52 (2H, m), 2.72 (1H, br d), 3.02 (1H, br d), 3.15 (1H, dd), 3.48 (2H, m), 3.84 (2H, s), 4.64 (2H, s), 5.07 (1H, m), 6.98 (2H, m), 7.20 (1H, d), 7.65 (1H, dd), 8.15 (1H, s)
MS (ES+) m/z 465(MH⁺)

This material was converted to the corresponding dihydrochloride (59 mg) by adding 2 equivalents of hydrochloric acid in 1,4-dioxane to a chloroform/dichloromethane solution of the free base then evaporating. Chromatography of a portion (30 mg) of this material on a Chiralpak AS-H column eluting with 85:15:0.1 acetonitrile:methanol:isopropylamine afforded separate enantiomers (Rt Enantiomer 1, 5.5 min minutes; Rt Enantiomer 2, 7.0 minutes), giving 9.5 mg E1 and 9.5 mg E2.

### Example 32 8-Fluoro-6-(4-{[(3-oxo-3,4-dihydro-2H-pyrido[3,2-b][1,4]oxazin-6-yl)methyl]amino}-1-piperidinyl)-6,7-dihydro-3H,5H-pyrido[1,2,3-de]quinoxalin-3-one dihydrochloride, Enantiomer E1

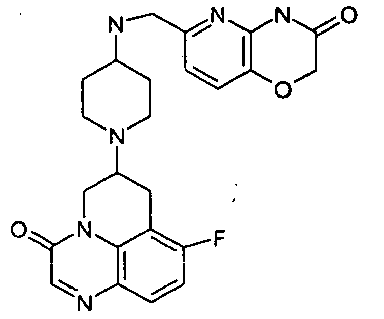

(a) 6-(4-Amino-1-piperidinyl)-8-fluoro-6,7-dihydro-3H,5H-pyrido[1,2,3-de]quinoxalin-3-one, Enantiomers E1 and E2

(6R/S)-6-(4-Amino-1-piperidinyl)-8-fluoro-6,7-dihydro-3H,5H-pyrido[1,2,3-de]quinoxalin-3-one (for a preparation see Example 20(a)) (500 mg) was chromatographed on a Chiralpak AS-H column eluting with 85:15:0.1 acetonitrile:methanol:isopropylamine afforded separate enantiomers (Rt Enantiomer 1, 5.3 minutes; Rt Enantiomer 2, 13.6 minutes), giving 189 mg E1 and 159 mg E2.

### (b) Title compound

A mixture of 6-(4-amino-1-piperidinyl)-8-fluoro-6,7-dihydro-3H,5H-pyrido[1,2,3-de]quinoxalin-3-one, Enantiomer E1 (100 mg, 0.33 mmol) and 3-oxo-3,4-dihydro-2H-pyrido[3,2-b][1,4]oxazine-6-carboxaldehyde (for a synthesis, see WO2004058144, Example 1(1)) (60 mg, 0.33 mmol) in chloroform/methanol (5 ml/5 ml) with 3A molecular sieves and acetic acid (16 drops) was stirred at room temperature for 1 hour then sodium cyanoborohydride (84 mg) was added. After 3 hours saturated aqueous sodium bicarbonate solution was added. The phases were separated and the aqueous phase further extracted with 10% methanol in dichloromethane. The combined extracts were dried and evaporated. Chromatography eluting with 0-20% methanol in dichloromethane afforded a yellow gum (40 mg). This material was converted to the dihydrochloride salt by treatment with 2 equivalents of hydrochloric acid in 1,4-dioxane (40 mg). This material was dissolved in dichloromethane/methanol (1 ml/6 ml) and treated with MP-carbonate resin followed by manganese(II) oxide. After 2 hours the mixture was filtered and evaporated to give the free base of the title compound (28 mg). This material was converted to the dihydrochloride salt by treating a solution of the free base with 2 equivalents of HCl in 1,4-dioxane followed by evaporation affording the title compound as an off-white solid (25 mg).
¹H NMR (di-HCl salt) (250 MHz, d6-DMSO) 2.19 (2H, br), 2.38 (2H, br), 3.65 (2H, br), 3.96 (2H, br), 4.18 (2H, s), 4.71 (2H, s), 4.89 (1H, br), 7.24 (1H, d), 7.30 (1H, t), 7.46 (1H, d), 7.81 (1H, m), 8.25 (1H, s), 9.59 (2H, br).
MS (ES+) m/z 465(MH⁺)

### Example 33 8-Fluoro-6-(4-{[(3-oxo-3,4-dihydro-2H-pyrido[3,2-b][1,4]oxazin-6-yl)methyl]amino}-1-piperidinyl)-6,7-dihydro-3H,5H-pyrido[1,2,3-de]quinoxalin-3-one dihydrochloride, Enantiomer E2

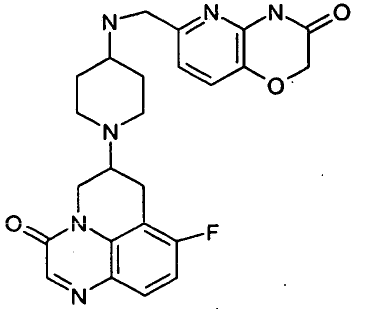

This was prepared from 6-(4-amino-1-piperidinyl)-8-fluoro-6,7-dihydro-3H,5H-pyrido[1,2,3-de]quinoxalin-3-one, Enantiomer E2 by the procedure of Example 32, yielding the title product as an off-white solid (32 mg).
¹H NMR (di-HCl salt) (250 MHz, CD₃OD) 1.72 (2H, br ), 2.22 (2H, br), 2.58 (2H, br), 3.11 (2H, m), 3.69 (2H, m), 4.07 (1H, m), 4.26 (2H, s), 4.44 (1H, d), 4.70 (2H, s), 7.09 (1H, d), 7.18 (1H, t), 7.37 (1H, d), 7.75 (1H, m), 8.18 (1H, s).
MS (ES+) m/z 465(MH⁺)

### Example 34 7-Fluoro-5-[(4-{[(7-oxo-1,5,6,7-tetrahydropyrido[2,3-d]pyrimidin-2-yl)methyl]amino}-1-piperidinyl)methyl]-5,6-dihydro-3H-pyrrolo[1,2,3-de]quinoxalin-3-one hydrochloride

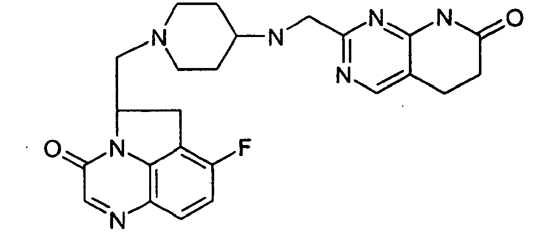

### (a) 3-Ethyl 1,1-dimethyl 1,1,3-propanetricarboxylate

To a solution of dimethyl malonate (2.5 g, 18.9 mmol) in anhydrous THF (20 mL) was added NaH (0.038g, 0.95 mmol, 60% in mineral oil). The reaction was stirred at ambient temperature for 15 minutes. In a separate flask, ethyl acrylate (1.02mL, 9.45mmol) was dissolved in anhydrous THF (1mL) and then added dropwise over 30 minutes to the dimethyl malonate solution. The reaction was stirred at ambient temperature overnight and then concentrated under vacuum. The residue was dissolved in EtOAc, washed with saturated NH₄Cl solution and brine. The organic phase was dried over Na₂SO₄, filtered, and concentrated under vacuum. The crude residue was purified by column chromatography (silica gel) using an EtOAc/hexanes gradient to yield the desired compound (1.68 g, 77%).
¹H NMR (400 MHz, CDCl₃) δ 1.24 (t, *J*=7.07 Hz, 3 H) 2.20 (q, *J*=7.24 Hz, 2 H) 2.37 (t, *J*=7.33 Hz, 2 H) 3.47 (t, *J*=7.33 Hz, 1H) 3.70 - 3.75 (m, 6 H) 4.12 (q, *J*=7.24 Hz, 2 H).

### (b) (2E)-3-Phenyl-2-propenimidamide

Cinnamonitrile (25.0 g, 194 mmol) was dissolved in EtOH. The solution was cooled to 0 °C and HCl gas bubbled through the solution for 30 minutes. The solution was stirred at ambient temperature for 1 h and then concentrated under vacuum. The residue was dissolved in EtOH (100 mL), cooled to 0°C and a solution of NH₃/MeOH (7M, 69 mL, 484 mmol) was added dropwise through an addition funnel. Once added, the solution was allowed to warm to ambient temperature and stirred overnight and the resulting NH₄Cl was filtered off. The solution was concentrated under vacuum and the resulting product was used without further purification (28.6 g crude).
LC-MS: m/z 147.4 (MH+).

### (c) Ethyl 3-{4-hydroxy-6-oxo-2-[(E)-2-phenylethenyl]-1,6-dihydro-5-pyrimidinyl}propanoate

3-Ethyl 1,1-diethyl 1,1,3-propanetricarboxylate (1.65 g, 7.11 mmol) and (2*E*)-3-phenyl-2-propenimidamide (1.04 g, 7.11 mmol) were combined in EtOH (36 mL). Triethylamine (1.98 mL, 14.2 mmol) was added and the solution was heated at reflux for 3 h with no change based on LC-MS. The solution was cooled to room temperature and treated with NaOMe in MeOH (1.0 mL, 5.33 mmol, 25-30% w/w solution) and the solution was refluxed for 3 h. Another two portions of NaOMe in MeOH (2 x 1.0 mL) were added and the solution was refluxed overnight. After this time, a yellow precipitate had formed which was filtered off. The mother liquor was acidified to pH 2 with 1N HCl, and the solution was concentrated under vacuum. The resulting material was combined with the yellow solid and used without further purification.
LC-MS: m/z 315.2 (MH+).

### (d) Ethyl 3-{4,6-dichloro-2-[(E)-2-phenylethenyl]-5-pyrimidinyl}propanoate

Crude ethyl 3-{4-hydroxy-6-oxo-2-[(*E*)-2-phenylethenyl]-1,6-dihydro-5-pyrimidinyl}propanoate (7.1mmol) was dissolved in POCl₃ (25 mL) and N,N-dimethylaniline (0.862g, 0.9 mL, 7.1 mmol) was slowly added to the solution. The reaction was then heated at reflux for 2 h. After cooling to ambient temperature, the resulting solution was carefully and slowly added to ice water to quench the excess POCl_{3.} The mixture was extracted with EtOAc (3X) and concentrated under vacuum. The crude residue was then purified by column chromatography (silica gel) using an EtOAc/hexanes gradient to yield the desired compound as a yellow solid (0.48 g, 19% over 2 steps).
LC-MS: m/z 351.4 (MH+).

### (e) 4-Chloro-2-[(E)-2-phenylethenyl]-5,8-dihydropyrido[2,3-d]pyrimidin-7(6H)-one

To a solution of ethyl 3-{4,6-dichloro-2-[(*E*)-2-phenylethenyl]-5-pyrimidinyl}propanoate (0.42 g, 1.19 mmol) in 1,4-dioxane (5mL) was added conc. NH₄OH (3.5 mL). The reaction was heated at 75 °C in a sealed tube overnight. The solution was concentrated under vacuum, diluted with water, and extracted with EtOAc/DCM. The organic layer was washed with brine, dried over Na₂SO_{4,} and concentrated under vacuum. The crude residue was then purified by column chromatography (silica gel) to yield the desired compound (0.072 g, 21%).
LC-MS: m/z 286.2 (MH+).
Also obtained was 3-{4-amino-6-chloro-2-[(*E*)-2-phenylethenyl]-5-pyrimidinyl}propanamide (0.175g).
LC-MS: m/z 303.3(MH+).

3-{4-Amino-6-chloro-2-[(*E*)-2-phenylethenyl]-5-pyrimidinyl}propanamide (0.175 g, 0.58 mmol) was dissolved in EtOH and HCl gas was bubbled through the solution until saturated. The solution was heated at reflux for 2 h, cooled to ambient temperature and concentrated under vacuum. The residue was dissolved in water, neutralised to pH9 with K₂CO₃ solution and extracted with EtOAc (3X). The organic layers were combined, dried over Na₂SO_{4,} filtered and concentrated under vacuum to yield ethyl 3-{4-amino-6-chloro-2-[(*E*)-2-phenylethenyl]-5-pyrimidinyl}propanoate as a white solid. LC-MS: m/z 332.2 (MH+). This product was then dissolved in DMF (5mL), treated with K₂CO₃ (0.16 g, 1.16 mmol) and heated at 75 °C for 30 minutes. The solution was cooled, diluted with water and extracted with Et₂O (3X). The organic layer was dried over Na₂SO₄, filtered, and concentrated under vacuum. The crude residue was then purified by column chromatography (silica gel) with DCM/(DCM:MeOH:NH₄OH) 90:10:1 to yield an additional 0.11 g of the desired compound.
LC-MS: m/z 286.2 (MH+).

### (f) 4-Chloro-7-oxo-5,6,7,8-tetrahydropyrido[2,3-d]pyrimidine-2-carbaldehyde

4-Chloro-2-[(*E*)-2-phenylethenyl]-5,8-dihydropyrido[2,3-*d*]pyrimidin-7(6*H*)-one (0.18 g, 0.64 mmol) was dissolved in a 2:1 solution of 1,4-dioxane/water (6mL) and cooled to 0 °C. NaIO₄ (0.314 g, 1.47 mmol) and catalytic OsO₄ (1 mL, 4% aq. solution) were added and the solution was then stirred at ambient temperature overnight. The reaction solution was concentrated under vacuum, diluted with water, and extracted with 10% MeOH/DCM (4X). The organic layers were combined, dried over Na₂SO_{4,} filtered and concentrated under vacuum. The crude residue was then purified by column chromatography (silica gel) using a DCM/DCM-MeOH-NH₄OH (90:10:1) gradient to yield the desired compound (0.05 g, 44%).
LC-MS: m/z 212.0 (MH+).

### (g) 2-[Bis(methyloxy)methyl]-4-chloro-5,8-dihydropyrido[2,3-d]pyrimidin-7(6H)-one

To a solution of 4-chloro-7-oxo-5,6,7,8-tetrahydropyrido[2,3-*d*]pyrimidine-2-carbaldehyde (1.43 g, 6.78 mmol) in MeOH was added *p*-TsOH • H₂O (0.13 g, 0.68 mmol). The solution was heated at reflux for 2.5 h and then cooled to ambient temperature. The solution was concentrated under vacuum to yield the desired product which was used without further purification.
LC-MS: m/z 257.9 (MH+).

### (h) 2-[Bis(methyloxy)methyl]-5,8-dihydropyrido[2,3-d]pyrimidin-7(6H)-one

To crude 2-[bis(methyloxy)methyl]-4-chloro-5,8-dihydropyrido[2,3-*d*]pyrimidin-7(6*H*)-one (presumed 6.78mmol) dissolved in MeOH was added 10% Pd/C (0.15 g). The solution was stirred under an atmosphere of H₂ (balloon) overnight. The Pd/C was filtered off and the solution concentrated under vacuum. The crude residue was purified by column chromatography (silica gel) using a DCM/DCM-MeOH-NH₄OH (90:10:1) gradient to yield the desired product as a white solid (0.873 g, 58% over 2 steps).
LC-MS: m/z 223.9 (MH+).

### (i) 7-Oxo-5,6,7,8-tetrahydropyrido[2,3-d]pyrimidine-2-carbaldehyde

To a solution of 2-[bis(methyloxy)methyl]-5,8-dihydropyrido[2,3-*d*]pyrimidin-7(6*H*)-one (0.873 g, 3.91 mmol) in 1:1 H₂O/acetone (10 mL) was added p-TsOH.H₂O (0.074 g, 0.391 mmol) and the reaction was heated to 80°C for 3 days with additional p-TsOH.H₂O (0.20 g). After the disappearance of starting material, the solution was concentrated under vacuum to yield the desired product (1.023g).
LC-MS: m/z 178.0 (MH+).

### (j) Title compound

To a solution of 5-[(4-amino-1-piperidinyl)methyl]-7-fluoro-5,6-dihydro-3*H-*pyrrolo[1,2,3-*de*]quinoxalin-3-one (for a preparation see Example 18(o)) (60 mg, 0.2 mmol) in methanol/DCM (10 ml/10 ml), was added 7-oxo-5,6,7,8-tetrahydropyrido[2,3-*d*]pyrimidine-2-carbaldehyde (50 mg; 0.2 mmol), and excess solid sodium sulphate. The solution was stirred at ambient temperature for 16 hours, followed by addition of sodium triacetoxyborohydride (0.126g; 0.6 mmol). The resulting solution was stirred for 2 hours. The solution was then concentrated onto silica gel under vacuum and the crude residue purified by column chromatography on silica gel (CH₂Cl₂/ 90:10:1 CH₂Cl₂/MeOH/NH₄OH) to yield the free base of the desired product as a yellowish oily film (0.033 g, 36%).
1H NMR δ (400 MHz, CDCl₃) 1.55 (d, 2 H) 1.88-2.04 (m, 2H), 2.14-2.37 (m, 2H) 2.53 (dd, 1H), 2.61-2.81 (m, 4H), 2.98 (t, 2H), 3.06 (d, 1H), 3.17 (dd, 1H), 3.51 (d, 2H), 4.05 (s, 2H), 5.09 (dd, 1H), 5.31 (s, 1H), 6.99 (t, 1H), 7.66 (dd, 1H), 8.16 (s, 1H) 8.39 (s, 1H). MS (ES+) m/z 464.2 (MH+).

The free base of the title compound dissolved in DCM was treated with one equivalent 1M hydrogen chloride in diethyl ether and evaporated to dryness to give the title compound as a light tan solid. (31 mg).

### Example 35 5-({4-[(6,7-Dihydro[1,4]dioxino[2,3-c]pyridazin-3-ylmethyl)amino]-1-piperidinyl}methyl)-3-(methyloxy)-4,5-dihydro-7H-pyrrolo[3,2,1-de]-1,5-naphthyridin-7-one dihydrochloride

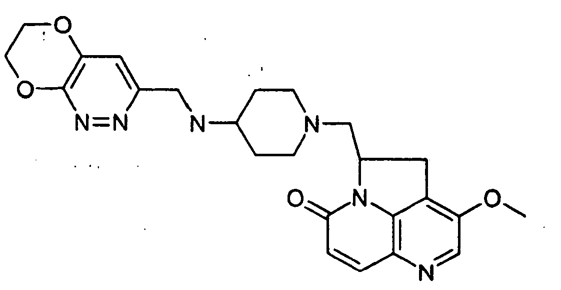

A solution of 5-[(4-amino-1-piperidinyl)methyl]-3-(methyloxy)-4,5-dihydro-7H-pyrrolo[3,2,1-de]-1,5-naphthyridin-7-one (for a preparation see Example 25(a)) (170 mg, 0.54 mmol) and 6,7-dihydro[1,4]dioxino[2,3-c]pyridazine-3-carbaldehyde (for a preparation see Example 3(e)) (100 mg, 0.60 mmol) in dichloromethane/methanol (10 ml/2 ml) was treated with sodium triacetoxyborohydride (343 mg, 0.52 mmol) and stirred for 18 hours. More aldehyde (20 mg) and more sodium triacetoxyborohydride (100 mg) were added. After 6 hours the mixture was treated with saturated aqueous sodium bicarbonate solution and dichloromethane. The phases were separated and the aqueous phase further extracted with dichloromethane. The combined extracts were washed with brine, dried and evaporated. Chromatography eluting with 0-30% methanol in dichloromethane afforded partially purified material which was triturated with ether then dried in vacuo affording the free base of the title compound as a yellow gum (56 mg).
¹H NMR (250 MHz) δ (CDCl₃) 1.30-1.45 (2H, m), 1.75-1.95 (2H, m), 2.20 (1H, t), 2.28 (1H, t), 2.45-2.55 (2H, m), 2.65 (1H, d), 3.05 (1H, d), 3.15 (1H, dd), 3.45 (1H, dd), 3.55 (1H, dd), 3.98 (2H, s), 4.07 (3H, s) , 4.35 (2H, m), 4.52 (2H, m), 5.05 (1H, m), 6.68 (1H, d), 7.03 (1H, s), 7.85 (1H, d), 8.10 (1H, s), 8.25 (1H, s),
MS (ES+) m/z 465 (MH⁺).

The free base of the title compound was dissolved in 2.5 ml of 19:1 chloroform:methanol and treated with 1M hydrochloric acid in ether (1 ml). More ether (4 ml) was added and the resulting suspension was centrifuged. Decantation of the supernatant and drying of the residue afforded the title compound (70 mg) as a solid.

### Biological Activity

### Antimicrobial Activity Assay:

Whole-cell antimicrobial activity was determined by broth microdilution using the Clinical and Laboratory Standards Institute (CLSI) recommended procedure, Document M7-A7, "Methods for Dilution Susceptibility Tests for Bacteria that Grow Aerobically". The compounds were tested in serial two-fold dilutions ranging from 0.016 to 16 mcg/mL.

Compounds were evaluated against Gram-positive organisms, selected from *Staphylococcus aureus, Streptococcus pneumoniae, Streptococcus pyogenes, Enterococcus faecalis* and *Enterococcus faecium.*

In addition, compounds were evaluated against Gram-negative organisms selected from *Haemophilus influenzae, Moraxella catarrhalis, Escherichia coli, Pseudomonas aeruginosa, Proteus mirabilis, Chlamydia pneumoniae, Enterobacter cloacae, Enterobacter aerogenes, Klebsiella pneumoniae* and *Stenotrophomonas maltophilia.*

For the *C. pneumoniae* isolates, stocks were thawed and diluted in CCM (Chlamydia Culture Media) to yield an inoculum containing ∼1 x 10⁴ inclusion forming units/ml (IFUs/ml). A 100 µL aliquot of the inoculum was added to all wells of a microtitre plate containing HEp-2 (Human Epithelial (pharyngeal) cell line) cells grown to confluence. Microtitre plates were centrifuged for 1 hour at 1700g., then incubated for 1 hour at 35°C in 5% CO₂. One hundred microliters of diluted test compounds, prepared as a 2-fold dilution series in CCM/cycloheximide was then added to the microtiter plates. After 72 hours incubation at 35°C in 5% CO_{2,} the microtitre plates were stained with a murine monoclonal fluorescein-conjugated antibody (Kallestad Cat. #532 Roche Biomedical Products) in accordance with the manufacturer recommendations. Upon staining, the IFUs produced an apple-green color, visible against the red counter stained HEp-2 cells when viewed at 100x magnification. The MIC was defined as the lowest concentration of compound at which no IFUs were seen.

The minimum inhibitory concentration (MIC) was determined as the lowest concentration of compound that inhibited visible growth. A mirror reader was used to assist in determining the MIC endpoint.

Each of the listed Examples, as identified in the present application, was tested in at least one exemplified salt form. The listed Examples had a MIC <2µg/ml against a strain of at least one of the organisms listed above. For at least one strain of every organism listed above, at least one Example had a MIC ≤32µg/ml.

## Claims

1. A compound of formula (I) or a pharmaceutically acceptable salt, solvate and/or N-oxide thereof: 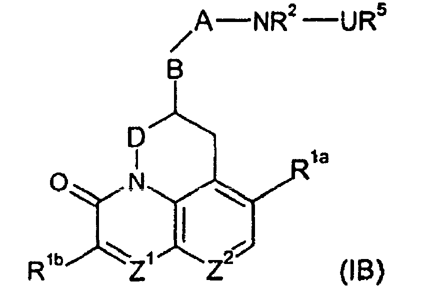 wherein:
one of B and D is CH₂ and the other is a bond;
one of Z¹ and Z² is CH or N and the other is CH;
R^{1a} and R^{1b} are independently selected from hydrogen; halogen; cyano; (C₁₋₆)alkyl; (C₁₋₆)alkylthio; trifluoromethyl; trifluoromethoxy; carboxy; hydroxy optionally substituted with (C₁₋₆)alkyl or (C₁₋₆)alkoxy-substituted (C₁₋₆)alkyl; (C₁₋₆)alkoxy-substituted (C₁₋₆)alkyl; hydroxy (C₁₋₆)alkyl; an amino group optionally N-substituted by one or two (C₁₋₆)alkyl, formyl, (C₁₋₆)alkylcarbonyl or (C₁₋₆)alkylsulphonyl groups; or aminocarbonyl wherein the amino group is optionally substituted by (C₁₋₄)alkyl;
provided that R^{1b} is H when Z¹ is N;
R² is hydrogen, or (C₁₋₄)alkyl, or together with R⁶ forms Y as defined below;
A is a group (i): 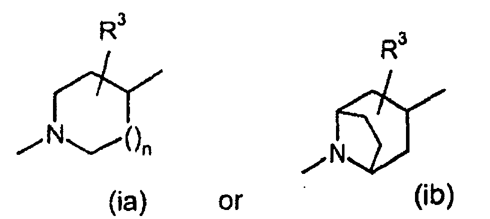
in which: R³ is as defined for R^{1a} or R^{1b} or is oxo and n is 1 or 2:
or A is a group (ii): 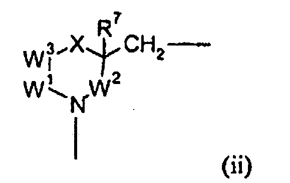
wherein:
W¹, W² and W³ are CR⁴R⁸
or W² and W³ are CR⁴R⁸ and W¹ represents a bond between W³ and N;
X is O, CR⁴R⁸, or NR⁶;
one R⁴ is as defined for R^{1a} and R^{1b} and the remainder and R⁸ are hydrogen or one R⁴ and R⁸ are together oxo and the remainder are hydrogen;
R⁶ is hydrogen or (C₁₋₆)alkyl; or together with R² forms Y;
R⁷ is hydrogen; halogen; hydroxy optionally substituted with (C₁₋₆)alkyl; or (C₁₋₆) alkyl;
Y is CR⁴R⁸CH₂; CH₂CR⁴R⁸; (C=O); CR⁴R⁸; CR⁴R⁸(C=O); or (C=O)CR⁴R⁸; or when X is CR⁴R⁸, R⁸ and R⁷ together represent a bond;
U is selected from CO and CH₂, and
R⁵ is an optionally substituted bicyclic carbocyclic or heterocyclic ring system (B): 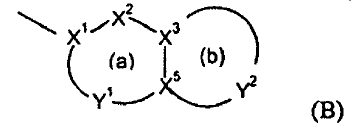 containing up to four heteroatoms in each ring in which
at least one of rings (a) and (b) is aromatic;
X¹ is C or N when part of an aromatic ring, or CR¹⁴ when part of a non-aromatic ring;
X² is N, NR¹³, O, S(O)_{X}, CO or CR¹⁴ when part of an aromatic or non-aromatic ring or may in addition be CR¹⁴R¹⁵ when part of a non-aromatic ring;
X³ and X⁵ are independently N or C;
Y¹ is a 0 to 4 atom linker group each atom of which is independently selected from N, NR¹³, O, S(O)_{X}, CO and CR¹⁴ when part of an aromatic or non-aromatic ring or may additionally be CR¹⁴R¹⁵ when part of a non-aromatic ring;
Y² is a 2 to 6 atom linker group, each atom of Y² being independently selected from N, NR¹³, O, S(O)_{X}, CO, CR¹⁴ when part of an aromatic or non-aromatic ring or may additionally be CR¹⁴R¹⁵ when part of a non-aromatic ring;
each of R¹⁴ and R¹⁵ is independently selected from: H; (C₁₋₄)alkylthio; halo; carboxy(C₁₋₄)alkyl; (C₁₋₄)alkyl; (C₁₋₄)alkoxycarbonyl; (C₁₋₄)alkylcarbonyl; (C₁₋₄₎ alkoxy (C₁₋₄)alkyl; hydroxy; hydroxy(C₁₋₄)alkyl; (C₁₋₄)alkoxy; nitro; cyano; carboxy; amino or aminocarbonyl optionally mono- or di-substituted by (C₁₋₄)alkyl; or
R¹⁴ and R¹⁵ may together represent oxo;
each R¹³ is independently H; trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; (C₁₋₄) alkoxycarbonyl; (C₁₋₄)alkylcarbonyl; (C₁₋₆)alkylsulphonyl; aminocarbonyl wherein the amino group is optionally mono or disubstituted by (C₁₋₄)alkyl; and
each x is independently 0, 1 or 2.

2. A compound according to claim 1 wherein
(1) B is CH₂ and D is a bond, Z¹ is CH and Z² is N;
(2) B is CH₂ and D is a bond, Z¹ is CH and Z² is CH;
(3) B is CH₂ and D is a bond, Z¹ is N and Z² is CH;
(4) B is a bond and D is CH₂, Z¹ is N and Z² is CH; or
(5) B is a bond and D is CH₂, Z¹ is CH and Z² is N.

3. A compound according to any preceding claim wherein R^{1a} is fluoro or methoxy and R^{1b} is hydrogen.

4. A compound according to any preceding claim wherein R² is hydrogen.

5. A compound according to any preceding claim wherein A is (ia), n is 1 and R³ is H or hydroxy in the 3-position, or A is (ii), X is CR⁴R⁸ and R⁸ is H and R⁴ is H or OH.

6. A compound according to any preceding claim wherein U is CH₂.

7. A compound according to any preceding claim wherein R⁵ is an aromatic heterocyclic ring (B) having 8-11 ring atoms including 2-4 heteroatoms of which at least one is N or NR¹³ in which Y² contains 2-3 heteroatoms, one of which is S and 1-2 are N, with one N bonded to X³, or the heterocyclic ring (B) has ring (a) aromatic selected from optionally substituted benzo, pyrido, pyridazino and pyrimidino and ring (b) non aromatic and Y² has 3-5 atoms, including at least one heteroatom, with O, S, CH₂ or NR¹³ bonded to X⁵ where R¹³ is other than hydrogen, and either NHCO bonded via N to X³, or O, S, CH₂ or NH bonded to X³.

8. A compound according to any of claims 1 to 6 wherein R⁵ is selected from:
3-oxo-3,4-dihydro-2H-pyrido[3,2-b][1,4]oxazin-6-yl
3-oxo-3,4-dihydro-2*H*-pyrido[3,2-*b*][1,4]thiazin-6-yl
6,7-dihydro[1,4]dioxino[2,3-*c*]pyridazin-3-yl
6,7-dihydro[1,4]oxathiino[2,3-*c*]pyridazin-3-yl
2,3-dihydro-[1,4]dioxino[2,3-c]pyridin-7-yl
[1,3]oxathiolo[5,4-*c*]pyridin-6-yl
2,3-dihydro[1,4]oxathiino[2,3-*c*]pyridin-7-yl
2-substituted 1H-pyrimido[5,4-b][1,4]oxazin-7(6H)-one
2-substituted 5,6-dihydropyrido[2,3-*d*]pyrimidin-7(1*H*)-one.

9. A compound according to claim 1 selected from:
5-({4-[(2,3-Dihydro[1,4]dioxino[2,3-*c*]pyridin-7-ylmethyl)amino]-1-piperidinyl}methyl)-3-fluoro-4,5-dihydro-7*H*-pyrrolo[3,2,1-*de*]-1,5-naphthyridin-7-one, Enantiomer 1
5-({4-[(2,3-Dihydro[1,4]dioxino[2,3-*c*]pyridin-7-ylmethyl)amino]-1-piperidinyl}methyl)-3-fluoro-4,5-dihydro-7*H*-pyrrolo[3,2,1-*de*]-1,5-naphthyridin-7-one, Enantiomer 2
5-({4-[(6,7-Dihydro[1,4]dioxino[2,3-c]pyridazin-3-ylmethyl)amino]-1-piperidinyl}methyl)-3-fluoro-4,5-dihydro-7H-pyrrolo[3,2,1-de]-1,5-naphthyridin-7-one, Enantiomer 1
5-({4-[(6,7-Dihydro[1,4]dioxino[2,3-c]pyridazin-3-ylmethyl)amino]-1-piperidinyl}methyl)-3-fluoro-4,5-dihydro-7H-pyrrolo[3,2,1-de]-1,5-naphthyridin-7-one, Enantiomer 2
3-Fluoro-5-({4-[([1,3]oxathiolo[5,4-c]pyridin-6-ylmethyl)amino]-1-piperidinyl}methyl)-4,5-dihydro-7H-pyrrolo[3,2,1-de]-1,5-naphthyridin-7-one, Enantiomer 1
3-Fluoro-5-({4-[([1,3]oxathiolo[5,4-c]pyridin-6-ylmethyl)amino]-1-piperidinyl}methyl)-4,5-dihydro-7H-pyrrolo[3,2,1-de]-1,4-naphthyridin-7-one, Enantiomer 2
5-({4-[(2,3-dihydro[1,4]oxathiino[2,3-c]pyridin-7-ylmethyl)amino]-1-piperidinyl}methyl)-3-fluoro-4,5-dihydro-7H-pyrrolo[3,2,1-de]-1,5-naphthyridin-7-one, Enantiomer 1
5-({4-[(2,3-dihydro[1,4]oxathiino[2,3-c]pyridin-7-ylmethyl)amino]-1-piperidinyl}methyl)-3-fluoro-4,5-dihydro-7H-pyrrolo[3,2,1-de]-1,5-naphthyridin-7-one, Enantiomer 2
3-Fluoro-5-[(4-{[(3-oxo-3,4-dihydro-2H-pyrido[3,2-b][1,4]thiazin-6-yl)methyl]amino}-1-piperidinyl)methyl]-4,5-dihydro-7H-pyrrolo[3,2,1-de]-1,5-naphthyridin-7-one, Enantiomer 1
3-Fluoro-5-[(4-{[(3-oxo-3,4-dihydro-2H-pyrido[3,2-b][1,4]thiazin-6-yl)methyl]amino}-1-piperidinyl)methyl]-4,5-dihydro-7H-pyrrolo[3,2,1-de]-1,5-naphthyridin-7-one, Enantiomer 2
(5*R*/*S*)-3-Fluoro-5-[(4-{[(3-oxo-3,4-dihydro-2*H*-pyrido[3,2-*b*][1,4]oxazin-6-yl)methyl]amino}-1-piperidinyl)methyl]-4,5-dihydro-7*H*-pyrrolo[3,2,1-*de*]-1,5-naphthyridin-7-one
2-({4-[(6,7-Dihydro[1,4]dioxino[2,3-c]pyridazin-3-ylmethyl)amino]-1-piperidinyl}methyl)-9-fluoro-1,2-dihydro-4*H*-pyrrolo[3,2,1-*ij*]quinolin-4-one, Enantiomer 1
2-({4-[(6,7-Dihydro[1,4]dioxino[2,3-*c*]pyridazin-3-ylmethyl)amino]-1-piperidinyl}methyl)-9-fluoro-1,2-dihydro-4*H*-pyrrolo[3,2,1-*ij*]quinolin-4-one, Enantiomer 2
2-({4-[(2,3-Dihydro[1,4]dioxino[2,3-*c*]pyridin-7-ylmethyl)amino]-1-piperidinyl}methyl)-9-fluoro-1,2-dihydro-4*H*-pyrrolo[3,2,1-*ij*]quinolin-4-one, Enantiomer 1
2-({4-[(2,3-Dihydro[1,4]dioxino[2,3-*c*]pyridin-7-ylmethyl)amino]-1-piperidinyl}methyl)-9-fluoro-1,2-dihydro-4*H*-pyrrolo[3,2,1-*ij*]quinolin-4-one, Enantiomer 2
2-({4-[(6,7-Dihydro[1,4]oxathiino[2,3-*c*]pyridazin-3-ylmethyl)amino]-1-piperidinyl}methyl)-9-fluoro-1,2-dihydro-4*H*-pyrrolo[3,2,1-*ij*]quinolin-4-one, Enantiomer 1
2-({4-[(6,7-Dihydro[1,4]oxathiino[2,3-*c*]pyridazin-3-ylmethyl)amino]-1-piperidinyl}methyl)-9-fluoro-1,2-dihydro-4*H*-pyrrolo[3,2,1-*ij*]quinolin-4-one, Enantiomer 2
5-({4-[(6,7-Dihydro[1,4]dioxino[2,3-*c*]pyridazin-3-ylmethyl)amino]-1-piperidinyl}methyl)-7-fluoro-5,6-dihydro-3*H*-pyrrolo[1,2,3-*de*]quinoxalin-3-one, Enantiomer 1
5-({4-[(6,7-Dihydro[1,4]dioxino[2,3-*c*]pyridazin-3-ylmethyl)amino]-1-piperidinyl}methyl)-7-fluoro-5,6-dihydro-3*H*-pyrrolo[1,2,3-*de*]quinoxalin-3-one, Enantiomer 2
(6R/S)-6-{4-[(2,3-Dihydro[1,4]dioxino[2,3-c]pyridin-7-ylmethyl)amino]-1-piperidinyl}-8-fluoro-6,7-dihydro-3H,5H-pyrido[1,2,3-de]quinoxalin-3-one
6-{4-[(6,7-Dihydro[1,4]dioxino[2,3-c]pyridazin-3-ylmethyl)amino]-1-piperidinyl}-8-fluoro-6,7-dihydro-3H,5H-pyrido[1,2,3-de]quinoxalin-3-one, Enantiomer 1
6-{4-[(6,7-Dihydro[1,4]dioxino[2,3-c]pyridazin-3-ylmethyl)amino]-1-piperidinyl}-8-fluoro-6,7-dihydro-3H,5H-pyrido[1,2,3-de]quinoxalin-3-one, Enantiomer 2
(5R/S)-5-({4-[(6,7-Dihydro[1,4]dioxino[2,3-c]pyridazin-3-ylmethyl)atnino]-1-piperidinyl}methyl)-7-(methyloxy)-5,6-dihydro-3H-pyrrolo[1,2,3-de]quinoxalin-3-one
(5R/*S*)-5-{4-[(2,3-dihydro[1,4]dioxino[2,3-*c*]pyridin-7-ylmethyl)amino]-1-piperidinyl}-3-fluoro-5,6-dihydro-4*H*,8*H*-pyrido[3,2,1-*de*]-1,5-naphthyridin-8-one
5-({4-[(2,3-Dihydro[1,4]dioxino[2,3-*c*]pyridin-7-ylmethyl)amino]-1-piperidinyl}methyl)-3-(methyloxy)-4,5-dihydro-7*H*-pyrrolo[3,2,1-*de*]-1,5-naphthyridin-7-one, Enantiomer I
5-({4-[(2,3-Dihydro[1,4]dioxino[2,3-*c*]pyridin-7-ylmethyl)amino]-1-piperidinyl}methyl)-3-(methyloxy)-4,5-dihydro-7*H*-pyrrolo[3,2,1-*de*]-1,5-naphthyridin-7-one, Enantiomer 2
5-({4-[(2,3-dihydro[1,4]dioxino[2,3-*c*]pyridin-7-ylmethyl)amino]-1-piperidinyl}methyl)-7-oxo-4,5-dihydro-7*H*-pyrrolo[3,2,1-*de*]-1,5-naphthyridine-3-carbonitrile
5-({4-[(2,3-Dihydro[1,4]dioxino[2,3-*c*]pyridin-7-ylmethyl)amino]-1-piperidinyl}methyl)-7-fluoro-5,6-dihydro-3*H*-pyrrolo[1,2,3-*de*]quinoxalin-3-one, Enantiomer E1
5-({4-[(2,3-Dihydro[1,4]dioxino[2,3-*c*]pyridin-7-ylmethyl)amino]-1-piperidinyl}methyl)-7-fluoro-5,6-dihydro-3*H*-pyrrolo[1,2,3-*de*]quinoxalin-3-one, Enantiomer E2
7-Fluoro-5-[(4-{[(3-oxo-3,4-dihydro-2*H*-pyrido[3,2-*b*][1,4]oxazin-6-yl)methyl]amino}-1-piperidinyl)methyl]-5,6-dihydro-3*H*-pyrrolo[1,2,3-*de*]quinoxalin-3-one, Enantiomer E1
7-Fluoro-5-[(4-{[(3-oxo-3,4-dihydro-2*H*-pyrido[3,2-*b*][1,4]oxazin-6-yl)methyl]amino}-1-piperidinyl)methyl]-5,6-dihydro-3*H*-pyrrolo[1,2,3-*de*]quinoxalin-3-one, Enantiomer E2
8-Fluoro-6-(4-{[(3-oxo-3,4-dihydro-2*H*-pyrido[3,2-*b*][1,4]oxazin-6-yl)methyl]amino}-1-piperidinyl)-6,7-dihydro-3*H*,5*H*-pyrido[1,2,3-*de*]quinoxalin-3-one, Enantiomer E1
8-Fluoro-6-(4-{[(3-oxo-3,4-dihydro-2*H*-pyrido[3,2-*b*][1,4]oxazin-6-yl)methyl]amina}-1-piperidinyl)-6,7-dihydro-3*H*,5*H*-pyrido[1,2,3-*de*]quinoxalin-3-one, Enantiomer E2
7-Fluoro-5-[(4-{[(7-oxo-1,5,6,7-tetrahydropyrido[2,3-*d*]pyrimidin-2-yl)methyl]amino}-1-piperidinyl)methyl]-5,6-dihydro-3*H*-pyrrolo[1,2,3-*de*]quinoxalin-3-one
5-({4-[(6,7-Dihydro[1,4]dioxino[2,3-c]pyridazin-3-ylmethyl)amino]-1-piperidinyl}methyl)-3-(methyloxy)-4,5-dihydro-7*H*-pyrrolo[3,2,1-*de*]-1,5-naphthyridin-7-one
or a pharmaceutically acceptable salt of any of the foregoing compounds.

10. A compound according to any preceding claim for use in a method of treatment of bacterial infections in mammals.

11. The use of a compound according to any one of claims 1 to 9, in the manufacture of a medicament for use in the treatment of bacterial infections in mammals.

12. A pharmaceutical composition comprising a compound according to any one of claims 1 to 9 and a pharmaceutically acceptable carrier.

## Patentansprüche

1. Eine Verbindung der Formel (I) oder ein pharmazeutisch verträgliches Salz, Solvat und/oder N-Oxid davon: 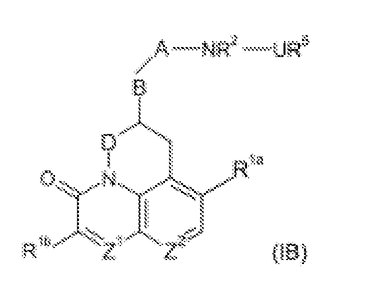 wobei:
einer der Reste B und D CH₂ ist und der andere eine Bindung ist;
einer der Reste Z¹ und Z² CH oder N ist und der andere CH ist;
R^{1a} und R^{1b} unabhängig ausgewählt sind aus Wasserstoff; Halogen; Cyano; (C₁₋₆)-Alkyl;
(C₁₋₆)-Alkylthio; Trifluormethyl; Trifluormethoxy; Carboxy; Hydroxy, gegebenenfalls substituiert mit (C₁₋₆)-Alkyl oder (C₁₋₆)-Alkoxy-substituiertem (C₁₋₆)-Alkyl;
(C₁₋₆)-Alkoxy-substituiertem (C₁₋₆)-Alkyl; Hydroxy-(C₁₋₆)-alkyl; einer Aminogruppe,
gegebenenfalls N-substituiert mit einem oder zwei (C₁₋₆)-Alkyl-, Formyl-,
(C₁₋₆)-Alkylcarbonyl- oder (C₁₋₆)-Alkylsulfonylresten; oder Aminocarbonyl, wobei die Aminogruppe gegebenenfalls mit (C₁₋₄)-Alkyl substituiert ist;
mit der Maßgabe, dass R^{1b} H ist, wenn Z¹ N ist;
R² Wasserstoff oder (C₁₋₄)-Alkyl ist oder zusammen mit R⁶ Y wie nachstehend definiert bildet;
A ein Rest (i) ist: 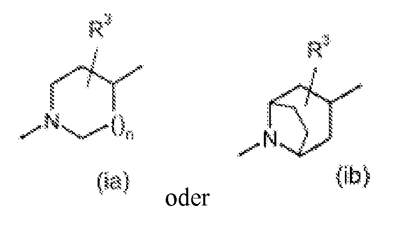
wobei: R³ wie R^{1a} oder R^{1b} definiert ist oder Oxo ist und n gleich 1 oder 2 ist:
oder A ein Rest (ii) ist: 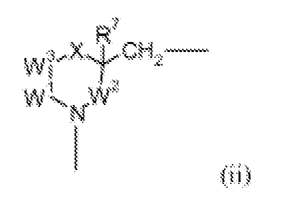
wobei: W¹, W² und W³ gleich CR⁴R⁸ sind
oder W² und W³ gleich CR⁴R⁸ sind und W¹ eine Bindung zwischen W³ und N darstellt; X gleich O, CR⁴R⁸ oder NR⁶ ist;
einer der Reste R⁴ wie für R^{1a} und R^{1b} definiert ist und die Übrigen und die Reste R⁸ Wasserstoff sind, oder einer der Reste R⁴ und R⁸ zusammen Oxo sind und die Übrigen Wasserstoff sind;
R⁶ Wasserstoff oder (C₁₋₆)-Alkyl ist oder zusammen mit R² Y bildet;
R⁷ Wasserstoff; Halogen; Hydroxy, gegebenenfalls substituiert mit (C_{I-6})-Alkyl; oder (C₁₋₆)-Alkyl ist;
Y gleich CR⁴R⁸CH₂, CH₂CR⁴R⁸; (C=O); CR⁴R⁸; CR⁴R⁸(C=O) oder (C=O)CR⁴R⁸ ist; oder wenn X gleich CR⁴R⁸ ist, R⁸ und R⁷ zusammen eine Bindung darstellen;
U ausgewählt ist aus CO und CH₂, und
R⁵ ein gegebenenfalls substituiertes bicyclisches carbocyclisches oder heterocyclisches Ringsystem (B): 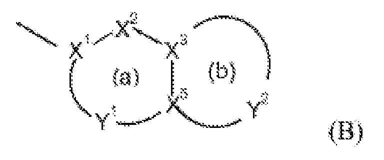
welches bis zu vier Heteroatome in jedem Ring enthält, ist, wobei mindestens einer der Ringe (a) und (b) aromatisch ist;
X¹ gleich C oder N ist, wenn es Teil eines aromatischen Rings ist, oder CR¹⁴ ist, wenn es Teil eines nicht aromatischen Rings ist;
X² gleich N, NR¹³, O, S(O)ₓ, CO oder CR¹⁴ ist, wenn es Teil eines aromatischen oder nicht aromatischen Rings ist, oder zusätzlich CR¹⁴R¹⁵ sein kann, wenn es Teil eines nicht aromatischen Rings ist;
X³ und X⁵ unabhängig N oder C sind;
Y¹ ein Linkerrest mit 0 bis 4 Atomen ist, wobei jedes Atom unabhängig ausgewählt ist aus N, NR¹³, O, S(O)ₓ, CO und CR¹⁴, wenn es Teil eines aromatischen oder nicht aromatischen Rings ist, oder zusätzlich CR¹⁴R¹⁵ sein kann, wenn es Teil eines nicht aromatischen Rings ist;
Y² ein Linkerrest mit 2 bis 6 Atomen ist, wobei jedes Atom von Y² unabhängig ausgewählt ist aus N, NR¹³, O, S(O)ₓ, CO, CR¹⁴, wenn es Teil eines aromatischen oder nicht aromatischen Rings ist, oder zusätzlich CR¹⁴R¹⁵ sein kann, wenn es Teil eines nicht aromatischen Rings ist;
jeder der Reste R¹⁴ und R¹⁵ unabhängig ausgewählt ist aus H; (C₁₋₄)-Alkylthio; Halogen; Carboxy-(C₁₋₄)-alkyl; (C₁₋₄)-Alkyl; (C₁₋₄)-Alkoxycarbonyl; (C₁₋₄)-Alkylcarbonyl; (C₁₋₄)-Alkoxy-(C₁₋₄)-alkyl; Hydroxy; Hydroxy-(C₁₋₄)-alkyl; (C₁₋₄)-Alkoxy; Nitro; Cyano; Carboxy; Amino oder Aminocarbonyl, gegebenenfalls mono- oder disubstituiert mit (C₁₋₄)-Alkyl; oder
R¹⁴ und R¹⁵ zusammen Oxo darstellen können;
jedes R¹³ unabhängig H; Trifluormethyl; (C₁₋₄)-Alkyl, gegebenenfalls substituiert mit Hydroxy, (C₁₋₆)-Alkoxy, (C₁₋₆)-Alkylthio, Halogen oder Trifluormethyl; (C₂₋₄)-Alkenyl; (C₁₋₄)-Alkoxycarbonyl; (C₁₋₄)-Alkylcarbonyl; (C₁₋₆)-Alkylsulfonyl; Aminocarbonyl, wobei die Aminogruppe gegebenenfalls mit (C₁₋₄)-Alkyl mono- oder disubstituiert ist, ist; und
jedes x unabhängig 0, 1 oder 2 ist.

2. Eine Verbindung gemäß Anspruch 1, wobei
(1) B CH₂ ist und D eine Bindung ist, Z¹ CH ist und Z² N ist;
(2) B CH₂ ist und D eine Bindung ist, Z¹ CH ist und Z² CH ist;
(3) B CH₂ ist und D eine Bindung ist, Z¹ N ist und Z² CH ist;
(4) B eine Bindung ist und D CH₂ ist, Z¹ N ist und Z² CH ist; oder
(5) B eine Bindung ist und D CH₂ ist, Z¹ CH ist und Z² N ist.

3. Eine Verbindung gemäß einem der vorstehenden Ansprüche, wobei R^{1a} Fluor oder Methoxy ist und R^{1b} Wasserstoff ist.

4. Eine Verbindung gemäß einem der vorstehenden Ansprüche, wobei R² Wasserstoff ist.

5. Eine Verbindung gemäß einem der vorstehenden Ansprüche, wobei A (ia) ist, n gleich 1 ist und R³ gleich H oder Hydroxy an Position 3 ist oder A (ii) ist, X gleich CR⁴R⁸ ist und R⁸ gleich H ist und R⁴ gleich H oder OH ist.

6. Eine Verbindung gemäß einem der vorstehenden Ansprüche, wobei U gleich CH₂ ist.

7. Eine Verbindung gemäß einem der vorstehenden Ansprüche, wobei R⁵ ein aromatischer heterocyclischer Ring (B) mit 8 bis 11 Ringatomen, einschließlich 2 bis 4 Heteroatomen, ist, wovon mindestens eines N oder NR¹³ ist, wobei Y² 2 bis 3 Heteroatomen enthält, wovon eines S ist und 1 bis 2 N sind, wobei ein N an X³ gebunden ist, oder der heterocyclische Ring (B) einen aromatischen Ring (a), ausgewählt aus gegebenenfalls substituiertem Benzo, Pyrido, Pyridazino und Pyrimidino, und einen nicht aromatischen Ring (b) aufweist, und Y² 3 bis 5 Atome aufweist, einschließlich mindestens eines Heteroatoms, wobei O, S, CH₂ oder NR¹³ an X⁵ gebunden sind, wobei R¹³ von Wasserstoff verschieden ist, und entweder NHCO über N an X³ oder O, S, CH₂ oder NH, an X³ gebunden ist.

8. Eine Verbindung gemäß einem der Ansprüche 1 bis 6, wobei R⁵ ausgewählt ist aus:
3-Oxo-3,4-dihydro-2H-pyrido[3,2-b][1,4]oxazin-6-yl,
3-Oxo-3,4-dihydro-2H-pyrido[3,2-b][1,4]thiazin-6-yl,
6,7-Dihydro[1,4]dioxino[2,3-c]pyridazin-3-yl,
6,7-Dihydro[1,4]oxathiino[2,3-c]pyridazin-3-yl,
2,3-Dihydro-[1,4]dioxino[2,3-c]pyridin-7-yl,
[1,3]Oxathiolo[5,4-c]pyridin-6-yl,
2,3-Dihydro[1,4]oxathiino[2,3-c]pyridin-7-yl,
2-substitutiertem 1H-Pyrimido[5,4-b][1,4]oxazin-7(6H)-on,
2-substitutiertem 5,6-Dihydropyrido[2,3-d]pyrimidin-7(1H)-on.

9. Eine Verbindung gemäß Anspruch 1, ausgewählt aus:
5-({4-[(2,3-Dihydro[1,4]dioxino[2,3-c]pyridin-7-ylmethyl)amino]-1-piperidinyl}methyl)-3-fluor-4,5-dihydro-7H-pyrrolo[3,2,1-de]-1,5-naphthyridin-7-on, Enantiomer 1,
5-({4-[(2,3-Dihydro[1,4]dioxino[2,3-c]pyridin-7-ylmethyl)amino]-1-piperidinyl}methyl)-3-fluor-4,5-dihydro-7H-pyrrolo[3,2,1-de]-1,5-naphthyridin-7-on, Enantiomer 2,
5-( {4-[(6,7-Dihydro[1,4]dioxino[2,3-c]pyridazin-3-ylmethyl)amino]-1-piperidinyl}-methyl)-3-fluor-4,5-dihydro-7H-pyrrolo[3,2,1-de]-1,5-naphthyridin-7-on, Enantiomer 1,
5-({4-[(6,7-Dihydro[1,4]dioxino[2,3-c]pyridazin-3-ylmethyl)amino]-1-piperidinyl}-methyl)-3-fluor-4,5-dihydro-7H-pyrrolo[3,2,1-de]-1,5-naphthyridin-7-on, Enantiomer 2,
3-Fluor-5-({4-[([1,3]oxathiolo[5,4-c]pyridin-6-ylmethyl)amino]-1-piperidinyl}methyl)-4,5-dihydro-7H-pyrrolo[3,2,1-de]-1,5-naphthyridin-7-on, Enantiomer 1,
3-Fluor-5-({4-[([1,3]oxathiolo[5,4-c]pyridin-6-ylmethyl)amino]-1-piperidinyl}methyl)-4,5-dihydro-7H-pyrrolo[3,2,1-de]-1,5-naphthyridin-7-on, Enantiomer 2,
5-({4-[(2,3-Dihydro[1,4]oxathiino[2,3-c]pyridin-7-ylmethyl)amino]-1-piperidinyl}-methyl)-3-fluor-4,5-dihydro-7H-pyrrolo[3,2,1-de]-1,5-naphthyridin-7-on, Enantiomer 1,
5-({4-[(2,3-dihydro[1,4]oxathiino[2,3-c]pyridin-7-ylmethyl)amino]-1-piperidinyl}-methyl)-3-fluor-4,5-dihydro-7H-pyrrolo[3,2,1-de]-1,5-naphthyridin-7-on, Enantiomer 2,
3-Fluor-5-[(4-{[(3-oxo-3,4-dihydro-2H-pyrido[3,2-b][1,4]thiazin-6-yl)methyl]amino}-1-piperidinyl)methyl]-4,5-dihydro-7H-pyrrolo[3,2,1-de]-1,5-naphthyridin-7-on, Enantiomer 1,
3-Fluor-5-[(4-{[(3-oxo-3,4-dihydro-2H-pyrido[3,2-b][1,4]thiazin-6-yl)methyl]amino}-1-piperidinyl)methyl]-4,5-dihydro-7H-pyrrolo[3,2,1-de]-1,5-naphthyridin-7-on, Enantiomer 2,
(5R/S)-3-Fluor-5-[(4-{[(3-oxo-3,4-dihydro-2H-pyrido[3,2-b][1,4]oxazin-6-yl)methyl]-amino}-1-piperidinyl)methyl]-4,5-dihydro-7H-pyrrolo[3,2,1-de]-1,5-naphthyridin-7-on,
2-({4-[(6,7-Dihydro[1,4]dioxino[2,3-c]pyridazin-3-ylmethyl)amino]-1-pipendinyl}-methyl)-9-fluor-1,2-dihydro-4H-pyrrolo[3,2,1-ij]chinolin-4-on, Enantiomer 1,
2-({4-[(6,7-Dihydro[1,4]dioxino[2,3-c]pyridazin-3-ylmethyl)amino]-1-piperidinyl}-methyl)-9-fluor-1,2-dihydro-4H-pyrrolo[3,2,1-ij]chinolin-4-on, Enantiomer 2,
2-({4-[(2,3-Dihydro[1,4]dioxino[2,3-c]pyridin-7-ylmethyl)amino]-1-piperidinyl}methyl)-9-fluor-1,2-dihydro-4H-pyrrolo[3,2,1-ij]chinolin-4-on, Enantiomer 1,
2-({4-[(2,3-Dihydro[1,4]dioxino[2,3-c]pyridin-7-ylmethyl)amino]-1-piperidinyl}methyl)-9-fluor-1,2-dihydro-4H-pyrrolo[3,2,1-ij]quinolin-4-on, Enantiomer 2,
2-({4-[(6,7-Dihydro[1,4]oxathiino[2,3-c]pyridazin-3-ylmethyl)amino]-1-piperidinyl}-methyl)-9-fluor-1,2-dihydro-4H-pyrrolo[3,2,1-ij]chinolin-4-on, Enantiomer 1,
2-({4-[(6,7-Dihydro[1,4]oxathiino[2,3-c]pyridazin-3-ylmethyl)amino]-1-piperidinyl}-methyl)-9-fluor-1,2-dihydro-4H-pyrrolo[3,2,1-ij]chinolin-4-on, Enantiomer 2,
5-({4-[(6,7-Dihydro[1,4]dioxino[2,3-c]pyridazin-3-ylmethyl)amino]-1-piperidinyl}-methyl)-7-fluor-5,6-dihydro-3H-pyrrolo[1,2,3-de]chinoxalin-3-on, Enantiomer 1,
5-({4-[(6,7-Dihydro[1,4]dioxino[2,3-c]pyridazin-3-ylmethyl)amino]-1-piperidinyl}-methyl)-7-fluor-5,6-dihydro-3H-pyrrolo[1,2,3-de]chinoxalin-3-on, Enantiomer 2,
(6R/S)-6-{4-[(2,3-Dihydro[1,4]dioxino[2,3-c]pyridin-7-ylmethyl)amino]-1-piperidinyl}-8-fluor-6,7-dihydro-3H,5H-pyrido[1,2,3-de]chinoxalin-3-on,
6-{4-[(6,7-Dihydro[1,4]dioxino[2,3-c]pyridazin-3-ylmethyl)amino]-1-piperidinyl}-8-fluor-6,7-dihydro-3H,5H-pyrido[1,2,3-de]chinoxalin-3-on, Enantiomer 1,
6-{4-[(6,7-Dihydro[1,4]dioxino[2,3-c]pyridazin-3-ylmethyl)amino]-1-piperidinyl}-8-fluor-6,7-dihydro-3H,5H-pyrido[1,2,3-de]chinoxalin-3-on, Enantiomer 2,
(5R/S)-5-({4-[(6,7-Dihydro[1,4]dioxino[2,3-c]pyridazin-3-ylmethyl)amino]-1-piperidinyl}methyl)-7-(methyloxy)-5,6-dihydro-3H-pyrrolo[1,2,3-de]chinoxalin-3-on,
(5R/S)-5-{4-[(2,3-dihydro[1,4]dioxino[2,3-c]pyridin-7-ylmethyl)amino]-1-piperidinyl}-3-fluor-5,6-dihydro-4H,8H-pyrido[3,2,1-de]-1,5-naphthyridin-8-on,
5-({4-[(2,3-Dihydro[1,4]dioxino[2,3-c]pyridin-7-ylmethyl)amino]-1-piperidinyl}methyl)-3-(methyloxy)-4,5-dihydro-7H-pyrrolo[3,2,1-de]-1,5-naphthyridin-7-on, Enantiomer 1,
5-({4-[(2,3-Dihydro[1,4]dioxino[2,3-c]pyridin-7-ylmethyl)amino]-1-piperidinyl}methyl)-3-(methyloxy)-4,5-dihydro-7H-pyrrolo[3,2,1-de]-1,5-naphthyridin-7-on, Enantiomer 2,
5-({4-[(2,3-dihydro[1,4]dioxino[2,3-c]pyridin-7-ylmethyl)amino]-1-piperidinyl}methyl)-7-oxo-4,5-dihydro-7H-pyrrolo[3,2,1-de]-1,5-naphthyridin-3-carbonitril,
5-({4-[(2,3-Dihydro[1,4]dioxino[2,3-c]pyridin-7-ylmethyl)amino]-1-piperidinyl}methyl)-7-fluor-5,6-dihydro-3H-pyrrolo[1,2,3-de]chinoxalin-3-on, Enantiomer E1,
5-({4-[(2,3-Dihydro[1,4]dioxino[2,3-c]pyridin-7-ylmethyl)amino]-1-piperidinyl}methyl)-7-fluor-5,6-dihydro-3H-pyrrolo[1,2,3-de]chinoxalin-3-on, Enantiomer E2,
7-Fluor-5-[(4-{[(3-oxo-3,4-dihydro-2H-pyrido[3,2-b][1,4]oxazin-6-yl)methyl]amino}-1-piperidinyl)methyl]-5,6-dihydro-3H-pyrrolo[1,2,3-de]chinoxalin-3-on, Enantiomer E1,
7-Fluor-5-[(4-{[(3-oxo-3,4-dihydro-2H-pyrido[3,2-b][1,4]oxazin-6-yl)methyl]amino}-1-piperidinyl)methyl]-5,6-dihydro-3H-pyrrolo[1,2,3-de]chinoxalin-3-on, Enantiomer E2,
8-Fluor-6-(4-{[(3-oxo-3,4-dihydro-2H-pyrido[3,2-b][1,4]oxazin-6-yl)methyl]amino}-1-piperidinyl)-6,7-dihydro-3H,5H-pyrido[1,2,3-de]chinoxalin-3-on, Enantiomer E1,
8-Fluor-6-(4-{[(3-oxo-3,4-dihydro-2H-pyrido[3,2-b][1,4]oxazin-6-yl)methyl]amino}-1-piperidinyl)-6,7-dihydro-3H,5H-pyrido[1,2,3-de]chinoxalin-3-on, Enantiomer E2,
7-Fluor-5-[(4-{[(7-oxo-1,5,6,7-tetrahydropyrido[2,3-d]pyrimidin-2-yl)methyl]amino}-1-piperidinyl)methyl]-5,6-dihydro-3H-pyrrolo[1,2,3-de]chinoxalin-3-on,
5-({4-[(6,7-Dihydro[1,4]dioxino[2,3-c]pyridazin-3-ylmethyl)amino]-1-piperidinyl}-methyl)-3-(methyloxy)-4,5-dihydro-7H-pyrrolo[3,2,1-de]-1,5-naphthyridin-7-on,
oder ein pharmazeutisch verträgliches Salz einer der vorstehenden Verbindungen.

10. Eine Verbindung gemäß einem der vorstehenden Ansprüche zur Verwendung in einem Verfahren zur Behandlung von bakteriellen Infektionen in Säugern.

11. Die Verwendung einer Verbindung gemäß einem der Ansprüche 1 bis 9 zur Herstellung eines Medikaments zur Verwendung bei der Behandlung von bakteriellen Infektionen in Säugern.

12. Ein Arzneimittel, umfassend eine Verbindung gemäß einem der Ansprüche 1 bis 9 und einen pharmazeutisch verträglichen Träger.

## Revendications

1. Composé de formule (I) ou un de ses sels, produits de solvatation et/ou N-oxydes pharmaceutiquement acceptables : 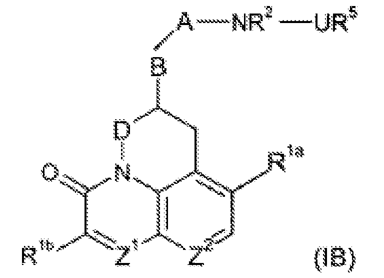 dans laquelle :
un de B et D représente un groupe CH₂ et l'autre représente une liaison ;
un de Z¹ et Z² représente un groupe CH ou N et l'autre représente un groupe CH ;
R^{1a} et R^{1b} sont choisis indépendamment entre un atome d'hydrogène ; des groupes halogéno ; cyano ; alkyle en C₁ à C₆ ; alkylthio en C₁ à C₆ ; trifluorométhyle ; trifluoro-méthoxy ; carboxy ; hydroxy facultativement substitué avec un substituant alkyle en C₁ à C₆ ou alkyle en C₁ à C₆ à substituant alkoxy en C₁ à C₆ ; alkyle en C₁ à C₆ à substituant alkoxy en C₁ à C₆ ; hydroxyalkyle en C₁ à C₆ ; un groupe amino facultativement N-substitué avec un ou deux groupes alkyle en C₁ à C₆, formyle, (alkyle en C₁ à C₆)carbonyle ou alkylsulfonyle en C₁ à C₆ ; ou aminocarbonyle dans lequel le groupe amino est facultativement substitué avec un groupe alkyle en C₁ à C₄ ;
sous réserve que R^{1b} représente H lorsque Z¹ représente N ;
R² représente un atome d'hydrogène ou un groupe alkyle en C₁ à C₄ ou bien, conjointement avec R⁶, forme un groupe Y tel que défini ci-dessous ;
A représente un groupe (i) : 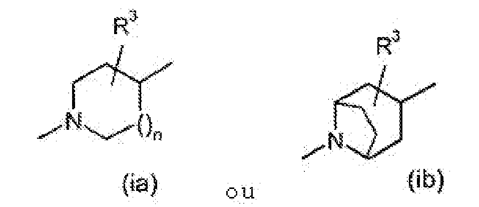
dans lequel : R³ est tel que défini pour R^{1a} ou R^{1b} ou représente un groupe oxo et n est égal à 1 ou 2 ;
ou bien A représente un groupe (ii) : 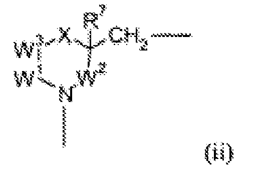
dans lequel :
W¹, W² et W³ représentent des groupes CR⁴R⁸
ou bien W² et W³ représentent un groupe CR⁴R⁸ et W¹ représente une liaison entre W³ et N ;
X représente 0, un groupe CR⁴R⁸ ou NR⁶ ;
un groupe R⁴ est tel que défini pour R^{1a} et R^{1b} et le reste et R⁸ représentent des atomes d'hydrogène ou bien un groupe R⁴ et un groupe R⁸ représentent conjointement un groupe oxo et le reste représente des atomes d'hydrogène ;
R⁶ représente un atome d'hydrogène ou un groupe alkyle en C₁ à C₆ ; ou bien, conjointement avec R², forme un groupe Y ;
R⁷ représente un atome d'hydrogène ; un groupe halogéno ;
hydroxy facultativement substitué avec un substituant alkyle en C₁ à C₆ ; ou alkyle en C₁ à C₆ ;
Y représente un groupe CR⁴R⁸CH₂ ; CH₂CR⁴R⁸ ; (C=O) ; CR⁴R⁸ ; CR⁴R⁸ (C=O) ; ou (C=O) CR⁴R⁸;
ou, lorsque X représente un groupe CR⁴R⁸, R⁸ et R⁷, conjointement, représentent une liaison ;
U est choisi entre des groupes CO et CH₂, et
R⁵ représente un système de noyau carbocyclique ou hétérocyclique bicyclique facultativement substitué (B) : 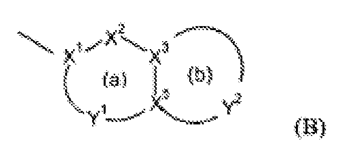
contenant jusqu'à quatre hétéroatomes dans chaque noyau, dans lequel
au moins l'un des noyaux (a) et (b) est aromatique ;
X¹ représente C ou N lorsqu'il fait partie d'un noyau aromatique, ou un groupe CR¹⁴ lorsqu'il fait partie d'un noyau non aromatique ;
X² représente N, un groupe NR¹³, 0, S(O)ₓ, CO ou CR¹⁴ lorsqu'il fait partie d'un noyau aromatique ou non aromatique ou bien peut représenter en outre un groupe CR¹⁴R¹⁵ lorsqu'il fait partie d'un noyau non aromatique ;
X³ et X⁵ représentent indépendamment N ou C ;
Y¹ représente un groupe de liaison de 0 à 4 atomes, dont chaque atome est choisi indépendamment entre N, NR¹³, O, S(O)ₓ, CO et CR¹⁴ lorsqu'il fait partie d'un noyau aromatique ou non aromatique ou bien peut représenter en outre un groupe CR¹⁴R¹⁵ lorsqu'il fait partie d'un noyau non aromatique ;
Y² représente un groupe de liaison de 0 à 6 atomes, chaque atome de Y² étant choisi indépendamment entre N, NR¹³, O, S(O)ₓ, CO et CR¹⁴ lorsqu'il fait partie d'un noyau aromatique ou non aromatique ou bien peut représenter en outre un groupe CR¹⁴R¹⁵ lorsqu'il fait partie d'un noyau non aromatique ;
chacun de R¹⁴ et R¹⁵ est choisi indépendamment entre : H ; des groupes alkylthio en C₁ à C₄ ; halogéno ; carboxy-(alkyle en C₁ à C₄) ; alkyle en C₁ à C₄ ; (alkoxy en C₁ à C₄)carbonyle ; (alkyle en C₁ à C₄) carbonyle ; (alkoxy en C₁ à C₄) (alkyle en C₁ à C₄) ; hydroxy ; hydroxyalkyle en C₁ à C₄ ; alkoxy en C₁ à C₄ ; nitro ; cyano ; carboxy ; amino ou aminocarbonyle facultativement mono- ou disubstitué avec un substituant alkyle en C₁ à C₄ ; ou bien
R¹⁴ et R¹⁵ peuvent représenter conjointement un groupe oxo ;
chaque groupe R¹³ représente indépendamment H ; un groupe trifluorométhyle ; alkyle en C₁ à C₄ facultativement substitué avec un substituant hydroxy, alkoxy en C₁ à C₆, alkylthio en C₁ à C₆, halogéno ou trifluorométhyle ; alcényle en C₂ à C₄ ; (alkoxy en C₁ à C₄) carbonyle ; (alkyle en C₁ à C₄)carbonyle ; alkylsulfonyle en C₁ à C₆ ; aminocarbonyle dans lequel le groupe amino est facultativement mono- ou disubstitué avec un substituant alkyle en C₁ à C₄ ; et
chaque indice x est indépendamment égal à 0, 1 ou 2.

2. Composé suivant la revendication 1, dans lequel
(1) B représente un groupe CH₂ et D représente une liaison, Z¹ représente un groupe CH et Z² représente N ;
(2) B représente un groupe CH₂ et D représente une liaison, Z¹ représente un groupe CH et Z² représente CH ;
(3) B représente un groupe CH₂ et D représente une liaison, Z¹ représente N et Z² représente un groupe CH ;
(4) B représente une liaison et D représente un groupe CH₂, Z¹ représente N et Z² représente un groupe CH ; ou
(5) B représente une liaison et D représente un groupe CH₂, Z¹ représente un groupe CH et Z² représente N.

3. Composé suivant l'une quelconque des revendications précédentes, dans lequel R^{1a} représente un groupe fluoro ou méthoxy et R^{1b} représente un atome d'hydrogène.

4. Composé suivant l'une quelconque des revendications précédentes, dans lequel R² représente un atome d'hydrogène.

5. Composé suivant l'une quelconque des revendications précédentes, dans lequel A représente un groupe (ia), n est égal à 1 et R³ représente H ou un groupe hydroxy en position 3, ou bien A représente un groupe (ii), X représente un groupe CR⁴R⁸ et R⁸ représente H et R⁴ représente H ou un groupe OH.

6. Composé suivant l'une quelconque des revendications précédentes, dans lequel U représente un groupe CH₂.

7. Composé suivant l'une quelconque des revendications précédentes, dans lequel R⁵ représente un noyau hétérocyclique aromatique (B) comprenant 8 à 11 atomes dans le noyau, y compris 2 à 4 hétéroatomes dont au moins l'un consiste en N ou NR¹³, dans lequel Y² contient 2 ou 3 hétéroatomes, dont l'un consiste en un atome de S et 1 ou 2 consistent en un atome de N, avec un atome de N lié à X³, ou bien le noyau hétérocyclique (B) possède un noyau (a) aromatique choisi entre des noyaux benzo, pyrido, pyridazino et pyrimidino facultativement substitués et un noyau (b) non aromatique et Y² a 3 à 5 atomes, y compris au moins un hétéroatome, avec 0, S, CH₂ ou NR¹³ lié à X⁵, où R¹³ est autre qu'un atome d'hydrogène, et soit le groupe NHCO lié par l'intermédiaire de N à X³, soit 0, S, CH₂ ou NH lié à X³.

8. Composé suivant l'une quelconque des revendications 1 à 6, dans lequel R⁵ est choisi entre des groupes :
3-oxo-3,4-dihydro-2H-pyrido[3,2-b][1,4]oxazine-6-yle,
3-oxo-3,4-dihydro-2*H*-pyrido[3,2-*b*][1,4]thiazine-6-yle,
6,7-dihydro[1,4]dioxino[2,3-*c*]pyridazine-3-yle,
6,7-dihydro[1,4]oxathiino[2,3-*c*]pyridazine-3-yle,
2,3-dihydro[1,4]dioxino[2,3-*c*]pyridine-7-yle,
[1,3]oxathiolo[5,4-*c*]pyridine-6-yle,
2,3-dihydro[1,4]oxathiino[2,3-*c*]pyridine-7-yle, 1H-pyrimido[5,4-b][1,4]oxazine-7(6H)-one substitué en position 2,
5,6-dihydropyrido[2,3-*d*]pyrimidine-7(1*H*)-one substitué en position 2.

9. Composé suivant la revendication 1, choisi entre :
l'énantiomère 1 de 5-({4-[(2,3-dihydro[1,4]dioxino-[2,3-*c*]pyridine-7-ylméthyl)amino]-1-pipéridinyl}méthyl)-3-fluoro-4,5-dihydro-7*H*-pyrrolo[3,2,1-*de*]-1,5-naphtyridine-7-one,
l'énantiomère 2 de 5-({4-[(2,3-dihydro[1,4]dioxino-[2,3-*c*]pyridine-7-ylméthyl)amino]-1-pipéridinyl}méthyl)-3-fluoro-4,5-dihydro-7*H*-pyrrolo[3,2,1-*de*]-1,5-naphtyridine-7-one,
l'énantiomère 1 de 5-({4-[(6,7-dihydro[1,4]dioxino-[2,3-c]pyridazine-3-ylméthyl)amino]-1-pipéridinyl}méthyl)-3-fluoro-4,5-dihydro-7H-pyrrolo[3,2,1-de]-1,5-naphtyridine-7-one,
l'énantiomère 2 de 5-({4-[(6,7-dihydro[1,4]dioxino-[2,3-c]pyridazine-3-ylméthyl)amino]-1-pipéridinyl}méthyl)-3-fluoro-4,5-dihydro-7H-pyrrolo[3,2,1-de]-1,5-naphtyridine-7-one,
l'énantiomère 1 de 3-fluoro-5-({4-[([1,3]oxathiolo-[5,4-c]pyridine-6-ylméthyl)amino]-1-pipéridinyl}méthyl)-4,5-dihydro-7H-pyrrolo[3,2,1-de]-1,5-naphtyridine-7-one,
l'énantiomère 2 de 3-fluoro-5-({4-[([1,3]oxathiolo-[5,4-c]pyridine-6-ylméthyl)amino]-1-pipéridinyl}méthyl)-4,5-dihydro-7H-pyrrolo[3,2,1-de]-1,5-naphtyridine-7-one,
l'énantiomère 1 de 5-({4-[(2,3-dihydro[1,4]oxathiino-[2,3-c]pyridine-7-ylméthyl)amino]-1-pipéridinyl}méthyl)-3-fluoro-4,5-dihydro-7H-pyrrolo[3,2,1-de]-1,5-naphtyridine-7-one,
l'énantiomère 2 de 5-({4-[(2,3-dihydro[1,4]oxathiino-[2,3-c]pyridine-7-ylméthyl)amino]-1-pipéridinyl}méthyl)-3-fluoro-4,5-dihydro-7H-pyrrolo[3,2,1-de]-1,5-naphtyridine-7-one,
l'énantiomère 1 de 3-fluoro-5-[(4-{[(3-oxo-3,4-dihydro-2H-pyrido[3,2-b][1,4]thiazine-6-yl)méthyl]amino}-1-pipéridinyl)-méthyl]-4,5-dihydro-7H-pyrrolo[3,2,1-de]-1,5-naphtyridine-7-one,
l'énantiomère 2 de 3-fluoro-5-[(4-{[(3-oxo-3,4-dihydro-2H-pyrido[3,2-b][1,4]thiazine-6-yl)méthyl]amino}-1-pipéridinyl)-méthyl]-4,5-dihydro-7H-pyrrolo[3,2,1-de]-1,5-naphtyridine-7-one,
la (5*R*/*S*)-3-fluoro-5-[(4-{[(3-oxo-3,4-dihydro-2*H*-pyrido-[3,2-*b*][1,4]oxazine-6-yl)méthyl]amino}-1-pipéridinyl)méthyl]-4,5-dihydro-7H-pyrrolo[3,2,1-de]-1,5-naphtyridine-7-one,
l'énantiomère 1 de 2-({4-[(6,7-dihydro[1,4]dioxino-[2,3-*c*]pyridazine-3-ylméthyl)amino]-1-pipéridinyl}méthyl)-9-fluoro-1,2-dihydro-4*H*-pyrrolo[3,2,1-*ij*]quinoléine-4-one,
l'énantiomère 2 de 2-({4-[(6,7-dihydro[1,4]dioxino-[2,3-*c*]pyridazine-3-ylméthyl)amino]-1-pipéridinyl}méthyl)-9-fluoro-1,2-dihydro-4*H*-pyrrolo[3,2,1-*ij*]quinoléine-4-one,
l'énantiomère 1 de 2-({4-[(2,3-dihydro[1,4]dioxino-[2,3-*c*]pyridine-7-ylméthyl)amino]-1-pipéridinyl}méthyl)-9-fluoro-1,2-dihydro-4*H*-pyrrolo[3,2,1-*ij*]quinoléine-4-one,
l'énantiomère 2 de 2-({4-[(2,3-dihydro[1,4]dioxino-[2,3-*c*]pyridine-7-ylméthyl)amino]-1-pipéridinyl}méthyl)-9-fluoro-1,2-dihydro-4*H*-pyrrolo[3,2,1-*ij*]quinoléine-4-one,
l'énantiomère 1 de 2-({4-[(6,7-dihydro[1,4]oxathiino-[2,3-*c*]pyridazine-3-ylméthyl)amino]-1-pipéridinyl}méthyl)-9-fluoro-1,2-dihydro-4*H*-pyrrolo[3,2,1-*ij*]quinoléine-4-one,
l'énantiomère 2 de 2-({4-[(6,7-dihydro[1,4]oxathiino-[2,3-*c*]pyridazine-3-ylméthyl)amino]-1-pipéridinyl}méthyl)-9-fluoro-1,2-dihydro-4*H*-pyrrolo[3,2,1-*ij*]quinoléine-4-one,
l'énantiomère 1 de 5-({4-[(6,7-dihydro[1,4]dioxino-[2,3-*c*]pyridazine-3-ylméthyl)amino]-1-pipéridinyl}méthyl)-7-fluoro-5,6-dihydro-3H-pyrrolo[1,2,3-de]quinoxaline-3-one,
l'énantiomère 2 de 5-({4-[(6,7-dihydro[1,4]dioxino-[2,3-*c*]pyridazine-3-ylméthyl)amino]-1-pipéridinyl}méthyl)-7-fluoro-5,6-dihydro-3H-pyrrolo[1,2,3-de]quinoxaline-3-one,
la (6R/S)-6-{4-[(2,3-dihydro[1,4]dioxino[2,3-c]pyridine-7-ylméthyl)amino]-1-pipéridinyl}-8-fluoro-6,7-dihydro-3H,5H-pyrido[1,2,3-de]-quinoxaline-3-one,
l'énantiomère 1 de 6-{4-[(6,7-dihydro[1,4]dioxino-[2,3-c]pyridazine-3-ylméthyl)amino]-1-pipéridinyl}-8-fluoro-6,7-dihydro-3H,5H-pyrido[1,2,3-de]quinoxaline-3-one,
l'énantiomère 2 de 6-{4-[(6,7-dihydro[1,4]dioxino-[2,3-c]pyridazine-3-ylméthyl)amino]-1-pipéridinyl}-8-fluoro-6,7-dihydro-3H,5H-pyrido[1,2,3-de]quinoxaline-3-one,
la (5R/S)-5-({4-[(6,7-dihydro[1,4]dioxino[2,3-c]pyridazine-3-ylméthyl)amino]-1-pipéridinyl}méthyl)-7-(méthyloxy)-5,6-dihydro-3H-pyrrolo[1,2,3-de]-quinoxaline-3-one,
la (5R/*S*)-5-{4-[(2,3-dihydro[1,4]dioxino[2,3-c]pyridine-7-ylméthyl)amino]-1-pipéridinyl}-3-fluoro-5,6-dihydro-4*H*,8*H-*pyrido[3,2,1-*de*]-1,5-naphtyridine-8-one,
l'énantiomère 1 de 5-({4-[(2,3-dihydro[1,4]dioxino-[2,3-*c*]pyridine-7-ylméthyl)amino]-1-pipéridinyl}méthyl)-3-(méthyloxy)-4,5-dihydro-7*H*-pyrrolo[3,2,1-*de*]-1,5-naphtyridine-7-one,
l'énantiomère 2 de 5-({4-[(2,3-dihydro[1,4]dioxino-[2,3-*c*]pyridine-7-ylméthyl)amino]-1-pipéridinyl}méthyl)-3-(méthyloxy)-4,5-dihydro-7*H*-pyrrolo[3,2,1-*de*]-1,5-naphtyridine-7-one,
le 5-({4-[(2,3-dihydro[1,4]dioxino[2,3-*c*]pyridine-7-ylméthyl)amino]-1-pipéridinyl}méthyl)-7-oxo-4,5-dihydro-7*H-*pyrrolo[3,2,1-de]-1,5-naphtyridine-3-carbonitrile,
l'énantiomère E1 de 5-({4-[(2,3-dihydro[1,4]dioxino-[2,3-*c*]pyridine-7-ylméthyl)amino]-1-pipéridinyl}méthyl)-7-fluoro-5,6-dihydro-3H-pyrrolo[1,2,3-de]-quinoxaline-3-one,
l'énantiomère E2 de 5-({4-[(2,3-dihydro[1,4]dioxino-[2,3-*c*]pyridine-7-ylméthyl)amino]-1-pipéridinyl}méthyl)-7-fluoro-5,6-dihydro-3H-pyrrolo[1,2,3-de]-quinoxaline-3-one,
l'énantiomère E1 de 7-fluoro-5-[(4-{[(3-oxo-3,4-dihydro-2*H*-pyrido[3,2-*b*][1,4]oxazine-6-yl)méthyl]amino}-1-pipéridinyl)-méthyl]-5,6-dihydro-3H-pyrrolo[1,2,3-de]quinoxaline-3-one,
l'énantiomère E2 de 7-fluoro-5-[(4-{[(3-oxo-3,4-dihydro-2H-pyrido[3,2-*b*][1,4]oxazine-6-yl)méthyl]amino}-1-pipéridinyl)-méthyl]-5,6-dihydro-3H-pyrrolo[1,2,3-de]quinoxaline-3-one,
l'énantiomère E1 de 8-fluoro-6-(4-{[(3-oxo-3,4-dihydro-2*H*-pyrido[3,2-*b*][1,4]oxazine-6-yl)méthyl]amino}-1-pipéridinyl)-6,7-dihydro-3*H*,5*H*-pyrido[1,2,3-*de*]quinoxaline-3-one,
l'énantiomère E2 de 8-fluoro-6-(4-{[(3-oxo-3,4-dihydro-2*H*-pyrido[3,2-*b*][1,4]oxazine-6-yl)méthyl]amino}-1-pipéridinyl)-6,7-dihydro-3*H*,5*H*-pyrido[1,2,3-de]quinoxaline-3-one,
la 7-fluoro-5-[(4-{[(7-oxo-1,5,6,7-tétrahydropyrido-[2,3-*d*]pyrimidine-2-yl)méthyl]amino}-1-pipéridinyl]méthyl)-5,6-dihydro-3H-pyrrolo[1,2,3-de]quinoxaline-3-one,
la 5-({4-[(6,7-dihydro[1,4]dioxino[2,3-*c*]pyridazine-3-ylméthyl)amino]-1-pipéridinyl}méthyl)-3-(méthyloxy)-4,5-dihydro-7H-pyrrolo[3,2,1-de]-1,5-naphtyridine-7-one,
ou un sel pharmaceutiquement acceptable de n'importe lequel des composés précités.

10. Composé suivant l'une quelconque des revendications précédentes destiné à être utilisé dans une méthode de traitement d'infections bactériennes chez des mammifères.

11. Utilisation d'un composé suivant l'une quelconque des revendications 1 à 9, dans la production d'un médicament destiné à être utilisé dans le traitement d'infections bactériennes chez des mammifères.

12. Composition pharmaceutique comprenant un composé suivant l'une quelconque des revendications 1 à 9 et un support pharmaceutiquement acceptable.
